# EUROPEAN PATENT SPECIFICATION

(11) **EP 3 856 933 B1**
(45) Date of publication and mention of the grant of the patent: **02.12.2026**
(21) Application number: 19773848.7
(22) Date of filing: 26.09.2019
(51) Int. Cl.: C12Q 1/6886

(54) **PREDICTIVE AND PROGNOSTIC METHODS IN BREAST CANCER**
PRÄDIKTIVE UND PROGNOSTISCHE METHODEN FÜR BRUSTKREBS
MÉTHODES PRÉDICTIVES ET PRONOSTIQUES DANS LE CANCER DU SEIN

(30) Priority: 27.09.2018 WO PCT/EP2018/076279
(43) Date of publication of application: 04.08.2021
(73) Proprietor: BioNTech Diagnostics GmbH, 55131 Mainz (DE); STRATIFYER Molecular Pathology GmbH, 50935 Köln (DE)
(72) Inventor: LAIBLE, Mark, 55131 Mainz (DE); OED, Michael, 55131 Mainz (DE); WIRTZ, Ralph, 55131 Mainz (DE)
(74) Representative: Michalski Hüttermann & Partner mbB
(86) International application number: PCT/EP2019/076109
(87) International publication number: WO 2020/064966

(56) References cited:
- WO-A1-2013/014296
- WO-A1-2018/097678
- IWATA HIROJI ET AL: "Validation of the 21-gene test as a predictor of clinical response to neoadjuvant hormonal therapy for ER+, HER2-negative breast cancer: the TransNEOS study", BREAST CANCER RESEARCH AND TREATMENT, SPRINGER , NY, US, vol. 173, no. 1, 21 September 2018 (2018-09-21), pages 123 - 133, XP036709534, ISSN: 0167-6806, [retrieved on 20180921], DOI: 10.1007/S10549-018-4964-Y
- FASHING ET AL: "Validation of the MammaTyperVR pathological complete response (pCR)-score as a predictor for response after neoadjuvant chemotherapy (NACT) in patients with early breast cancer (BC)", ANNALS OF ONCOLOGY, 1 October 2018 (2018-10-01), XP055654018, Retrieved from the Internet <URL:https://academic.oup.com/annonc/article-pdf/29/suppl_8/mdy269.166/26140854/mdy269.166.pdf> [retrieved on 20191218], DOI: 10.1093/annonc/mdy269

## Description

### TECHNICAL FIELD OF THE INVENTION

The present invention relates to methods of predicting the probability of pathological complete response (pCR) of a breast cancer patient upon neo-adjuvant chemotherapy, to methods for selecting a breast cancer treatment, to methods of treatment of breast cancer, and to methods of prognosis of breast cancer upon breast cancer treatment.

### BACKGROUND OF THE INVENTION

Neo-adjuvant therapy is an increasingly common mode of chemotherapy in clinical practice and has been included as a standard treatment approach to render operable non-resectable breast cancers and evaluate invivo response to drugs. Excluding patients from the neo-adjuvant therapy regime who will have no benefit is the most important first step while planning the therapy; therefore, the prediction of response to such a neo-adjuvant treatment is of high clinical value.

Furthermore, the achievement of a pathologic complete response (pCR) is a highly significant predictor for improved disease-free survival (DFS) and overall survival (OS) regardless of the type of treatment (see, e.g., Broglio K.R. et al., 2016, JAMA Oncology 2(6):751-760).

Although several methods and parameters are described which allow for the prediction of pCR, no method is widely accepted as standard and applied routinely. This is mainly due to the fact that many of the methods, especially the implementation of Ki67 IHC (IHC for ImmunoHistoChemistry; Olfatbakhsh A. et al., 2018, Int J Cancer Manag. 11(5):e60098) and IHC4 (Elsamany S. et al., 2015, APJCP 16(17):7975-7979) are difficult to be standardized and would, therefore, yield significantly different results when applied routinely in different laboratories.

Accordingly, it was an object of the present invention to provide objective, quantitative, reproducible reliable and routinely applicable methods for the prediction of pCR of breast cancer patients upon neo-adjuvant chemotherapy, for selecting a breast cancer treatment for a given breast cancer patient and for the prognosis of breast cancer in a breast cancer patient upon breast cancer treatment.

These and other objects are solved by the present invention, which will be described in the following.

### SUMMARY OF THE INVENTION

The invention is defined by the features of the independent claims. The dependent claims refer to particular embodiments of the present invention.

In one aspect, the present invention relates to a method of predicting the probability of pathological complete response (pCR) of a breast cancer patient upon neo-adjuvant chemotherapy, said method comprising:
calculating a score unscaled (su) based on the relative expression levels of mRNA of
   i) ERBB2, ESR1, PGR and MKI67,
   ii) ERBB2, ESR1, and MKI67 or
   iii) ESR1, and MKI67
relative to the level of expression of one or more reference genes selected from the group comprising B2M, CALM2, TBP, PUM1, MRLP19, GUSB, RPL37A and CYFIP1 in a pre-treatment breast tumor sample of the breast cancer patient as determined by reverse transcription quantitative PCR (RT-qPCR), wherein
   a) a higher score su indicates a higher probability of pCR, wherein a higher relative expression level of mRNA of ERBB2 is associated with a higher su, a higher relative expression level of mRNA of ESR1 is associated with a lower su, a higher relative expression level of mRNA of PGR is associated with a lower su, and a higher relative expression level of mRNA of MKI67 is associated with a higher su; or
   b) a lower score su indicates a higher probability of pCR, wherein a higher relative expression level of mRNA of ERBB2 is associated with a lower su, a higher relative expression level of mRNA of ESR1 is associated with a higher su, a higher relative expression level of mRNA of PGR is associated with a higher su, and a higher relative expression level of mRNA of MKI67 is associated with a lower su,

wherein the method comprises, prior to calculating su:
   determining the relative expression levels of mRNA of
   i) ERBB2, ESR1, PGR and MKI67,
   ii) ERBB2, ESR1, and MKI67 or
   iii) ESR1, and MKI67, respectively,
in the pre-treatment breast tumor sample by RT-qPCR.

In one embodiment, the neo-adjuvant chemotherapy comprises administration of a taxane.

In one embodiment, the neo-adjuvant chemotherapy comprises administration of a taxane, optionally wherein the neo-adjuvant chemotherapy is accompanied by the administration of an anti-ERBB2 drug if the breast cancer is an ERBB2-positive breast cancer.

In one embodiment, the breast cancer is i) a luminal breast cancer, and/or ii) an ESR1- and/or PGR-positive breast cancer.

In one embodiment, the su is calculated based on the relative expression levels of mRNA of ERBB2, ESR1, PGR and MKI67, and in the calculation of su, the relative expression levels (RELs) of mRNA of ERBB2, ESR1, PGR and MKI67 are weighted as follows: 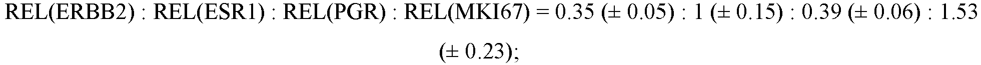 REL(ERBB2) : REL(ESR1) : REL(PGR) : REL(MKI67) = 0.35 (± 0.05) : 1 (± 0.15) : 0.39 (± 0.06) : 1.53 (± 0.23); or 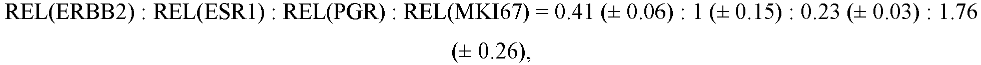 REL(ERBB2) : REL(ESR1) : REL(PGR) : REL(MKI67) = 0.41 (± 0.06) : 1 (± 0.15) : 0.23 (± 0.03) : 1.76 (± 0.26), the su is calculated based on the relative expression levels of mRNA of ERBB2, ESR1, and MKI67, and in the calculation of su, the relative expression levels (RELs) of mRNA of ERBB2, ESR1, and MKI67 are weighted as follows:$REL \left(ERBB2\right) : REL \left(ESR1\right) : REL \left(MKI67\right) = 0.34 \left(\pm 0.05\right) : 1 \left(\pm 0.15\right) : 1.61 \left(\pm 0.24\right) ,$ or the su is calculated based on the relative expression levels of mRNA of ESR1, and MKI67, and in the calculation of su, the relative expression levels (RELs) of mRNA of ESR1 and MKI67 are weighted as follows: $REL \left(ESR1\right) : REL \left(MKI67\right) = 1 \left(\pm 0.15\right) : 1.63 \left(\pm 0.24\right) .$

In one embodiment, the su is calculated based on the relative expression levels of mRNA of ERBB2, ESR1, PGR and MKI67, and a higher score su indicates a higher probability of pCR, and wherein su is calculated by using the formula: 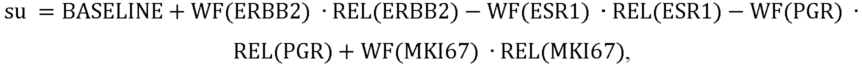 su = BASELINE + WF(ERBB2) · REL(ERBB2) - WF(ESR1) · REL(ESR1) - WF(PGR) · REL(PGR) + WF(MKI67) · REL(MKI67), wherein WF(ERBB2) is a weighting factor for REL(ERBB2), WF(ESR1) is a weighting factor for REL(ESR1), WF(PGR) is a weighting factor for REL(PGR2), and WF(MKI67) is a weighting factor for REL(MKI67).

In one embodiment, the su is calculated based on the relative expression levels of mRNA of ERBB2, ESR1, PGR and MKI67, and a higher score su indicates a higher probability of pCR, and wherein su is calculated by using the formula: 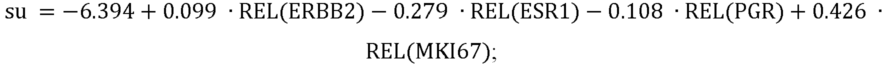 su = -6.394 + 0.099 · REL(ERBB2) - 0.279 · REL(ESR1) - 0.108 · REL(PGR) + 0.426 · REL(MKI67); or 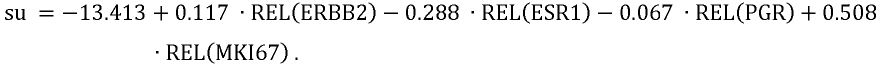 su = -13.413 + 0.117 · REL(ERBB2) - 0.288 · REL(ESR1) - 0.067 · REL(PGR) + 0.508 · REL(MKI67) .

In one embodiment, the su is calculated based on the relative expression levels of mRNA of ERBB2, ESR1, PGR and MKI67, and a lower score su indicates a higher probability of pCR, and wherein su is calculated by using the formula: 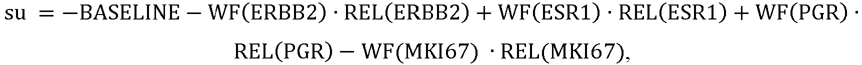 su = -BASELINE - WF(ERBB2) · REL(ERBB2) + WF(ESR1) · REL(ESR1) + WF(PGR) · REL(PGR) - WF(MKI67) · REL(MKI67), wherein WF(ERBB2) is a weighting factor for REL(ERBB2), WF(ESR1) is a weighting factor for REL(ESR1), WF(PGR) is a weighting factor for REL(PGR2), and WF(MKI67) is a weighting factor for REL(MKI67).

In one embodiment, the su is calculated based on the relative expression levels of mRNA of ERBB2, ESR1, PGR and MKI67, and a lower score su indicates a higher probability of pCR, and wherein su is calculated by using the formula: 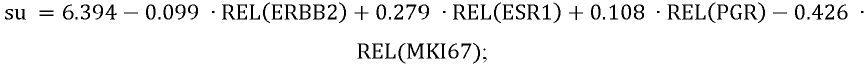 su = 6.394 - 0.099 · REL(ERBB2) + 0.279 · REL(ESR1) + 0.108 · REL(PGR) - 0.426 · REL(MKI67); or 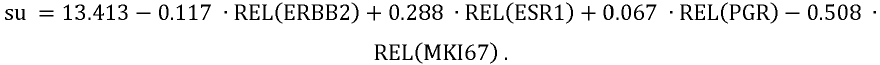 su = 13.413 - 0.117 · REL(ERBB2) + 0.288 · REL(ESR1) + 0.067 · REL(PGR) - 0.508 · REL(MKI67) .

In one embodiment, the method further comprises:
calculating a predicted probability of pCR q, wherein
a) if a higher score su indicates a higher probability of pCR, q is calculated by using the formula $q = \frac{exp \left(su\right)}{\left(1+exp\left(su\right)\right)} ;$ and
b) if a lower score su indicates a higher probability of pCR, q is calculated by using the formula $q = 1 - \frac{exp \left(su\right)}{\left(1+exp\left(su\right)\right)} ,$
wherein, preferably, a q which is equal to or greater than a pre-defined threshold indicates a high probability of pCR, and a q which is lower than a pre-defined threshold indicates a low probability of pCR.

In one embodiment, the method further comprises:
calculating a clinical score s based on su, wherein s has a scale from 0 to 100.

In one embodiment, su is calculated based on the relative expression levels of mRNA of ERBB2, ESR1, PGR and MKI67 by using the formula 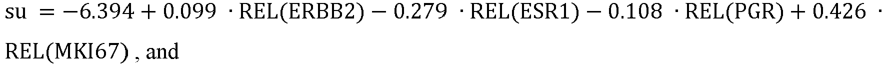 su = -6.394 + 0.099 · REL(ERBB2) - 0.279 · REL(ESR1) - 0.108 · REL(PGR) + 0.426 · REL(MKI67) , and
wherein the method further comprises:
calculating a clinical score s based on su, wherein s is calculated by using the formula $s = \left(su+3.960\right) ⋅ 18.191 \left(round to 0 decimal places\right) ,$ wherein
if (su + 3.960) · 18.191 < 0 s = 0, and
if (su + 3.960) · 18.191 > 100 s = 100.

In one embodiment,
a) if a higher score su indicates a higher probability of pCR, a score s or a score su which is equal to or greater than a pre-defined threshold indicates a high probability of pCR, and a score s or a score su which is lower than the pre-defined threshold indicates a low probability of pCR; and
b) if a lower score su indicates a higher probability of pCR, a score s or a score su which is lower than a pre-defined threshold indicates a high probability of pCR, and a score s or a score su which is equal to or greater than the pre-defined threshold indicates a low probability of pCR.

The present invention further relates to a method of predicting the probability of pathological complete response (pCR) of a breast cancer patient upon neo-adjuvant chemotherapy, said method comprising:
calculating a score unscaled (su) based on the relative expression levels of mRNA of ERBB2, ESR1 and MKI67 relative to the level of expression of one or more reference genes selected from the group comprising B2M, CALM2, TBP, PUM1, MRLP19, GUSB, RPL37A and CYFIP1 in a pre-treatment breast tumor sample of the breast cancer patient as determined by reverse transcription quantitative PCR (RT-qPCR), wherein
a) a higher score su indicates a higher probability of pCR, wherein a higher relative expression level of mRNA of ERBB2 is associated with a higher su, a higher relative expression level of mRNA of ESR1 is associated with a lower su, and a higher relative expression level of mRNA of MKI67 is associated with a higher su; or
b) a lower score su indicates a higher probability of pCR, wherein a higher relative expression level of mRNA of ERBB2 is associated with a lower su, a higher relative expression level of mRNA of ESR1 is associated with a higher su, and a higher relative expression level of mRNA of MKI67 is associated with a lower su,
wherein the method comprises, prior to calculating su:
determining the relative expression levels of mRNA of ERBB2, ESR1 and MKI67 in the pre-treatment breast tumor sample by RT-qPCR.

In one embodiment, the neo-adjuvant chemotherapy comprises administration of a taxane.

In one embodiment, the neo-adjuvant chemotherapy is accompanied by the administration of an anti-ERBB2 drug if the breast cancer is an ERBB2-positive breast cancer.

In one embodiment, the breast cancer is i) a luminal breast cancer, and/or ii) an ESR1- and/or PGR-positive breast cancer.

In one embodiment, in the calculation of su, the relative expression levels (RELs) of mRNA of ERBB2, ESR1, PGR and MKI67 are weighted as follows: $REL \left(ERBB2\right) : REL \left(ESR1\right) : REL \left(MKI67\right) = 0.34 \left(\pm 0.05\right) : 1 \left(\pm 0.15\right) : 1.61 \left(\pm 0.24\right) .$

In one embodiment, the su is calculated based on the relative expression levels of mRNA of ERBB2, ESR1 and MKI67, and a higher score su indicates a higher probability of pCR, and wherein su is calculated by using the formula: 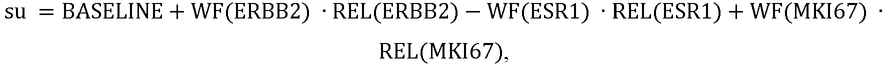 su = BASELINE + WF(ERBB2) · REL(ERBB2) - WF(ESR1) · REL(ESR1) + WF(MKI67) · REL(MKI67), wherein WF(ERBB2) is a weighting factor for REL(ERBB2), WF(ESR1) is a weighting factor for REL(ESR1), and WF(MKI67) is a weighting factor for REL(MKI67).

In one embodiment, the su is calculated based on the relative expression levels of mRNA of ERBB2, ESR1, and MKI67, and a higher score su indicates a higher probability of pCR, and wherein su is calculated by using the formula: $su = - 15.209 + 0.114 ⋅ REL \left(ERBB2\right) - 0.335 ⋅ REL \left(ESR1\right) + 0.539 ⋅ REL \left(MKI67\right) .$

In one embodiment, the su is calculated based on the relative expression levels of mRNA of ERBB2, ESR1, and MKI67, and a lower score su indicates a higher probability of pCR, and wherein su is calculated by using the formula: 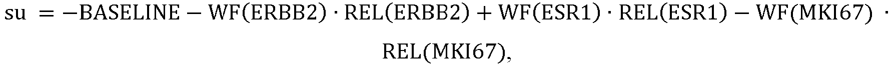 su = -BASELINE - WF(ERBB2) · REL(ERBB2) + WF(ESR1) · REL(ESR1) - WF(MKI67) · REL(MKI67), wherein WF(ERBB2) is a weighting factor for REL(ERBB2), WF(ESR1) is a weighting factor for REL(ESR1), and WF(MKI67) is a weighting factor for REL(MKI67).

In one embodiment, the su is calculated based on the relative expression levels of mRNA of ERBB2, ESR1, and MKI67, and a lower score su indicates a higher probability of pCR, and wherein su is calculated by using the formula: $su = 15.209 - 0.114 ⋅ REL \left(ERBB2\right) + 0.335 ⋅ REL \left(ESR1\right) - 0.539 ⋅ REL \left(MKI67\right) .$

In one embodiment, the method further comprises:
calculating a predicted probability of pCR q, wherein
a) if a higher score su indicates a higher probability of pCR, q is calculated by using the formula $q = \frac{exp \left(su\right)}{\left(1+exp\left(su\right)\right)} ;$ and
b) if a lower score su indicates a higher probability of pCR, q is calculated by using the formula $q = 1 - \frac{exp \left(su\right)}{\left(1+exp\left(su\right)\right)} ,$
wherein, preferably, a q which is equal to or greater than a pre-defined threshold indicates a high probability of pCR, and a q which is lower than a pre-defined threshold indicates a low probability of pCR.

In one embodiment, the method further comprises:
calculating a clinical score s based on su, wherein s has a scale from 0 to 100.

In one embodiment,
a) if a higher score su indicates a higher probability of pCR, a score s or a score su which is equal to or greater than a pre-defined threshold indicates a high probability of pCR, and a score s or a score su which is lower than the pre-defined threshold indicates a low probability of pCR; and
b) if a lower score su indicates a higher probability of pCR, a score s or a score su which is lower than a pre-defined threshold indicates a high probability of pCR, and a score s or a score su which is equal to or greater than the pre-defined threshold indicates a low probability of pCR.

The present invention further relates to a method predicting the probability of pathological complete response (pCR) of a breast cancer patient upon neo-adjuvant chemotherapy, said method comprising:
calculating a score unscaled (su) based on the relative expression levels of mRNA of ESR1 and MKI67 relative to the level of expression of one or more reference genes selected from the group comprising B2M, CALM2, TBP, PUM1, MRLP19, GUSB, RPL37A and CYFIP1 in a pre-treatment breast tumor sample of the breast cancer patient as determined by reverse transcription quantitative PCR (RT-qPCR), wherein
(i) a higher score su indicates a higher probability of pCR, wherein a higher relative expression level of mRNA of ESR1 is associated with a lower su, and a higher relative expression level of mRNA of MKI67 is associated with a higher su; or
(ii) a lower score su indicates a higher probability of pCR, wherein a higher relative expression level of mRNA of ESR1 is associated with a higher su, and a higher relative expression level of mRNA of MKI67 is associated with a lower su,
wherein the method comprises, prior to calculating su:
determining the relative expression levels of mRNA of ESR1 and MKI67
in the pre-treatment breast tumor sample by RT-qPCR.

In one embodiment, the neo-adjuvant chemotherapy comprises administration of a taxane.

In one embodiment, the neo-adjuvant chemotherapy is accompanied by the administration of an anti-ERBB2 drug if the breast cancer is an ERBB2-positive breast cancer.

In one embodiment, the breast cancer is i) a luminal breast cancer, and/or ii) an ESR1- and/or PGR-positive breast cancer.

In one embodiment, in the calculation of su, the relative expression levels (RELs) of mRNA of ESR1 and MKI67 are weighted as follows: $REL \left(ESR1\right) : REL \left(MKI67\right) = 1 \left(\pm 0.15\right) : 1.63 \left(\pm 0.24\right) .$

In one embodiment, a higher score su indicates a higher probability of pCR, and wherein su is calculated by using the formula: $su = BASELINE - WF \left(ESR1\right) ⋅ REL \left(ESR1\right) + WF \left(MKI67\right) ⋅ REL \left(MKI67\right) ,$ wherein WF(ESR1) is a weighting factor for REL(ESR1), and WF(MKI67) is a weighting factor for REL(MKI67).

In one embodiment, the su is calculated based on the relative expression levels of mRNA of ESR1 and MKI67, and a higher score su indicates a higher probability of pCR, and wherein su is calculated by using the formula: $su = - 10.625 - 0.324 ⋅ REL \left(ESR1\right) + 0.527 ⋅ REL \left(MKI67\right) .$

In one embodiment, the su is calculated based on the relative expression levels of mRNA of ESR1 and MKI67, and a lower score su indicates a higher probability of pCR, and wherein su is calculated by using the formula: $su = - BASELINE + WF \left(ESR1\right) ⋅ REL \left(ESR1\right) - WF \left(MKI67\right) ⋅ REL \left(MKI67\right) ,$ wherein WF(ESR1) is a weighting factor for REL(ESR1), and WF(MKI67) is a weighting factor for REL(MKI67).

In one embodiment, the su is calculated based on the relative expression levels of mRNA of ESR1 and MKI67, and a lower score su indicates a higher probability of pCR, and wherein su is calculated by using the formula: $su = 10.625 + 0.324 ⋅ REL \left(ESR1\right) - 0.527 ⋅ REL \left(MKI67\right) .$

In one embodiment, the method further comprises:
calculating a predicted probability of pCR q, wherein
a) if a higher score su indicates a higher probability of pCR, q is calculated by using the formula: $q = \frac{exp \left(su\right)}{\left(1+exp\left(su\right)\right)} ;$ and
b) if a lower score su indicates a higher probability of pCR, q is calculated by using the formula $q = 1 - \frac{exp \left(su\right)}{\left(1+exp\left(su\right)\right)} ,$
wherein, preferably, a q which is equal to or greater than a pre-defined threshold indicates a high probability of pCR, and a q which is lower than a pre-defined threshold indicates a low probability of pCR.

In one embodiment, the method further comprises:
calculating a clinical score s based on su, wherein s has a scale from 0 to 100.

In one embodiment,
a) if a higher score su indicates a higher probability of pCR, a score s or a score su which is equal to or greater than a pre-defined threshold indicates a high probability of pCR, and a score s or a score su which is lower than the pre-defined threshold indicates a low probability of pCR; and
b) if a lower score su indicates a higher probability of pCR, a score s or a score su which is lower than a pre-defined threshold indicates a high probability of pCR, and a score s or a score su which is equal to or greater than the pre-defined threshold indicates a low probability of pCR.

In another aspect, the present invention relates to a method for selecting a breast cancer treatment for a breast cancer patient, said method comprising:
calculating a score unscaled (su) based on the relative expression levels of mRNA of ERBB2, ESR1, PGR and/or MKI67 in a pre-treatment breast tumor sample of the breast cancer patient as defined above, and, optionally, a predicted probability of pCR q as defined above, or a clinical score s as defined above; and
selecting a breast cancer treatment for the breast cancer patient based on su and, optionally, q or s, wherein
   a) if a higher score su indicates a higher probability of pCR,
      - neo-adjuvant chemotherapy is selected if su and, optionally, q or s are equal to or greater than a pre-defined threshold; and/or
      - a breast cancer treatment selected from the group consisting of adjuvant chemotherapy, a nonchemotherapeutic treatment and endocrine therapy is selected if su and, optionally, q or s are lower than the pre-defined threshold; and
   b) if a lower score su indicates a higher probability of pCR,
      - neo-adjuvant chemotherapy is selected if su and, optionally, s are lower than a pre-defined threshold;
      - neo-adjuvant chemotherapy is selected if q is equal to or greater than a pre-defined threshold;
      - a breast cancer treatment selected from the group consisting of adjuvant chemotherapy, a nonchemotherapeutic treatment and endocrine therapy is selected if su and, optionally, s are equal to or greater than the pre-defined threshold; and/or
      - a breast cancer treatment selected from the group consisting of adjuvant chemotherapy, a nonchemotherapeutic treatment and endocrine therapy is selected if q is lower than the pre-defined threshold,
wherein the method comprises, prior to calculating su and, optionally, q or s:
determining the relative expression levels of mRNA of ERBB2, ESR1, PGR and/or MKI67 in the pre-treatment breast tumor sample by RT-qPCR.

In one embodiment, the neo-adjuvant or adjuvant chemotherapy comprises administration of a taxane.

In one embodiment, the endocrine therapy is administered in an adjuvant or a neo-adjuvant setting.

In one embodiment, the neo-adjuvant chemotherapy or the endocrine therapy is accompanied by the administration of an anti-ERBB2 drug if the breast cancer is an ERBB2-positive breast cancer.

In one embodiment, the breast cancer is i) a luminal breast cancer, and/or ii) an ESR1- and/or PGR-positive breast cancer.

In one embodiment, if a higher score su indicates a higher probability of pCR, endocrine therapy is selected if su and, optionally, q or s are lower than the pre-defined threshold. In another embodiment, if a lower score su indicates a higher probability of pCR, endocrine therapy is selected if su and, optionally, s are equal to or greater than the pre-defined threshold, and/or if q is lower than the pre-defined threshold.

In one embodiment, the endocrine therapy is administered in a neo-adjuvant setting. In one embodiment, the endocrine therapy comprises administration of an aromatase inhibitor.

In one embodiment, the breast cancer is i) a luminal breast cancer, and ii) an ESR1- and/or PGR-positive breast cancer (e.g., luminal and ESR1- or PGR-positive), and the endocrine therapy is accompanied by the administration of an anti-ERBB2 drug and/or of a tyrosine kinase inhibitor (TKI), if the breast cancer is an ERBB2-positive breast cancer. In one embodiment, the anti-ERBB2 drug comprises a combination of trastuzumab and pertuzumab. In one embodiment, the TKI is selected from the group consisting of neratinib and lapatinib.

In one embodiment, the breast cancer is i) a luminal breast cancer, and ii) an ESR1- and/or PGR-positive breast cancer (e.g., luminal and ESR1- or PGR-positive), and the endocrine therapy is accompanied by the administration of a CDK4/6 inhibitor and/or of a Pi3KCa or mTOR inhibitor, if the breast cancer is an ERBB2-negative breast cancer. In one embodiment, the CDK4/6 inhibitor is selected from the group consisting of ribociclib and palbociclib. In one embodiment, the mTOR inhibitor is everolimus. In one embodiment, the pi3KCa inhibitor is alpelisib.

In another aspect, the present invention relates to a method of prognosis of breast cancer in a breast cancer patient upon breast cancer treatment, said method comprising:
calculating a score unscaled (su) based on the relative expression levels of mRNA of ERBB2, ESR1, PGR and/or MKI67 in a pre-treatment breast tumor sample of the breast cancer patient as defined above, and, optionally, a predicted probability of pCR q as defined above, or a clinical score s as defined above, wherein
a) if a higher score su indicates a higher probability of pCR, an su and, optionally, q or s which are equal to or greater than a pre-defined threshold indicate a negative prognosis, and/or an su and, optionally, q or s which are lower than a pre-defined threshold indicate a positive prognosis; and
b) if a lower score su indicates a higher probability of pCR, i) an su and, optionally, s which are equal to or greater than a pre-defined threshold indicate a positive prognosis, and/or an su and, optionally, s which are lower than a pre-defined threshold indicate a negative prognosis, and ii) a q which is equal to or greater than a pre-defined threshold indicates a negative prognosis, and/or a q which is lower than a pre-defined threshold indicates a positive prognosis,
wherein the method comprises, prior to calculating su and, optionally, q or s:
determining the relative expression levels of mRNA of ERBB2, ESR1, PGR and/or MKI67 in the pre-treatment breast tumor sample by RT-qPCR.

In one embodiment, the positive prognosis comprises an increased/high probability of distant recurrence-free survival (DRFS), disease-free survival (DFS) and/or overall survival (OS).

In one embodiment, the negative prognosis comprises a reduced/low probability of distant recurrence-free survival (DRFS), disease-free survival (DFS) and/or overall survival (OS).

In one embodiment, the breast cancer treatment comprises neo-adjuvant or adjuvant chemotherapy.

In one embodiment, the breast cancer treatment comprises adjuvant endocrine therapy.

In another aspect, the present invention relates to the use of a kit in a method as defined above, wherein the kit comprises:
at least one pair of ERBB2-specific primers;
at least one pair of ESR1-specific primers;
at least one pair of PGR-specific primers; and/or
at least one pair of MKI67-specific primers.

In one embodiment, the kit further comprises at least one ERBB2-specific probe, at least one ESR1-specific probe, at least one PGR-specific probe and/or at least one MKI67-specific probe.

In one embodiment, the kit further comprises at least one pair of reference gene-specific primers and, optionally, at least one reference gene-specific probe.

In one embodiment, the reference gene is selected from the group consisting of B2M, CALM2, TBP, PUM1, MRLP19, GUSB, RPL37A and CYFIP1.

Also disclosed herein is a method of predicting the probability of pathological complete response (pCR) of a breast cancer patient upon neo-adjuvant chemotherapy as defined above, a method for selecting a breast cancer treatment for a breast cancer patient as defined above, or a method of prognosis of breast cancer in a breast cancer patient upon breast cancer treatment as defined above, which is computer-implemented or partially computer-implemented.

Also disclosed herein is a data processing apparatus/device/system comprising means for carrying out the computer-implemented or partially computer-implemented method as defined above.

Also disclosed herein is a computer program comprising instructions which, when the program is executed by a computer, cause the computer to carry out the computer-implemented or partially computer-implemented method as defined above.

Also disclosed herein is a transitory or non-transitory, computer-readable data carrier having stored thereon the computer program as defined above.

### BRIEF DESCRIPTION OF THE FIGURES

**Figure 1** shows the distribution of predicted probabilities of pCR **(A)** and of clinical score values **(B)** in a full set of samples from the training cohort.
**Figure 2** shows the distribution of predicted probabilities of pCR **(A)** and of clinical score values **(B)** in samples from the neo-adjuvant chemotherapy study S080.
**Figure 3** shows the distribution of predicted probabilities of pCR **(A)** and of clinical score values **(B)** in a full set of samples from the 1^{st} endocrine study.
**Figure 4** shows the distribution of MammaTyper^{®} subtypes (13^{th} St Gallen guidelines) when splitting the training cohort into four quartiles based on the clinical score. **(A), (B), (C)** and **(D):** Proportions of MammaTyper^{®} subtypes in quartiles 1, 2, 3 and 4, respectively.
**Figure 5** shows the distribution of score 1 for each sample in the 3^{rd} neo-adjuvant cohort separated by MammaTyper^{®} subtypes.
**Figure 6** shows an ROC analysis for the prediction of pCR using the clinical score in the samples from the S080 study.
**Figure 7** shows an x/y-plot comparing the predicted probabilities of pCR based on a model generated from the Techno/Prepare cohort (x-axis) with the predicted pCR probability from the pre-defined model (y-axis). The pre-defined score is limited to values between 0 and 100, while the Techno/Prepare model is not.
**Figure 8** shows pCR rates in the Techno/Prepare cohorts according to the clinical score. Quartiles (Q 1-4) are pre-defined according to the training cohort. pCR rates are higher for small tumors (cT1 or cT2).
**Figure 9** shows the distribution of the clinical score according to subtypes as defined by MammaTyper^{®} (13^{th} St Gallen guidelines) in samples from the Techno/Prepare cohorts. Samples with a score below the pre-defined threshold 42 have a mean pCR rate of ~3%, while samples with a high score (≥42) have a mean pCR rate of ~25%.
**Figure 10** shows the distribution of the clinical score according to sample groups as defined by a combination of MammaTyper^{®} ESR1 and PGR (hormone receptors = HR) and ERBB2 (HER2) in samples from the Techno/Prepare cohorts. Samples with a score below the pre-defined threshold 42 have a mean pCR rate of ~3%, while samples with a high score (≥42) have a mean pCR rate of ~25%.
**Figure 11** shows a ROC curve of the continuous prediction score 1 for the prediction of pCR in the samples of the Techno/Prepare cohorts. The arrow which starts at 80% on the x-axis refers to 80% specificity, the arrow which ends at ~70% on the x-axis refers to 80% sensitivity, the arrow which ends at ~60% on the x-axis refers to the pre-defined Q2 threshold (CLASS1_42).
**Figure 12** shows a regression model as a function of the continuous score 1 estimating the likelihood of a pCR; the thick curve denotes the estimate, and the thin curves denote the 95%-confidence interval (pointwise for a fixed score value). The two arrows mark the thresholds corresponding to 10% and 20% predicted probability of pCR.
**Figure 13** shows a Kaplan Meier analysis of patients from the Techno/Prepare cohorts divided according to the score high/low result (threshold 42) in cT1-T2 tumors with 0-3 positive lymph nodes in patients who did not achieve a pCR. DFS = disease-free survival, DDFS = distant disease-free survival (also referred herein to as distant recurrence-free survival, DRFS), OS = overall survival. In all three Kaplan Meier plots, the upper line refers to patients with a low score result and the lower line refers to patients with a high score result (threshold ≥42). HR = hazard ratio.

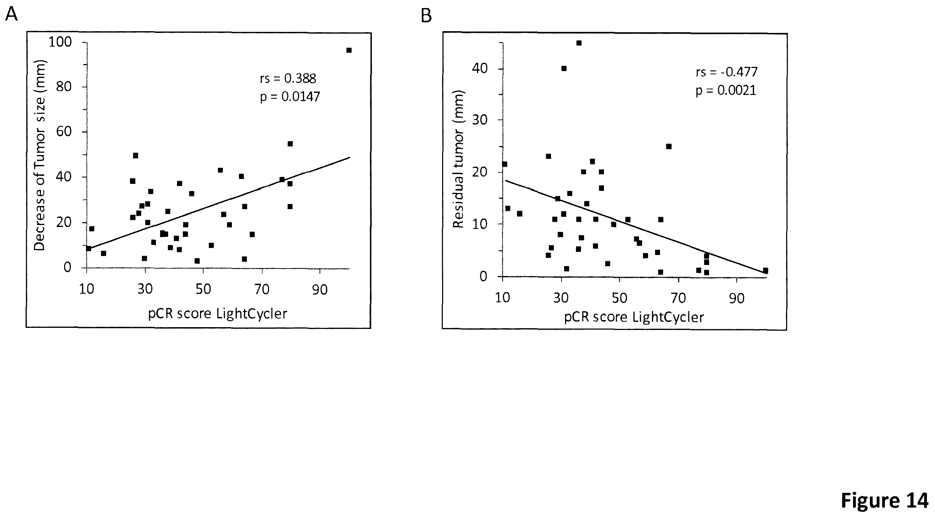

**Figure 14** shows a correlation analysis of the continuous pCR score (score 1) with decrease of tumor size **(A)** and residual tumor **(B)** after neo-adjuvant therapy in patients from a second validation cohort (Neo-Italy) who did not achieve a pCR (residual tumor remaining).

Other objects, advantages and features of the present invention will become apparent from the following detailed description, in particular when considered in conjunction with the accompanying figures.

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

In the following, certain elements of the present invention will be described. These elements may be listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 3rd Edition, J. Sambrook et al. eds., Cold Spring Harbor Laboratory Press, Cold Spring Harbor 2000).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Nothing herein is to be construed as an admission that the invention is not entitled to antedate such disclosure by virtue of prior invention.

In one aspect, the present invention relates to a method of predicting the probability of pathological complete response (pCR) of a breast cancer patient upon neo-adjuvant chemotherapy, said method comprising:
calculating a score unscaled (su) based on the relative expression levels of mRNA of
   i) ERBB2, ESR1, PGR and MKI67 or
   ii) ERBB2, ESR1, and MKI67 or
   iii) ESR1, and MKI67
      relative to the level of expression of one or more reference genes selected from the group comprising B2M, CALM2, TBP, PUM1, MRLP19, GUSB, RPL37A and CYFIP1 in a pre-treatment breast tumor sample of the breast cancer patient as determined by reverse transcription quantitative PCR (RT-qPCR), wherein
      a) a higher score su indicates a higher probability of pCR, wherein a higher expression level of mRNA of ERBB2 is associated with a higher su, a higher expression level of mRNA of ESR1 is associated with a lower su, a higher expression level of mRNA of PGR is associated with a lower su, and a higher expression level of mRNA of MKI67 is associated with a higher su; or
      b) a lower score su indicates a higher probability of pCR, wherein a higher expression level of mRNA of ERBB2 is associated with a lower su, a higher expression level of mRNA of ESR1 is associated with a higher su, a higher expression level of mRNA of PGR is associated with a higher su, and a higher expression level of mRNA of MKI67 is associated with a lower su,
wherein the method comprises, prior to calculating su:
   determining the relative expression levels of mRNA of
   iv) ERBB2, ESR1, PGR and MKI67,
   v) ERBB2, ESR1, and MKI67 or
   vi) ESR1, and MKI67, respectively,
in the pre-treatment breast tumor sample by RT-qPCR.

The term "breast cancer" relates to a type of cancer originating from breast tissue, most commonly from the inner lining of milk ducts or the lobules that supply the ducts with milk. Cancers originating from ducts are known as ductal carcinomas, while those originating from lobules are known as lobular carcinomas.

Occasionally, breast cancer presents as metastatic disease. Common sites of metastasis include bone, liver, lung and brain. Breast cancer occurs in humans and other mammals. While the overwhelming majority of human cases occur in women, male breast cancer can also occur. In one embodiment of the present invention, the breast cancer is primary breast cancer (also referred to as early breast cancer). Primary breast cancer is breast cancer that hasn't spread beyond the breast or the lymph nodes under the arm.

The term "tumor", as used herein, refers to all neoplastic cell growth and proliferation whether malignant or benign, and all pre-cancerous and cancerous cells and tissues. The terms "tumor" and "cancer" may be used interchangeably herein. In one embodiment of the present invention, the tumor is a solid tumor. Several molecular subtypes of breast cancer/tumors are known to the skilled person. The term "molecular subtype of a tumor" (or "molecular subtype of a cancer"), as used herein, refers to subtypes of a tumor/cancer that are characterized by distinct molecular profiles, e.g., gene expression profiles.

As disclosed herein, the molecular subtype is selected from the group comprising, preferably consisting of, ERBB2/HER2-positive, triple-negative (also referred to as "basal-like"), luminal A(-like) and luminal B(-like). The term "basal-like" refers to the fact that such tumors have some similarity in gene expression to that of basal epithelial cells. The term "luminal" derives from the similarity in gene expression between the tumors and the luminal epithelium. In one embodiment, the molecular subtype is selected from the group comprising, preferably consisting of, the molecular subtypes according to the 13^{th} St Gallen guidelines (Goldhirsch A. et al., 2013, Ann Oncol. 24(9):2206-2223), which are shown in below Table 1.

As disclosed herein, the molecular subtype is determined by immunohistochemistry (IHC) on the protein level and/or by RT-qPCR on the mRNA level, preferably exclusively on the mRNA level, e.g., as described in WO 2015/024942 A1. In one embodiment, the molecular subtype, e.g., the molecular subtype according to the 13^{th} St Gallen guidelines, is determined by means of the MammaTyper^{®} kit (BioNTech Diagnostics GmbH, Mainz, Germany; see also Laible M. et al., 2016, BMC Cancer 16:398), e.g., essentially as described in Example 2.

The term "ERBB2-positive breast cancer" (also referred to as "HER2-positive breast cancer") refers to a breast cancer with high expression levels of ERBB2, as determined by methods known in the art, e.g., by IHC and/or RT-qPCR.

The term "ESR1- and/or PGR-positive breast cancer" refers to breast cancer with expression of at least one of ESR1 and PGR, as determined by methods known in the art, e.g., by IHC and/or RT-qPCR. Such breast cancers may also be referred to as "hormone-receptor positive breast cancer".

The term "patient", as used herein, refers to a human or another mammal. Preferably, the patient is a human. Preferably, the patient is a female patient.

Pathological complete response (pCR; also referred to as pathological complete remission) generally refers to
1. the absence of residual invasive cancer based on hematoxylin and eosin evaluation of the complete resected breast specimen and all sampled regional lymph nodes, following completion of neo-adjuvant systemic therapy (i.e., ypT0/Tis ypN0 in the current AJCC staging system);
   or
2. the absence of residual invasive and in situ cancer based on hematoxylin and eosin evaluation of the complete resected breast specimen and all sampled regional lymph nodes following completion of neo-adjuvant systemic therapy (i.e., ypT0 ypN0 in the current AJCC staging system).

The term "treatment", in particular in connection with the treatment of cancer, as used herein, relates to any treatment which improves the health status and/or prolongs (increases) the lifespan of a patient. Said treatment may eliminate cancer, reduce the size or the number of tumors in a patient, arrest or slow the development of cancer in a patient, inhibit or slow the development of new cancer in a patient, decrease the frequency or severity of symptoms in a patient, and/or decrease recurrences in a patient who currently has or who previously has had cancer.

The term "breast cancer treatment", as used herein, may include surgery, medications (anti-hormonal/endocrine therapy and chemotherapy), radiation, immunotherapy/targeted therapy as well as combinations of any of the foregoing.

Endocrine therapy (also referred to as "anti-hormonal therapy" or "anti-hormone" therapy), as used herein, refers to treatment that blocks or removes hormones. Endocrine therapy targets cancers that require estrogen to continue growing by administration of drugs that either block/down-regulate estrogen and/or progesterone receptors, e.g., tamoxifen (Nolvadex^{®}) or fulvestrant (Faslodex^{®}), or alternatively block the production of estrogen with an aromatase inhibitor, e.g., anastrozole (Arimidex^{®}) or letrozole (Femara^{®}). Aromatase inhibitors, however, are only suitable for postmenopausal patients. This is because the active aromatase in postmenopausal women is different from the prevalent form in premenopausal women, and therefore these agents are ineffective in inhibiting the predominant aromatase of premenopausal women. In one embodiment, endocrine therapy comprises administration of an aromatase inhibitor. Aromatase inhibitors are especially well suited for neo-adjuvant endocrine therapy in postmenopausal patients for downstaging of tumors to enable breast conserving therapy.

Chemotherapy comprises the administration of chemotherapeutic agents. Chemotherapeutic agents or compounds according to the invention include cytostatic compounds and cytotoxic compounds. Traditional chemotherapeutic agents act by killing cells that divide rapidly, one of the main properties of most cancer cells. According to the invention, the term "chemotherapeutic agent" or "chemotherapeutic compound" includes taxanes, platinum compounds, nucleoside analogs, camptothecin analogs, anthracyclines and anthracycline analogs, etoposide, bleomycin, vinorelbine, cyclophosphamide, antimetabolites, antimitotics, and alkylating agents, including the agents disclosed above in connection with antibody conjugates, and combinations thereof. In one embodiment, the chemotherapy is platinum-based, i.e. comprises the administration of platinum-based compounds, e.g., cisplatin. According to the invention a reference to a chemotherapeutic agent is to include any prodrug such as ester, salt or derivative such as a conjugate of said agent. Examples are conjugates of said agent with a carrier substance, e.g., protein-bound paclitaxel such as albumin-bound paclitaxel. Preferably, salts of said agent are pharmaceutically acceptable. Chemotherapeutic agents are often given in combinations, usually for 3-6 months. One of the most common treatments is cyclophosphamide plus doxorubicin (adriamycin; belonging to the group of anthracyclines and anthracycline analogs), known as AC. Sometimes, a taxane drug, such as docetaxel, is added, and the regime is then known as CAT; taxane attacks the microtubules in cancer cells. Thus, in one embodiment, chemotherapy, e.g., neo-adjuvant chemotherapy, comprises administration of cyclophosphamide, an anthracycline and a taxane. Another common treatment, which produces equivalent results, is cyclophosphamide, methotrexate, which is an antimetabolite, and fluorouracil, which is a nucleoside analog (CMF). Another standard chemotherapeutic treatment comprises fluorouracil, epirubicin and cyclophosphamide (FEC), which may be supplemented with a taxane, such as docetaxel, or with vinorelbine.

As disclosed herein, the term "anti-ERBB2 drug", as used herein, refers to anti-ERBB2/HER2 antibodies, in particular monoclonal anti-ERBB2/HER2 antibodies. Monoclonal anti-ERBB2/HER2 antibodies include trastuzumab (Herceptin^{®}) and pertuzumab (Perjeta^{®}), which may be administered alone or in combination. A combination of trastuzumab and pertuzumab is also referred to as "dual blockade" of ERBB2/HER2. Trastuzumab is effective only in cancers where ERBB2/HER2 is overexpressed. Other monoclonal antibodies, such as ertumaxomab (Rexomun^{®}), are presently undergoing clinical trials. The anti-ERBB2/HER2 antibodies can further be modified to comprise a therapeutic moiety/agent, such as a cytotoxic agent, a drug (e.g., an immunosuppressant), a chemotherapeutic agent or a radionuclide, or a radioisotope. Thus, if the tumor treatment regimen comprises (a combination of) anti-ERBB2/HER2 therapy and chemotherapy, an anti-ERBB2/HER2 antibody conjugated to a chemotherapeutic agent may be used. A cytotoxin or cytotoxic agent includes any agent that is detrimental to and, in particular, kills cells. Examples include mertansine or emtansine (DM1), taxol, cytochalasin B, gramicidin D, ethidium bromide, emetine, mitomycin, etoposide, tenoposide, vincristine, vinblastine, colchicin, doxorubicin, daunorubicin, dihydroxy anthracin, dione, mitoxantrone, mithramycin, actinomycin D, amanitin, 1-dehydrotestosterone, glucocorticoids, procaine, tetracaine, lidocaine, propranolol, and puromycin and analogs or homologs thereof. In one embodiment, the antibody conjugate is trastuzumab (T)-DM1, e.g., trastuzumab emtansine. Other suitable therapeutic agents for forming antibody conjugates include, but are not limited to, antimetabolites (e.g., methotrexate, 6-mercaptopurine, 6-thioguanine, cytarabine, fludarabin, 5-fluorouracil decarbazine), alkylating agents (e.g., mechlorethamine, thioepachlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). In a preferred embodiment, the therapeutic agent is a cytotoxic agent or a radiotoxic agent. In another embodiment, the therapeutic agent is an immunosuppressant. In yet another embodiment, the therapeutic agent is GM-CSF. In another preferred embodiment, the therapeutic agent is doxorubicin, cisplatin, bleomycin, sulfate, carmustine, chlorambucil, cyclophosphamide or ricin A. Further therapeutic moieties include therapeutic moieties acting on mRNA and/or protein synthesis. Several inhibitors of transcription are known. For instance, actinomycin D, which is both a transcriptional inhibitor and a DNA damage agent, intercalates within the DNA and thus inhibits the initiation stage of transcription. Flavopiridol targets the elongation stage of transcription. alpha-Amanitin binds directly to RNA polymerase II, which leads to the inhibition of both initiation and elongation stages. Anti-ERBB2/HER2 antibodies also can be conjugated to a radioisotope, e.g., iodine-131, yttrium-90 or indium-111, to generate cytotoxic radiopharmaceuticals. In another embodiment, the term "anti-ERBB2 drug", as used herein, refers to small compounds targeting ERBB2/HER2, such as lapatinib (Tykerb^{®} or Tyverb^{®}), afatinib or neratinib.

Adjuvant therapy is a treatment that is given in addition to (i.e. after) the primary, main or initial treatment. An example of adjuvant therapy is the additional treatment (e.g., by chemotherapy) given after surgery (post-surgically), wherein, preferably, all detectable disease has been removed, but where there remains a statistical risk of relapse due to occult disease. Neo-adjuvant therapy is treatment given before the main treatment, e.g., chemotherapy before surgery (pre-surgical chemotherapy).

The term "mRNA" relates to "messenger RNA" and relates to a "transcript" which encodes a peptide or protein. mRNA typically comprises a 5' non-translated region (5'-UTR), a protein or peptide coding region and a 3' non-translated region (3'-UTR). mRNA has a limited halftime in cells and *in vitro.*

According to the present invention, the expression level of mRNA is determined by reverse transcription quantitative PCR (RT-qPCR). As RNA cannot be directly amplified in PCR, it must be reverse transcribed into cDNA using the enzyme reverse transcriptase. For this purpose, a one-step RT-qPCR can be utilized, which combines the reactions of reverse transcription with DNA amplification by PCR in the same reaction. In one-step RT-qPCR, the RNA template is mixed in a reaction mix containing reverse transcriptase, DNA polymerase, primers and probes, dNTPs, salts and detergents. In a first step, the target RNA is reverse transcribed by the enzyme reverse transcriptase using the target-specific reverse primers. Afterwards, the cDNA is amplified in a PCR reaction using the primers/probes and DNA polymerase.

As disclosed herein, the quantitative PCR is fluorescence-based quantitative real-time PCR, in particular fluorescence-based quantitative real-time PCR. The fluorescence-based quantitative real-time PCR comprises the use of a fluorescently labeled probe. Preferably, the fluorescently labeled probe consists of an oligonucleotide labeled with both a fluorescent reporter dye and a quencher dye (= dual-label probe). Suitable fluorescent reporter and quencher dyes/moieties are known to a person skilled in the art and include, but are not limited to the reporter dyes/moieties 6-FAM^{™}, JOE^{™}, Cy5^{®}, Cy3^{®} and the quencher dyes/moieties dabcyl, TAMRA^{™}, BHQ^{™}-1, -2 or -3. Amplification of the probe-specific product causes cleavage of the probe (= amplification-mediated probe displacement), thereby generating an increase in reporter fluorescence. The increase of fluorescence in the reaction is directly proportional to the increase of target amplificates. By using the LightCycler^{®} 480 II system (Roche Diagnostics) or the Versant kPCR system (Siemens) or the Mx3005P system (Agilent Technologies) or equivalent real-time instruments for detection of fluorescence originating from the probe, one can measure the increase in fluorescence in real-time. In one embodiment, the RT-qPCR is performed with a LightCycler^{®} 480 II system (Roche Diagnostics). In another embodiment, RT-qPCR is performed with a qPCR system other than a LightCycler^{®} 480 II system, and the results obtained with said system are mathematically transformed to correspond to the results obtained with the LightCycler^{®} 480 II system. Analysis output is a Cq value (Cq = quantification cycle) for each target gene/sequence. The Cq value (also referred to as cycle threshold (CT) value) is determined by the number of PCR amplification cycles, after which the fluorescence signal of the probe exceeds a certain background signal, wherein the Cq value is a measure for the amount of target molecules in the sample before the PCR amplification. Preferably, Cq values are further analyzed with appropriate software (e.g., Microsoft Excel^{™}) or statistical software packages (e.g., SAS JMP^{®} 9.0.0, GraphPad Prism4, Genedata Expressionist^{™}). The Cq value can either be converted to an absolute target molecule amount (e.g., ng/µl or molecules/µl) based on the Cq results of a standard curve with known target concentrations. Alternatively, the target amount can be reported as x-fold decreased or increased amount based on a reference (= ΔCq). Low ΔCq values (small difference) indicate higher amounts of target relative to the reference compared to high ΔCq (big difference). It is suitable to re-calculate the ΔCq by subtracting it from a fixed value (such as the number of PCR cycles, e.g., 40). The result is a value with direct correlation to target amount (high value = high amount) and expressed as 40-ΔCq values, wherein one integer refers to a doubling of the target amount (e.g., a value of 34 indicates an amount which is twice as much as that with a value of 33). Depending on the desired reproducibility and precision of the system, it is possible to panel multiple reference assays or to re-calculate/normalize the ΔCq of the sample with the ΔCq of a calibrator, resulting in a ΔΔCq value (1 point calibration; Pfaffl, 2001, Nucleic Acid Res. 29(9):e45). Preferably, Cq values are not transformed by any other mathematical operation which could skew the scale of the Cq values. By using different fluorophores for specific probes it is also possible to multiplex different target assays in the same reaction. During PCR, each target in the multiplex is amplified in parallel, but separately detected utilizing the different fluorescent emission.

As disclosed herein, the term "expression level of mRNA", as used herein, refers to the absolute expression level of mRNA, preferably given as Cq value. In one embodiment, the Cq value is used directly in calculations (e.g., subtraction from other Cq values) without prior normalization with one or more reference genes.

As disclosed herein, the term "expression level of mRNA", as used herein, refers to the relative expression level of mRNA.

As disclosed herein, the amplification efficiency of the qPCR is from 90% to 110%. Preferably, if the amplification efficiency of the qPCR is below 90% or above 110%, the respective Cq values are corrected in order to be in accordance with a 100% amplification efficiency.

Preferably, primers for use in accordance with the present invention have a length of 15 to 30 nucleotides, in particular deoxyribonucleotides. In one embodiment, the primers are designed so as to (1) be specific for the target mRNA-sequence (e.g., ERBB2, ESR1, PGR or MKI67), (2) provide an amplicon size of less than 150 bp (preferably less than 100 bp), (3) detect all known protein-encoding splicing variants, (4) not include known polymorphisms (e.g., single nucleotide polymorphisms, SNPs), (5) be mRNA-specific (consideration of exons/introns; preferably no amplification of DNA), (6) have no tendency to dimerize and/or (7) have a melting temperature Tₘ in the range of from 58°C to 62°C (preferably, Tₘ is approximately 60°C).

As used herein, the term "nucleotide" includes native (naturally occurring) nucleotides, which include a nitrogenous base selected from the group consisting of adenine (A), thymidine (T), cytosine (C), guanine (G) and uracil (U), a sugar selected from the group of ribose, arabinose, xylose, and pyranose, and deoxyribose (the combination of the base and sugar generally referred to as a "nucleoside"), and one to three phosphate groups, and which can form phosphodiester internucleosidyl linkages. Further, as used herein, "nucleotide" refers to nucleotide analogues. As used herein, "nucleotide analogue" shall mean an analogue of A, G, C, T or U (that is, an analogue of a nucleotide comprising the base A, G, C, T or U) which is recognized by DNA or RNA polymerase (whichever is applicable) and incorporated into a strand of DNA or RNA (whichever is appropriate). Examples of such nucleotide analogues include, without limitation, 5-propynyl pyrimidines (i.e., 5-propynyl-dTTP and 5-propynyl-dCTP), 7-deaza purines (i.e., 7-deaza-dATP and 7-deaza-dGTP), aminoallyl-dNTPs, biotin-AA-dNTPs, 2-amino-dATP, 5-methyl-dCTP, 5-iodo-dUTP, 5-bromo-dUTP, 5-fluoro-dUTP, N4-methyl-dCTP, 2-thio-dTTP, 4-thio-dTTP and alphathio-dNTPs. Also included are labelled analogues, e.g. fluorescent analogues such as DEAC-propylenediamine (PDA)-ATP, analogues based on morpholino nucleoside analogues as well as locked nucleic acid (LNA) analogues.

The wording "specific for the target mRNA-sequence", as used in connection with primers for use in accordance with the present invention, is meant to refer to the ability of the primer to hybridize (i.e. anneal) to the cDNA of the target mRNA-sequence under appropriate conditions of temperature and solution ionic strength, in particular PCR conditions. The conditions of temperature and solution ionic strength determine the stringency of hybridization. Hybridization requires that the two nucleic acids (i.e. primer and cDNA) contain complementary sequences, although depending on the stringency of the hybridization, mismatches between bases are possible. In one embodiment, "appropriate conditions of temperature and solution ionic strength" refer to a temperature in the range of from 58°C to 62°C (preferably a temperature of approximately 60°C) and a solution ionic strength commonly used in PCR reaction mixtures. In one embodiment, the sequence of the primer is 80%, preferably 85%, more preferably 90%, even more preferably 95%, 96%, 97%, 98%, 99% or 100% complementary to the corresponding sequence of the cDNA of the target mRNA-sequence, as determined by sequence comparison algorithms known in the art.

As disclosed herein, the primer hybridizes to the cDNA of the target mRNA-sequence under stringent or moderately stringent hybridization conditions. "Stringent hybridization conditions", as defined herein, involve hybridizing at 68°C in 5x SSC/5x Denhardt's solution/1,0% SDS, and washing in 0,2x SSC/0,1 % SDS at room temperature, or involve the art-recognized equivalent thereof (e.g., conditions in which a hybridization is carried out at 60°C in 2,5x SSC buffer, followed by several washing steps at 37°C in a low buffer concentration, and remains stable). "Moderately stringent hybridization conditions", as defined herein, involve including washing in 3x SSC at 42°C, or the art-recognized equivalent thereof. The parameters of salt concentration and temperature can be varied to achieve the optimal level of identity between the primer and the target nucleic acid. Guidance regarding such conditions is available in the art, for example, by J. Sambrook et al. eds., 2000, Molecular Cloning: A Laboratory Manual, 3rd Edition, Cold Spring Harbor Laboratory Press, Cold Spring Harbor; and Ausubel et al. eds., 1995, Current Protocols in Molecular Biology, John Wiley and Sons, N.Y.

Preferably, probes for use in accordance with the present invention have a length of 20 to 35 nucleotides, in particular deoxyribonucleotides. In one embodiment, the probes are designed so as to (1) be specific for the target mRNA-sequence (e.g., ERBB2, ESR1, PGR or MKI67), (2) not include known polymorphisms (e.g., single nucleotide polymorphisms, SNPs) and/or (3) have a melting temperature Tₘ, which is approximately 5°C to 8°C higher than the melting temperature Tₘ of the corresponding primer(s).

The wording "specific for the target mRNA-sequence", as used in connection with probes for use in accordance with the present invention, is meant to refer to the ability of the probe to hybridize (i.e. anneal) to the (amplified) cDNA of the target mRNA-sequence under appropriate conditions of temperature and solution ionic strength, in particular PCR conditions. The conditions of temperature and solution ionic strength determine the stringency of hybridization. Hybridization requires that the two nucleic acids (i.e. probe and cDNA) contain complementary sequences, although depending on the stringency of the hybridization, mismatches between bases are possible. In one embodiment, "appropriate conditions of temperature and solution ionic strength" refer to a temperature in the range of from 63°C to 70°C and a solution ionic strength commonly used in PCR reaction mixtures. In one embodiment, the sequence of the probe is 80%, preferably 85%, more preferably 90%, even more preferably 95%, 96%, 97%, 98%, 99% or 100% complementary to the corresponding sequence of the (amplified) cDNA of the target mRNA-sequence, as determined by sequence comparison algorithms known in the art.

In one embodiment, the probe hybridizes to the (amplified) cDNA of the target mRNA-sequence under stringent or moderately stringent hybridization conditions as defined above.

The probes as defined above are preferably labeled, e.g., with a label selected from a fluorescent label, a fluorescence quenching label, a luminescent label, a radioactive label, an enzymatic label and combinations thereof. Preferably, the probes as defined above are dual-label probes comprising a fluorescence reporter moiety and a fluorescence quencher moiety.

As disclosed herein, the expression level is normalized against the (mean) expression level of one or more reference genes in the sample of the tumor. The term "reference gene", as used herein, is meant to refer to a gene which has a relatively invariable level of expression on the RNA transcript/mRNA level in the system which is being examined, i.e. cancer. Such gene may be referred to as housekeeping gene. In one embodiment, the one or more reference genes are selected from the group comprising B2M, CALM2, TBP, PUM1, MRLP19, GUSB, RPL37A and CYFIP1. Other suitable reference genes are known to a person skilled in the art.

B2M refers to the gene of beta-2 microglobulin (UniProt: P61769), CALM2 refers to the gene of calmodulin-2 (UniProt: P0DP24), TBP refers to the gene of TATA-box-binding protein (UniProt: P20226), PUM1 refers to the gene of pumilio homolog 1 (UniProt: Q14671), MRLP19 refers to the gene of 39S ribosomal protein L19, mitochondrial (UniProt: P49406), GUSB refers to the gene of beta-glucuronidase (UniProt: P08236), RPL37A refers to the gene of Ribosomal Protein L37a (UniProt: P61513), and CYFIP1 refers to the gene of cytoplasmic FMR1-interacting protein 1 (UniProt: Q7L576).

As disclosed herein, the primers for use in accordance with the present invention are selected from primers as described in WO 2015/024942 A1 and/or WO 2016/131875 A1,

In one embodiment, the RT-qPCR is performed by means of the MammaTyper^{®} kit (BioNTech Diagnostics GmbH, Mainz, Germany; see also Laible M. et al., 2016, BMC Cancer 16:398), e.g., essentially as described in Example 2.

The term "relative expression level (REL)", as used herein, refers to the level of expression of a given marker gene (e.g., ERBB2, ESR1, PGR or MKI67) relative to the level of expression of one or more reference genes, e.g., one or more reference genes as defined herein. According to the present invention, the level of expression is determined on the mRNA level (transcriptional level) by RT-qPCR.

As disclosed herein, the relative expression level (REL) is given as ΔCq value which is calculated by subtracting the Cq value or mean/median Cq value of one or more reference genes from the Cq value or mean/median Cq value of the marker gene. In one embodiment, the ΔCq value is further normalized by subtracting from said ΔCq value the ΔCq value of a calibrator (e.g., a positive control, such as *in vitro* transcribed RNA of the marker gene), resulting in a ΔΔCq value.

As disclosed herein, the relative expression level (REL) for a given marker gene, i.e., REL(ERBB2), REL (ESR1), REL(PGR) or REL(MKI67), is given as a value selected from the group consisting of ΔCq value, ΔΔCq value, X-ΔCq value and X-ΔΔCq value, wherein, preferably, X is an integer, wherein, preferably, the integer is the number of PCR cycles of the RT-qPCR, e.g., 40. In one embodiment, REL is given as X-ΔΔCq value, e.g., 40-ΔΔCq value.

As disclosed herein, the ΔCq value is calculated as follows: Cq of the respective marker (e.g., ERBB2, ESR1, PGR and/or MKI67) of a patient sample - Cq of a reference gene (e.g., B2M and/or CALM2) of a patient sample (= calculation method 1). In one embodiment, the Cq is the median/mean Cq. If more than one reference gene is used, the ΔCq value is calculated as follows: Cq of the respective marker of a patient sample - mean/median Cq of selected reference genes of a patient sample) (= calculation method 2).

In one embodiment, the ΔΔCq is calculated as follows: ΔΔCq = (Cq marker of a patient sample - Cq marker of a reference sample) - (Cq reference gene of patient sample - Cq reference gene of a reference sample) (= calculation method 3).

As disclosed herein, the ΔΔCq value is calculated as follows: (Cq marker of a patient sample - Cq reference gene of the patient sample) - (Cq marker of a control sample - Cq reference gene of the control sample)] (= calculation method 4). In one embodiment, the Cq is the median/mean Cq. The Cq of the reference gene can be the Cq of a single reference gene or the mean Cq of two or more reference genes (referred to as mean/median CombRef). Preferably, the same control sample (also referred to as calibrator) is used in all analyses and leads to the same RT-qPCR or qPCR results. In one embodiment, the calibrator is a positive control (PC). In one embodiment, the control sample is a cell line RNA, an *in vitro* transcribed RNA or an equimolar mixture of DNA oligonucleotides, representing the marker mRNA or cDNA or the marker amplicon or a part of the marker amplicon with a constant ratio. In one embodiment, CALM2 and/or B2M are used as reference genes and a positive control, e.g., *in vitro* transcribed RNA, is used as control sample (calibrator).

The gene ERBB2 (also referred to as HER2; location: 17q12, annotation: chromosome: 17; NC_000017.10; UniProt: P04626) encodes a member of the epidermal growth factor (EGF) receptor family of receptor tyrosine kinases. Amplification and/or overexpression of this gene have been reported in numerous cancers, including breast and ovarian tumors. In the NCBI database, two mRNA variants for ERBB2 are listed which code for two protein versions. Protein and mRNA sequences can be found under the accession numbers NM_001005862.1 (receptor tyrosine-protein kinase erbB-2 isoform b) and NM_004448.2 (receptor tyrosine-protein kinase erbB-2 isoform a precursor).

The gene ESR1 (location: 6q25, annotation: chromosome 6, NC_000006.11; UniProt: P03372) encodes an estrogen receptor (ER), a ligand-activated transcription factor composed of several domains important for hormone binding, DNA binding, and activation of transcription. Estrogen receptors are known to be involved in pathological processes including breast cancer, endometrial cancer, and osteoporosis. Four ESR1 mRNA variants are known, wherein the transcript variants differ in the 5' UTR and/or use different promoters, but each variant codes for the same protein.

The gene PGR (also referred to as PR; location: 11q22-q23, annotation: chromosome: 11; NC_000011.9; UniProt: P06401) encodes the progesterone receptor. Steroid hormones such as progesterone and their receptors are involved in the regulation of eukaryotic gene expression and affect cellular proliferation and differentiation in target tissues. This gene uses two distinct promoters and translation start sites in the first exon to produce two mRNA isoforms, A and B. The two isoforms are identical except for the additional 165 amino acids found in the N-terminus of isoform B.

The gene MKI67 (also referred to as Ki67; location: 10q26.2, annotation: chromosome: 10; NC_000010.10; UniProt: P46013) encodes a nuclear protein that is associated with and may be necessary for cellular proliferation. Two mRNA variants have been described. A related pseudogene exists on chromosome 10.

As disclosed herein, the term "breast tumor sample" refers to a breast tumor tissue sample isolated from the cancer patient (e.g., a biopsy or resection tissue of the breast tumor). In a preferred embodiment, the breast tumor tissue sample is a cryo-section of a breast tumor tissue sample or is a chemically fixed breast tumor tissue sample. In a more preferred embodiment, the breast tumor tissue sample is a formalin-fixed and paraffin-embedded (FFPE) breast tumor tissue sample. In one embodiment, the sample of the breast tumor is (total) RNA extracted from the breast tumor tissue sample. In a particularly preferred embodiment, the sample of the breast tumor is (total) RNA extracted from a FFPE breast tumor tissue sample. In another embodiment, the breast tumor sample is a sample of one or more circulating tumor cells (CTCs) or (total) RNA extracted from the one or more CTCs. Those skilled in the art are able to perform RNA extraction procedures. For example, total RNA from a 5 to 10 µm curl of FFPE tumor tissue can be extracted using the High Pure RNA Paraffin kit (Roche, Basel, Switzerland), the XTRAKT RNA Extraction kit XL (Stratifyer Molecular Pathology, Cologne, Germany) or the RNXtract^{®} Extraction kit (BioNTech Diagnostics GmbH, Mainz, Germany). It is also possible to store the sample material to be used/tested in a freezer and to carry out the methods of the present invention at an appropriate point in time after thawing the respective sample material. A "pre-treatment" breast tumor sample is obtained from the breast cancer patient prior to initiation/administration of breast cancer treatment.

As disclosed herein, the method comprises, prior to calculating su:
determining the expression levels, preferably the relative expression levels, of mRNA of ERBB2, ESR1, PGR and MKI67
in the pre-treatment breast tumor sample by RT-qPCR.

As disclosed herein, no expression level, preferably no relative expression level, of mRNA of a gene other than ERBB2, ESR1, PGR and MKI67, and, optionally, one or more reference genes is determined.

In one embodiment, the neo-adjuvant chemotherapy comprises administration of a taxane.

In one embodiment, the neo-adjuvant chemotherapy is accompanied by the administration of an anti-ERBB2 drug if the breast cancer is an ERBB2-positive breast cancer.

In one embodiment, the breast cancer is i) a luminal breast cancer, and/or ii) an ESR1- and/or PGR-positive breast cancer.

In one embodiment, the su is calculated based on the relative expression levels of mRNA of ERBB2, ESR1, PGR and MKI67, and in the calculation of su, the relative expression levels (RELs) of mRNA of ERBB2, ESR1, PGR and MKI67 are weighted as follows: 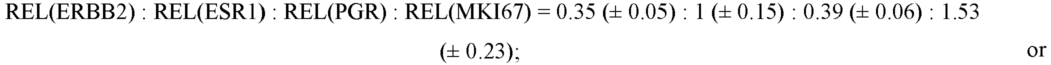 REL(ERBB2) : REL(ESR1) : REL(PGR) : REL(MKI67) = 0.35 (± 0.05) : 1 (± 0.15) : 0.39 (± 0.06) : 1.53 (± 0.23); or 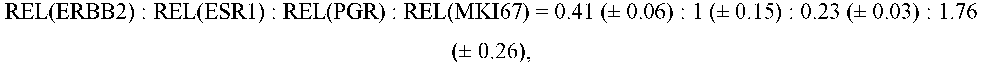 REL(ERBB2) : REL(ESR1) : REL(PGR) : REL(MKI67) = 0.41 (± 0.06) : 1 (± 0.15) : 0.23 (± 0.03) : 1.76 (± 0.26), the su is calculated based on the relative expression levels of mRNA of ERBB2, ESR1, and MKI67, and in the calculation of su, the relative expression levels (RELs) of mRNA of ERBB2, ESR1, and MKI67 are weighted as follows: $REL \left(ERBB2\right) : REL \left(ESR1\right) : REL \left(MKI67\right) = 0.34 \left(\pm 0.05\right) : 1 \left(\pm 0.15\right) : 1.61 \left(\pm 0.24\right) ,$ or the su is calculated based on the relative expression levels of mRNA of ESR1, and MKI67, and in the calculation of su, the relative expression levels (RELs) of mRNA of ESR1 and MKI67 are weighted as follows: $REL \left(ESR1\right) : REL \left(MKI67\right) = 1 \left(\pm 0.15\right) : 1.63 \left(\pm 0.24\right) .$

In one embodiment, the su is calculated based on the relative expression levels of mRNA of ERBB2, ESR1, PGR and MKI67, and a higher score su indicates a higher probability of pCR, and wherein su is calculated by using the formula: 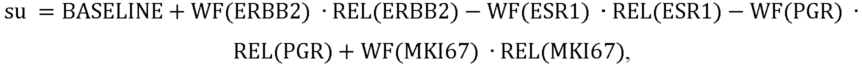 su = BASELINE + WF(ERBB2) · REL(ERBB2) - WF(ESR1) · REL(ESR1) - WF(PGR) · REL(PGR) + WF(MKI67) · REL(MKI67), wherein WF(ERBB2) is a weighting factor for REL(ERBB2), WF(ESR1) is a weighting factor for REL(ESR1), WF(PGR) is a weighting factor for REL(PGR2), and WF(MKI67) is a weighting factor for REL(MKI67).

In one embodiment, the su is calculated based on the relative expression levels of mRNA of ERBB2, ESR1, PGR and MKI67, and a higher score su indicates a higher probability of pCR, and wherein su is calculated by using the formula: 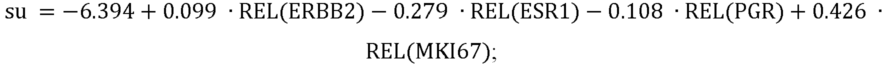 su = -6.394 + 0.099 · REL(ERBB2) - 0.279 · REL(ESR1) - 0.108 · REL(PGR) + 0.426 · REL(MKI67); or 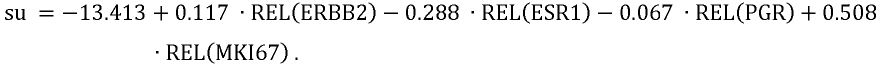 su = -13.413 + 0.117 · REL(ERBB2) - 0.288 · REL(ESR1) - 0.067 · REL(PGR) + 0.508 · REL(MKI67) .

In one embodiment, the su is calculated based on the relative expression levels of mRNA of ERBB2, ESR1, PGR and MKI67, and a lower score su indicates a higher probability of pCR, and wherein su is calculated by using the formula: 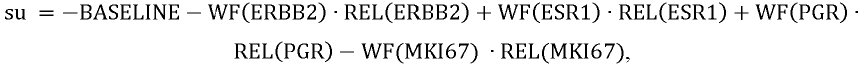 su = -BASELINE - WF(ERBB2) · REL(ERBB2) + WF(ESR1) · REL(ESR1) + WF(PGR) · REL(PGR) - WF(MKI67) · REL(MKI67), wherein WF(ERBB2) is a weighting factor for REL(ERBB2), WF(ESR1) is a weighting factor for REL(ESR1), WF(PGR) is a weighting factor for REL(PGR2), and WF(MKI67) is a weighting factor for REL(MKI67).

In one embodiment, the su is calculated based on the relative expression levels of mRNA of ERBB2, ESR1, PGR and MKI67, and a lower score su indicates a higher probability of pCR, and wherein su is calculated by using the formula: 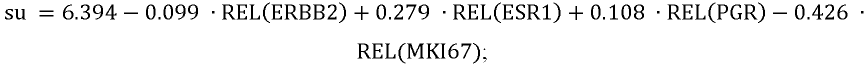 su = 6.394 - 0.099 · REL(ERBB2) + 0.279 · REL(ESR1) + 0.108 · REL(PGR) - 0.426 · REL(MKI67); or 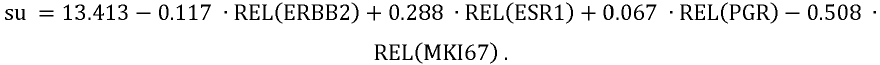 su = 13.413 - 0.117 · REL(ERBB2) + 0.288 · REL(ESR1) + 0.067 · REL(PGR) - 0.508 · REL(MKI67) .

In one embodiment, the method further comprises:
calculating a predicted probability of pCR q, wherein
a) if a higher score su indicates a higher probability of pCR, q is calculated by using the formula $q = \frac{exp \left(su\right)}{\left(1+exp\left(su\right)\right)} ;$ and
b) if a lower score su indicates a higher probability of pCR, q is calculated by using the formula $q = 1 - \frac{exp \left(su\right)}{\left(1+exp\left(su\right)\right)} ,$
wherein, preferably, a q which is equal to or greater than a pre-defined threshold indicates a high probability of pCR, and a q which is lower than a pre-defined threshold indicates a low probability of pCR.

In one embodiment, the method further comprises: calculating a clinical score s based on su, wherein s has a scale from 0 to 100.

In one embodiment, su is calculated based on the relative expression levels of mRNA of ERBB2, ESR1, PGR and MKI67 by using the formula 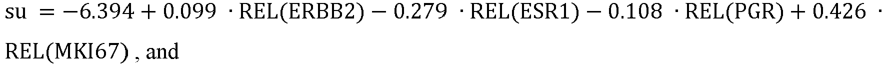 su = -6.394 + 0.099 · REL(ERBB2) - 0.279 · REL(ESR1) - 0.108 · REL(PGR) + 0.426 · REL(MKI67) , and wherein the method further comprises:
calculating a clinical score s based on su, wherein s is calculated by using the formula $s = \left(su+3.960\right) ⋅ 18.191 \left(round to 0 decimal places\right) ,$ wherein

| | | |
|---|---|---|
| if (su + 3.960) · 18.191 < 0 | s = 0, | and |
| if (su + 3.960) · 18.191 > 100 | s = 100. | |

In one embodiment,
a) if a higher score su indicates a higher probability of pCR, a score s or a score su which is equal to or greater than a pre-defined threshold indicates a high probability of pCR, and a score s or a score su which is lower than the pre-defined threshold indicates a low probability of pCR; and
b) if a lower score su indicates a higher probability of pCR, a score s or a score su which is lower than a pre-defined threshold indicates a high probability of pCR, and a score s or a score su which is equal to or greater than the pre-defined threshold indicates a low probability of pCR.

Suitable baselines for use in the formulae described herein as well as pre-defined thresholds/cut-offs, e.g., thresholds/cut-offs for dichotomization of pCR scores in "low probability of pCR" or "high probability of pCR" or prognostic thresholds/cut-offs, can be readily determined by the skilled person based on his or her general knowledge and the technical guidance provided herein (see Examples). For example, concordance studies in a training-testing setting can be used for the definition and validation of suitable thresholds/cut-offs. In one embodiment, the thresholds/cut-offs are defined based on one or more previous clinical studies. Moreover, additional clinical studies may be conducted for the establishment and validation of the thresholds/cut-offs. The thresholds/cut-offs may be determined/defined by techniques known in the art. In one embodiment, the thresholds/cut-offs are determined/defined on the basis of the data for pCR, overall survival (OS), disease-free survival (DFS), and/or distant recurrence-free survival (DRFS), in training cohorts by partitioning tests, ROC analyses or other statistical methods and are, preferably, dependent on a specific clinical utility (e.g., by using the SAS Software JMP^{®} 9.0.0).

The present invention further relates to a method of predicting the probability of pathological complete response (pCR) of a breast cancer patient upon neo-adjuvant chemotherapy, said method comprising:
calculating a score unscaled (su) based on the relative expression levels of mRNA of ERBB2, ESR1 and MKI67 relative to the level of expression of one or more reference genes selected from the group comprising B2M, CALM2, TBP, PUM1, MRLP19, GUSB, RPL37A and CYFIP1 in a pre-treatment breast tumor sample of the breast cancer patient as determined by reverse transcription quantitative PCR (RT-qPCR), wherein
a) a higher score su indicates a higher probability of pCR, wherein a higher expression level of mRNA of ERBB2 is associated with a higher su, a higher expression level of mRNA of ESR1 is associated with a lower su, and a higher expression level of mRNA of MKI67 is associated with a higher su; or
b) a lower score su indicates a higher probability of pCR, wherein a higher expression level of mRNA of ERBB2 is associated with a lower su, a higher expression level of mRNA of ESR1 is associated with a higher su, and a higher expression level of mRNA of MKI67 is associated with a lower su.
wherein the method comprises, prior to calculating su:
determining the expression levels, preferably the relative expression levels, of mRNA of ERBB2, ESR1 and MKI67
in the pre-treatment breast tumor sample by RT-qPCR.

As disclosed herein, no expression level, preferably no relative expression level, of mRNA of a gene other than ERBB2, ESR1 and MKI67, and, optionally, one or more reference genes is determined.

In one embodiment, the neo-adjuvant chemotherapy comprises administration of a taxane.

In one embodiment, the neo-adjuvant chemotherapy is accompanied by the administration of an anti-ERBB2 drug if the breast cancer is an ERBB2-positive breast cancer.

In one embodiment, the breast cancer is i) a luminal breast cancer, and/or ii) an ESR1- and/or PGR-positive breast cancer.

In one embodiment, in the calculation of su, the relative expression levels (RELs) of mRNA of ERBB2, ESR1, PGR and MKI67 are weighted as follows: $REL \left(ERBB2\right) : REL \left(ESR1\right) : REL \left(MKI67\right) = 0.34 \left(\pm 0.05\right) : 1 \left(\pm 0.15\right) : 1.61 \left(\pm 0.24\right) .$

In one embodiment, the su is calculated based on the relative expression levels of mRNA of ERBB2, ESR1 and MKI67, and a higher score su indicates a higher probability of pCR, and wherein su is calculated by using the formula: 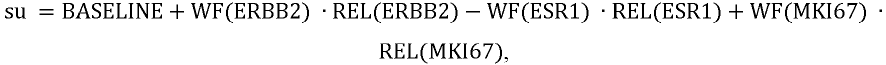 su = BASELINE + WF(ERBB2) · REL(ERBB2) - WF(ESR1) · REL(ESR1) + WF(MKI67) · REL(MKI67), wherein WF(ERBB2) is a weighting factor for REL(ERBB2), WF(ESR1) is a weighting factor for REL(ESR1), and WF(MKI67) is a weighting factor for REL(MKI67).

In one embodiment, the su is calculated based on the relative expression levels of mRNA of ERBB2, ESR1, and MKI67, and a higher score su indicates a higher probability of pCR, and wherein su is calculated by using the formula: $su = - 15.209 + 0.114 ⋅ REL \left(ERBB2\right) - 0.335 ⋅ REL \left(ESR1\right) + 0.539 ⋅ REL \left(MKI67\right) .$

In one embodiment, the su is calculated based on the relative expression levels of mRNA of ERBB2, ESR1, and MKI67, and a lower score su indicates a higher probability of pCR, and wherein su is calculated by using the formula: 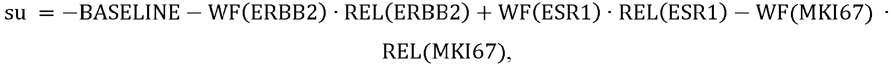 su = -BASELINE - WF(ERBB2) · REL(ERBB2) + WF(ESR1) · REL(ESR1) - WF(MKI67) · REL(MKI67), wherein WF(ERBB2) is a weighting factor for REL(ERBB2), WF(ESR1) is a weighting factor for REL(ESR1), and WF(MKI67) is a weighting factor for REL(MKI67).

In one embodiment, the su is calculated based on the relative expression levels of mRNA of ERBB2, ESR1, and MKI67, and a lower score su indicates a higher probability of pCR, and wherein su is calculated by using the formula: $su = 15.209 - 0.114 ⋅ REL \left(ERBB2\right) + 0.335 ⋅ REL \left(ESR1\right) - 0.539 ⋅ REL \left(MKI67\right) .$

In one embodiment, the method further comprises:
calculating a predicted probability of pCR q, wherein
a) if a higher score su indicates a higher probability of pCR, q is calculated by using the formula $q = \frac{exp \left(su\right)}{\left(1+exp\left(su\right)\right)} ;$ and
b) if a lower score su indicates a higher probability of pCR, q is calculated by using the formula $q = 1 - \frac{exp \left(su\right)}{\left(1+exp\left(su\right)\right)} ,$
wherein, preferably, a q which is equal to or greater than a pre-defined threshold indicates a high probability of pCR, and a q which is lower than a pre-defined threshold indicates a low probability of pCR.

In one embodiment, the method further comprises:
calculating a clinical score s based on su, wherein s has a scale from 0 to 100.

In one embodiment,
a) if a higher score su indicates a higher probability of pCR, a score s or a score su which is equal to or greater than a pre-defined threshold indicates a high probability of pCR, and a score s or a score su which is lower than the pre-defined threshold indicates a low probability of pCR; and
b) if a lower score su indicates a higher probability of pCR, a score s or a score su which is lower than a pre-defined threshold indicates a high probability of pCR, and a score s or a score su which is equal to or greater than the pre-defined threshold indicates a low probability of pCR.

The present invention further relates to a method predicting the probability of pathological complete response (pCR) of a breast cancer patient upon neo-adjuvant chemotherapy, said method comprising:
calculating a score unscaled (su) based on the relative expression levels of mRNA of ESR1 and MKI67 relative to the level of expression of one or more reference genes selected from the group comprising B2M, CALM2, TBP, PUM1, MRLP19, GUSB, RPL37A and CYFIP1 in a pre-treatment breast tumor sample of the breast cancer patient as determined by reverse transcription quantitative PCR (RT-qPCR), wherein
(i) a higher score su indicates a higher probability of pCR, wherein a higher expression level of mRNA of ESR1 is associated with a lower su, and a higher expression level of mRNA of MKI67 is associated with a higher su; or
(ii) a lower score su indicates a higher probability of pCR, wherein a higher expression level of mRNA of ESR1 is associated with a higher su, and a higher expression level of mRNA of MKI67 is associated with a lower su,
wherein the method comprises, prior to calculating su:
determining the expression levels, preferably the relative expression levels, of mRNA of ESR1 and MKI67
in the pre-treatment breast tumor sample by RT-qPCR.

As disclosed herein, no relative expression level, preferably no relative expression level, of mRNA of a gene other than ESR1 and MKI67, and, optionally, one or more reference genes is determined.

In one embodiment, the neo-adjuvant chemotherapy comprises administration of a taxane.

In one embodiment, the neo-adjuvant chemotherapy is accompanied by the administration of an anti-ERBB2 drug if the breast cancer is an ERBB2-positive breast cancer.

In one embodiment, the breast cancer is i) a luminal breast cancer, and/or ii) an ESR1- and/or PGR-positive breast cancer.

In one embodiment, in the calculation of su, the relative expression levels (RELs) of mRNA of ESR1 and MKI67 are weighted as follows: $REL \left(ESR1\right) : REL \left(MKI67\right) = 1 \left(\pm 0.15\right) : 1.63 \left(\pm 0.24\right) .$

In one embodiment, a higher score su indicates a higher probability of pCR, and wherein su is calculated by using the formula: $su = BASELINE - WF \left(ESR1\right) ⋅ REL \left(ESR1\right) + WF \left(MKI67\right) ⋅ REL \left(MKI67\right) ,$ wherein WF(ESR1) is a weighting factor for REL(ESR1), and WF(MKI67) is a weighting factor for REL(MKI67).

In one embodiment, the su is calculated based on the relative expression levels of mRNA of ESR1 and MKI67, and a higher score su indicates a higher probability of pCR, and wherein su is calculated by using the formula: $su = - 10.625 - 0.324 ⋅ REL \left(ESR1\right) + 0.527 ⋅ REL \left(MKI67\right) .$

In one embodiment, the su is calculated based on the relative expression levels of mRNA of ESR1 and MKI67, and a lower score su indicates a higher probability of pCR, and wherein su is calculated by using the formula: $su = - BASELINE + WF \left(ESR1\right) ⋅ REL \left(ESR1\right) - WF \left(MKI67\right) ⋅ REL \left(MKI67\right) ,$ wherein WF(ESR1) is a weighting factor for REL(ESR1), and WF(MKI67) is a weighting factor for REL(MKI67).

In one embodiment, the su is calculated based on the relative expression levels of mRNA of ESR1 and MKI67, and a lower score su indicates a higher probability of pCR, and wherein su is calculated by using the formula: $su = 10.625 + 0.324 ⋅ REL \left(ESR1\right) - 0.527 ⋅ REL \left(MKI67\right) .$

In one embodiment, the method further comprises:
calculating a predicted probability of pCR q, wherein
a) if a higher score su indicates a higher probability of pCR, q is calculated by using the formula: $q = \frac{exp \left(su\right)}{\left(1+exp\left(su\right)\right)} ;$ and
b) if a lower score su indicates a higher probability of pCR, q is calculated by using the formula $q = 1 - \frac{exp \left(su\right)}{\left(1+exp\left(su\right)\right)} ,$
wherein, preferably, a q which is equal to or greater than a pre-defined threshold indicates a high probability of pCR, and a q which is lower than a pre-defined threshold indicates a low probability of pCR.

In one embodiment, the method further comprises:
calculating a clinical score s based on su, wherein s has a scale from 0 to 100.

In one embodiment,
a) if a higher score su indicates a higher probability of pCR, a score s or a score su which is equal to or greater than a pre-defined threshold indicates a high probability of pCR, and a score s or a score su which is lower than the pre-defined threshold indicates a low probability of pCR; and
b) if a lower score su indicates a higher probability of pCR, a score s or a score su which is lower than a pre-defined threshold indicates a high probability of pCR, and a score s or a score su which is equal to or greater than the pre-defined threshold indicates a low probability of pCR.

In another aspect, the present invention relates to a method for selecting a breast cancer treatment for a breast cancer patient, said method comprising:
calculating a score unscaled (su) based on the expression levels, preferably the relative expression levels, of mRNA of ERBB2, ESR1, PGR and/or MKI67 in a pre-treatment breast tumor sample of the breast cancer patient as defined above, and, optionally, a predicted probability of pCR q as defined above, or a clinical score s as defined above; and
selecting a breast cancer treatment for the breast cancer patient based on su and, optionally, q or s, wherein
   a) if a higher score su indicates a higher probability of pCR,
      - neo-adjuvant chemotherapy is selected if su and, optionally, q or s are equal to or greater than a pre-defined threshold; and/or
      - a breast cancer treatment selected from the group consisting of adjuvant chemotherapy, a nonchemotherapeutic treatment and endocrine therapy is selected if su and, optionally, q or s are lower than the pre-defined threshold; and
   b) if a lower score su indicates a higher probability of pCR,
      - neo-adjuvant chemotherapy is selected if su and, optionally, s are lower than a pre-defined threshold;
      - neo-adjuvant chemotherapy is selected if q is equal to or greater than a pre-defined threshold;
      - a breast cancer treatment selected from the group consisting of adjuvant chemotherapy, a nonchemotherapeutic treatment and endocrine therapy is selected if su and, optionally, s are equal to or greater than the pre-defined threshold; and/or
      - a breast cancer treatment selected from the group consisting of adjuvant chemotherapy, a nonchemotherapeutic treatment and endocrine therapy is selected if q is lower than the pre-defined threshold,
wherein the method comprises, prior to calculating su and, optionally, q or s:
determining the relative expression levels of mRNA of ERBB2, ESR1, PGR and/or MKI67 in the pre-treatment breast tumor sample by RT-qPCR.

As disclosed herein, if a higher score su indicates a higher probability of pCR, the breast cancer patient is excluded from neo-adjuvant chemotherapy if su and, optionally, q or s are lower than the pre-defined threshold.

As disclosed herein, if a lower score su indicates a higher probability of pCR, the breast cancer patient is excluded from neo-adjuvant chemotherapy if su and, optionally, s are equal to or greater than the pre-defined threshold and/or if q is lower than the pre-defined threshold.

In one embodiment, the neo-adjuvant or adjuvant chemotherapy comprises administration of a taxane.

In one embodiment, the endocrine therapy is administered in an adjuvant or a neo-adjuvant setting.

In one embodiment, the neo-adjuvant chemotherapy or the endocrine therapy is accompanied by the administration of an anti-ERBB2 drug if the breast cancer is an ERBB2-positive breast cancer.

In one embodiment, the breast cancer is i) a luminal breast cancer, and/or ii) an ESR1- and/or PGR-positive breast cancer.

In one embodiment, if a higher score su indicates a higher probability of pCR, endocrine therapy is selected if su and, optionally, q or s are lower than the pre-defined threshold. In another embodiment, if a lower score su indicates a higher probability of pCR, endocrine therapy is selected if su and, optionally, s are equal to or greater than the pre-defined threshold, and/or if q is lower than the pre-defined threshold.

In one embodiment, the endocrine therapy is administered in a neo-adjuvant setting. In one embodiment, the endocrine therapy comprises administration of an aromatase inhibitor.

In one embodiment, the breast cancer is i) a luminal breast cancer, and ii) an ESR1- and/or PGR-positive breast cancer (e.g., luminal and ESR1- or PGR-positive), and the endocrine therapy is accompanied by the administration of an anti-ERBB2 drug and/or of a tyrosine kinase inhibitor (TKI), if the breast cancer is an ERBB2-positive breast cancer. In one embodiment, the anti-ERBB2 drug comprises a combination of trastuzumab and pertuzumab. In one embodiment, the TKI is selected from the group consisting of neratinib and lapatinib.

In one embodiment, the breast cancer is i) a luminal breast cancer, and ii) an ESR1- and/or PGR-positive breast cancer (e.g., luminal and ESR1- or PGR-positive), and the endocrine therapy is accompanied by the administration of a CDK4/6 inhibitor and/or of a Pi3KCa or mTOR inhibitor, if the breast cancer is an ERBB2-negative breast cancer. In one embodiment, the CDK4/6 inhibitor is selected from the group consisting of ribociclib and palbociclib. In one embodiment, the mTOR inhibitor is everolimus. In one embodiment, the pi3KCa inhibitor is alpelisib.

In another aspect, the present invention relates to a method of prognosis of breast cancer in a breast cancer patient upon breast cancer treatment, said method comprising:
calculating a score unscaled (su) based on the expression levels, preferably the relative expression levels, of mRNA of ERBB2, ESR1, PGR and/or MKI67 in a pre-treatment breast tumor sample of the breast cancer patient as defined above, and, optionally, a predicted probability of pCR q as defined above, or a clinical score s as defined above, wherein
a) if a higher score su indicates a higher probability of pCR, an su and, optionally, q or s which are equal to or greater than a pre-defined threshold indicate a negative prognosis, and/or an su and, optionally, q or s which are lower than a pre-defined threshold indicate a positive prognosis; and
b) if a lower score su indicates a higher probability of pCR, i) an su and, optionally, s which are equal to or greater than a pre-defined threshold indicate a positive prognosis, and/or an su and, optionally, s which are lower than a pre-defined threshold indicate a negative prognosis, and ii) a q which is equal to or greater than a pre-defined threshold indicates a negative prognosis, and/or a q which is lower than a pre-defined threshold indicates a positive prognosis,
wherein the method comprises, prior to calculating su and, optionally, q or s:
determining the relative expression levels of mRNA of ERBB2, ESR1, PGR and/or
MKI67 in the pre-treatment breast tumor sample by RT-qPCR.

In one embodiment, the positive prognosis comprises an increased/high probability of distant recurrence-free survival (DRFS), disease-free survival (DFS) and/or overall survival (OS).

In one embodiment, the negative prognosis comprises a reduced/low probability of distant recurrence-free survival (DRFS), disease-free survival (DFS) and/or overall survival (OS).

The term "recurrence" with respect to cancer includes re-occurrence of tumor cells at the same site and organ of the origin disease, metastasis that can appear even many years after the initial diagnosis and therapy of cancer, or to local events such as infiltration of tumor cells into regional lymph nodes. "Distant recurrence" refers to a scenario, where the cancer cells have spread (metastasized) to a distant part (i.e., another organ) of the body beyond the regional lymph nodes. Recurrence-free survival is generally defined as the time from randomization to the first of recurrence, relapse, second cancer, or death.

The term "metastasis" is meant to refer to the spread of cancer cells from their original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor at the target site depends on angiogenesis. Tumor metastasis often occurs even after the removal of the primary tumor because tumor cells or components may remain and develop metastatic potential.

In one embodiment, the breast cancer treatment comprises neo-adjuvant or adjuvant chemotherapy.

In one embodiment, the breast cancer treatment comprises adjuvant endocrine therapy.

As disclosed herein, the methods of the invention as defined above do not comprise any other diagnostic steps, such as histological tumor grading or determining the (axillary) lymph nodal status. In one embodiment, the methods do not comprise any steps involving immunohistochemistry (IHC).

As disclosed herein, the methods of the invention further comprise the consideration of one or more clinical factors, such as histological tumor grade, (axillary) lymph-nodal status, tumor size, age of the patient etc.

In another aspect, the present invention relates to the use of a kit in a method as defined above, wherein the kit comprises:
at least one pair of ERBB2-specific primers;
at least one pair of ESR1-specific primers;
at least one pair of PGR-specific primers; and/or
at least one pair of MKI67-specific primers.

In one embodiment, the kit comprises:
at least one pair of ESR1-specific primers; and
at least one pair of MKI67-specific primers.

In one embodiment, the kit comprises:
at least one pair of ERBB2-specific primers;
at least one pair of ESR1-specific primers; and
at least one pair of MKI67-specific primers.

In one embodiment, the kit further comprises at least one ERBB2-specific probe, at least one ESR1-specific probe, at least one PGR-specific probe and/or at least one MKI67-specific probe. In one embodiment, the kit comprises at least one ESR1-specific probe and at least one MKI67-specific probe. In one embodiment, the kit further comprises at least one ERBB2-specific probe, at least one ESR1-specific probe and at least one MKI67-specific probe. In one embodiment, the kit further comprises at least one ERBB2-specific probe, at least one ESR1-specific probe, at least one PGR-specific probe and at least one MKI67-specific probe.

In one embodiment, the kit further comprises at least one pair of reference gene-specific primers and, optionally, at least one reference gene-specific probe. In one embodiment, the reference gene is selected from the group consisting of B2M, CALM2, TBP, PUM1, MRLP19, GUSB, RPL37A and CYFIP1. In one embodiment, B2M and/or CALM2 are used as references genes.

Preferably, the primers and/or the probes are as defined further above. In one embodiment, the primers provide an amplicon size of less than 150 bp, preferably less than 100 bp. In one embodiment, detection of the probe is based on amplification-mediated probe displacement. In one embodiment, the probe is a dual-label probe comprising a fluorescence reporter moiety and a fluorescence quencher moiety.

As disclosed herein, the kit does not comprise any primers and/or probes that are specific for additional non-reference genes. In other words, no primers and/or probes specific for a gene other than ERBB2, ESR1, PGR and MKI67, and, optionally, one or more reference genes is comprised in the kit. In one embodiment, no primers and/or probes specific for a gene other than ERBB2, ESR1, MKI67, and, optionally, one or more reference genes is comprised in the kit. In another embodiment, no primers and/or probes specific for a gene other than ESR1 and MKI67, and, optionally, one or more reference genes is comprised in the kit.

As disclosed herein, the kit further comprises at least one control RNA sample. In one embodiment, the at least one control RNA sample is used as a positive control and/or a control sample (calibrator), wherein, preferably, the at least one control RNA sample comprises synthetic mRNA coding for one or more gene products (or parts thereof) of one or more genes selected from the group comprising ERBB2, ESR1, PGR, MKI67 and one or more reference genes. In one embodiment, the one or more reference genes are selected from the group consisting of B2M, CALM2, TBP, PUM1, MRLP19, GUSB, RPL37A and CYFIP1. In one embodiment, B2M and/or CALM2 are used as references genes.

As disclosed herein, the kit further comprises a reverse transcriptase and a DNA polymerase.As disclosed herein, the reverse transcriptase and the DNA polymerase are provided in the form of an enzyme-mix which allows a one-step RT-qPCR.

As disclosed herein, the kit may further comprise a DNase and a DNase reaction buffer.

As used herein, the term "kit of parts (in short: kit)" refers to an article of manufacture comprising one or more containers and, optionally, a data carrier. Said one or more containers may be filled with one or more of the above mentioned means or reagents. Additional containers may be included in the kit that contain, e.g., diluents, buffers and further reagents such as dNTPs. Said data carrier may be a non-electronical data carrier, e.g., a graphical data carrier such as an information leaflet, an information sheet, a bar code or an access code, or an electronical/computer-readable data carrier such as a compact disk (CD), a digital versatile disk (DVD), a microchip or another semiconductor-based electronical data carrier. The access code may allow the access to a database, e.g., an internet database, a centralized, or a decentralized database. Said data carrier may comprise instructions for the use of the kit in the methods of the invention. The data carrier may comprise threshold values or reference levels of (relative) expression levels of mRNA or of the scores calculated according to the methods of the present invention. In case that the data carrier comprises an access code which allows the access to a database, said threshold values or reference levels are deposited in this database. In addition, the data carrier may comprise information or instructions on how to carry out the methods of the present invention.

As disclosed herein, the kit is the MammaTyper^{®} kit (BioNTech Diagnostics GmbH, Mainz, Germany; see also Laible M. et al., 2016, BMC Cancer 16:398).

Disclosed herein is a method of predicting the probability of pathological complete response (pCR) of a breast cancer patient upon neo-adjuvant chemotherapy as defined above, a method for selecting a breast cancer treatment for a breast cancer patient as defined above, or a method of prognosis of breast cancer in a breast cancer patient upon breast cancer treatment as defined above, which is computer-implemented or partially computer-implemented.

The term "partially computer-implemented method" refers to a method in which only particular steps, e.g., calculating steps, are computer-implemented, whereas other steps of the method are not.

Disclosed herein is a data processing apparatus/device/system comprising means for carrying out the computer-implemented or partially computer-implemented method as defined above.

Disclosed herein is a computer program comprising instructions which, when the program is executed by a computer, cause the computer to carry out the computer-implemented or partially computer-implemented method as defined above.

Disclosed herein is a transitory or non-transitory, computer-readable data carrier having stored thereon the computer program as defined above.

The present disclosure provides, in particular, a method for predicting the pathological complete response (pCR) of a breast cancer after neo-adjuvant chemotherapy, in particular taxane-based chemotherapy, which, preferably, includes the administration of anti-ERBB2 drugs for ERBB2-positive breast cancers. The prediction is made based on a pre-treatment breast tumor sample, e.g., FFPE biopsy material. The present invention provides a gene expression profile algorithm/score which indicates the probability of pCR based on the expression of the mRNA markers ERBB2, ESR1, PGR and/or MKI67.

The present disclosure also provides cut-offs (also referred to as "thresholds" herein) based on which a breast cancer can be classified as having a high or low probability of pCR upon breast cancer treatment. In addition to such binary classification using a cut-off, for each sample the individual predicted probability of pCR can be calculated.

The method and algorithm/score provided by the present disclosure can also be applied to provide information on the prognosis of a breast cancer patient upon breast cancer treatment, e.g., neo-adjuvant chemotherapy or endocrine therapy only. The probability of pCR is a strong predictor of distant recurrence-free survival (DRFS), disease-free survival (DFS) and/or overall survival (OS). For example, patients who are likely to achieve a pCR should be treated with neo-adjuvant chemotherapy. However, for patients who will most likely not achieve a pCR it must be considered whether the other benefits of neo-adjuvant chemotherapy, such as a partial response, are important enough for choosing this treatment. In case where, based on the algorithm/score, a patient will most likely not achieve a pCR, adjuvant chemotherapy may be considered or the patient may generally be excluded from chemotherapy. While being applicable to all types of breast cancer, in particular primary breast cancer, the scores provided by the present invention show particular clinical utility within the subgroup of patients with luminal breast cancer and/or with ESR1-and/or PGR-positive breast cancer (ESR1- or PGR-positive; ERBB2/HER2-positive or -negative).

### EXAMPLES

### Example 1: Isolating total RNA from FFPE samples using the RNXtract^{®} protocol

Fixation of tumor tissue with formalin and subsequent embedding in paraffin is a standard method in clinical pathology and allows long-term archiving of samples. Because of chemical modifications of nucleic acids in FFPE samples, special protocols are necessary to extract amplifiable nucleic acids. Three steps are required for this: (1) removal of the paraffin, (2) lysis of the tissue and release of RNA (de-modification of nucleic acids if required), (3) purification of RNA by several washing steps.

The RNXtract^{®} kit (BioNTech Diagnostics GmbH, Mainz, Germany) allows purification without organic solvents, which can be conducted in a single reaction vessel.

In the first step, the paraffin contained in the FFPE sections is liquefied in an optimized lysis buffer. Subsequent addition of proteinase K leads to lysis of the tissue and release of cellular nucleic acids (RNA and DNA). The RNA is bound to magnetic particles, which are functionalized with germanium and allow a very efficient binding of RNA, within a binding buffer optimized for efficient enrichment of RNA. The RNA bound to magnetic particles is then washed in several increasingly stringent washing steps to ensure efficient removal of proteins and PCR-inhibiting substances, and is subsequently eluted in elution buffer. The eluate can be used directly in suitable molecular biological analyses such as reverse transcription, RT-qPCR, microarrays or NGS-applications. Quantification by RT-qPCR methods or UV/VIS spectrophotometry is possible. For use of RNXtract^{®} eluate in a MammaTyper^{®} RTqPCR (see below) no digestion of potential residual RNA is required.

### Example 2: Measuring the gene expression level of the biomarkers using the MammaTyper^{®} kit

The MammaTyper^{®} kit (BioNTech Diagnostics GmbH, Mainz, Germany) allows the determination of the level of expression of selected biomarkers at the mRNA level by means of reverse transcription quantitative PCR (RT-qPCR).

To determine the expression level of a biomarker at transcript level by PCR, RNA has first to be transcribed into complementary DNA (cDNA) via the enzyme reverse transcriptase (so-called first strand synthesis). The marker-specific cDNA is then amplified by a DNA polymerase and amplification is detected in the PCR in real time using fluorescently labeled hydrolysis probes. The RT-qPCR takes place as a one-step reaction in the MammaTyper^{®} assay, i.e., reverse transcription of the RNA and subsequent PCR of the DNA occur consecutively in the same reaction mixture. In addition to the enzymes (reverse transcriptase and DNA polymerase), the enzyme mix contains dNTPs as well as salts and PCR additives. For a MammaTyper^{®} RT-qPCR, the enzyme mix is supplemented with water, assay mix and the RNA sample.

In each of the three assay mixes, two assays (assay = primer pair and probe specific for the respective target sequence) are combined (= duplexed). Simultaneous detection of the two targets in the duplexed assays is realized using hydrolysis probes with different fluorophore-labeling; in each assay mix, detection is carried out using FAM in one assay and JOE in the other assay. Hydrolysis probes are modified with the respective fluorescent dye at the 5' end and a quencher at the 3' end. The quencher suppresses the fluorescence of the dye as long as it is in close proximity to the dye. In the course of amplification the probe binds to the target sequence. Due to the exonuclease activity of DNA polymerase, the bound probe is degraded and the dye and quencher are separated. The resulting fluorescence measured at the end of each cycle is directly proportional to the amount of synthesized product. In real-time PCR assays using hydrolysis probes, the number of PCR cycles required to obtain a fluorescence signal bigger than the background signal is used as a measure of the number of existing target molecules at the beginning of the reaction. The PCR cycle at which a signal can be detected above the background signal is referred to as the quantification cycle (Cq). In the relative expression analysis, the difference of the Cq values of the target assay and the reference assay (= ΔCq) is determined to compensate for variations in the amount of RNA starting material. In addition, the ΔCq value is offset against a calibrator to correct for inter-run and inter-instrument variations (ΔΔCq) for different instruments of one manufacturer.

Marker-specific primers and probes are selected in a way that no amplification and/or detection occurs without target gene RNA or with undesirable sequences or analytes (e.g., genomic DNA), whereas the target gene of interest is detected sensitively. Suitable primers are described, for example, in WO 2015/024942 A1 and/or WO 2016/131875 A1.

Using the MammaTyper^{®} kit at least one patient sample is analyzed per RT-qPCR run. Additionally, external controls are analyzed within each run, which determine the validity/invalidity of the run. For this purpose, a positive control RNA which also serves as calibrator (positive control = PC) and water (to prepare the reactions as well as a negative control = NC) are supplied in the MammaTyper^{®} kit. Each patient sample/control is analyzed with each assay mix (1, 2 and 3). The analysis is performed in triplicate resulting in 3 x 3 = 9 reactions per sample/control. Assay-mix 1 contains the assays for the biomarkers ERBB2 (FAM) and ESR1 (JOE), assay mix 2 contains the biomarker assay MKI67 (FAM) and reference assay B2M (JOE) and assay mix 3 contains the biomarker assay PGR (FAM) and reference assay CALM2 (JOE). The two reference assays are used to determine whether sufficient analyte (RNA) is present for an analysis of the patient sample. Invalid samples must not be used for calculation of results. For valid samples (sufficient RNA) the analysis starts with the calculation of the combined reference (CombRefSample, geometric mean value of the median Cq values of B2M and CALM2). The marker specific ΔCqSample value is then determined by subtraction of CombRefSample from the four median Cq values of the biomarkers ERBB2, ESR1, PGR and MKI67.

The resulting marker-specific ΔCq values are then corrected using the calibrator, by subtracting a calibrator ΔCqPC. The CombRefPC (CombRefPC, geometric mean value of the median Cq values of B2M and CALM2 of the positive control, PC) is subtracted from the respective marker Cq value of the positive control, to calculate the marker-specific calibrator.

This results in the ΔΔCq value: $ΔΔCq = ΔCqSample - ΔCqPC ,$ with $ΔCqSample = \left(Median Cq \left[MarkerSample\right]-\left[CombRefSample\right]\right) ,$ and $ΔCqPC = \left(Median Cq \left[MarkerPC\right]-\left[CombRefPC\right]\right) .$

The final results (40-ΔΔCq values) are obtained by subtracting the ΔΔCq values from the total number of PCR cycles (40), so that test results are positively correlated with marker expression, a format that facilitates interpretation for clinical decision making.

For tumor subtyping, the marker-specific 40-ΔΔCq values are dichotomized into "positive" or "negative" based on a clinically validated threshold value (cut-off). In addition, continuous values of each quantitative marker determination are reported. The combination of the four marker results (pos/neg) can then be used to determine the molecular subtype of the tumor sample (Table 1). For determination of a subtype, it is, therefore, necessary to analyze all three assay mixes in one run to obtain the four 40-ΔΔCq values of the sample.

**Table 1. Translation of MammaTyper^{®} single marker results into molecular subtypes according to the 13^{th} St Gallen guidelines (Goldhirsch A. et al., 2013, Ann Oncol. 24(9):2206-2223).**

| ***ERBB2*** | ***ESR1*** | ***PGR*** | ***MKI67*** | **St Gallen Subtype** |
|---|---|---|---|---|
| pos | pos | pos | pos | *Luminal B-like (HER2 positive)* |
| pos | pos | pos | neg | *Luminal B-like (HER2 positive)* |
| pos | pos | neg | pos | *Luminal B-like (HER2 positive)* |
| pos | pos | neg | neg | *Luminal B-like (HER2 positive)* |
| pos | neg | pos | pos | Not defined |
| pos | neg | pos | neg | Not defined |
| pos | neg | neg | pos | *HER2 positive (non-luminal)* |
| pos | neg | neg | neg | *HER2 positive (non-luminal)* |
| neg | pos | pos | pos | *Luminal B-like (HER2 negative)* |
| neg | pos | pos | neg | *Luminal A-like* |
| neg | pos | neg | pos | *Luminal B-like (HER2 negative)* |
| neg | pos | neg | neg | *Luminal B-like (HER2 negative)* |
| neg | neg | pos | pos | Not defined |
| neg | neg | pos | neg | Not defined |
| neg | neg | neg | pos | *Triple-negative (ductal)* |
| neg | neg | neg | neg | *Triple-negative (ductal)* |

### Example 3: Training of an unscaled score (score 1)

The unscaled score was trained on a set of routine FFPE biopsies from patients who received neo-adjuvant chemotherapy at the University Clinics of Erlangen (Germany) between 2000 and 2015. After selecting samples with sufficient tissue available for sectioning, a minimum of 20% tumor cell content and sufficient RNA for a MammaTyper^{®} test (valid result) a total of 598 samples were included into the study. The MammaTyper^{®} test (BioNTech Diagnostics GmbH, Mainz, Germany) was performed according to the manufacturer's instructions on RNA extracted from a 10µm curl from each sample using the nucleic acid isolation kit RNXtract^{®} according to the manufacturer's instructions. The MammaTyper^{®} measurements were performed on a LightCycler^{®} 480 II (Roche Diagnostics). The samples from the cohort also fulfilled these inclusion/exclusion criteria.

### Inclusion Criteria

- Female patients of the gynecology department of the University Clinics of Erlangen (Germany)
- Age: at least 18 years
- Diagnosed as invasive breast cancer and treated with neo-adjuvant chemotherapy between January 2008 and December 2014.
- Metastatic status: M0
- Neo-adjuvant chemotherapy containing anthracycline + cyclophosphamide and taxane (plus trastuzumab for ERBB2/HER2-positive patients) according to guideline recommendations followed by surgery followed by anti-hormone therapy, if the patient is hormone receptor-positive.
- Informed consent form (ICF) signed by the patients
- Information on the following parameters have to be available from the pre-treatment assessment:
   ▪ Patient's age
   ▪ Tumor size
   ▪ ER status (pos/neg and % positively stained cells)
   ▪ PR status (pos/neg and % positively stained cells)
   ▪ HER2 status (IHC score/ chromogenic in situ hybridization (CISH) amplification ratio)
   ▪ Ki-67 status (pos/neg and % positively stained cells)
   ▪ Histological tumor grade
   ▪ Axillary lymph-node status
   ▪ Follow-up information available for up to 10 years after the initial diagnosis regarding:
      - pCR (ypT0ypN0)
      - regression grading according to Sinn (22)
      - local recurrences
      - distant metastases
      - disease-specific survival (DSS) (if determined)
      - DDFS (or DRFS)
      - DFS
      - OS

### Exclusion Criteria

- Insufficient tissue material
- Secondary malignancies
- Suspicion of metastatic lesions at time of initial diagnosis

For generating the prediction score, the sample set was limited to the samples with full clinical information on age, body mass index (BMI), clinically determined tumor size (cT), clinically determined nodal status (cN) and tumor grading according to Elston and Ellis (N=462). The integration of the four biomarkers is done in a manner in which the two genes conferring tumor aggressiveness, ERBB2 and MKI67, lead to a higher score when expressed at higher levels, while the two genes ESR1 and PGR lead to a lower score when expressed higher.

The unscaled score was established using multivariable logistic regression with the four MammaTyper^{®} 40-ΔΔCq values for each sample as predictors and the occurrence of pCR (yes/no) as response. pCR was defined as (ypT0ypN0). The unscaled score derived from logistic regression was (su = score unscaled; REL(ERBB2), REL(ESR1), REL(PGR), REL(MKI67) = relative expression levels as determined with the MammaTyper^{®} kit in 40-ΔΔCq): 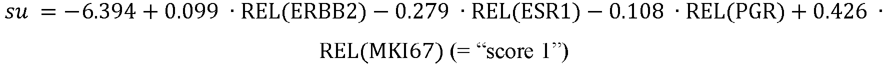 su = -6.394 + 0.099 · REL(ERBB2) - 0.279 · REL(ESR1) - 0.108 · REL(PGR) + 0.426 · REL(MKI67) (= "score 1")

The signs (+/-) can be exchanged in the entire formula, which yields a score correlated with a non-pCR rather than a pCR.

The result from the unscaled score can be interpreted the following way, wherein su = unscaled score: $Predicted probability of pCR = \frac{exp \left(su\right)}{\left(1+exp\left(su\right)\right)}$

The above score was trained on the data derived from a LightCycler^{®} 480 II qPCR instrument. To apply the score on data derived from a qPCR platform other than LightCycler^{®} 480 II, the 40-ΔΔCq values derived from such platform can be transformed into 40-ΔΔCq values as expected for this sample on the LightCycler^{®} 480 II system. This transformation of 40-ΔΔCq values can be done by using a linear equation, or by adding/subtracting a pre-defined Cq value to/from the respective 40-ΔΔCq values, thereby simulating LightCycler^{®} 480 II expression values. Another possible approach is to transfer the score to the other platform. In a dataset in which the same samples were measured with the LightCycler^{®} 480 II system and the other platform, the 40-ΔΔCq values from the other platform can be used as predictors to determine the pCR score which is calculated from the 40-ΔΔCq values determined on the LightCycler^{®} 480 II system for the same samples using linear regression analysis.

### Example 4: Development of a clinical score and thresholds

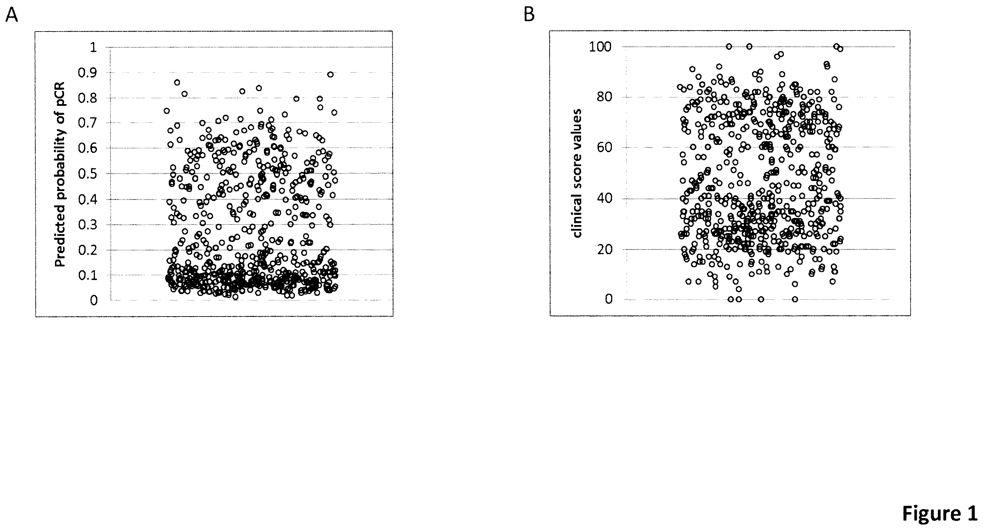

To enable applicability of the established score (score 1; see Example 3) in clinical routine the unscaled score was transformed to fit a range between 0 and 100. This format allows a better interpretability in everyday practice. The upper and lower borders of the score (0 and 100) were set using the distribution of unscaled score values in the full set of samples with valid MammaTyper^{®} results of the training cohort **(****Figure 1****),** wherein the 0.5% and 99.5% percentiles were used as minimum (0) and maximum (100) values. Applying this approach the formulas for the rescaled/clinical routine score "s" is (su = unscaled score):

| | |
|---|---|
| *if* (*su* + 3.960) · 18.191 < 0 | s = 0 |
| *if* (*su* + 3.960) · 18.191 > 100 | s = 100 |
| *otherwise* | *s* = (*su* + 3.960) · 18.191 (*round to* 0 *decimal places*) |

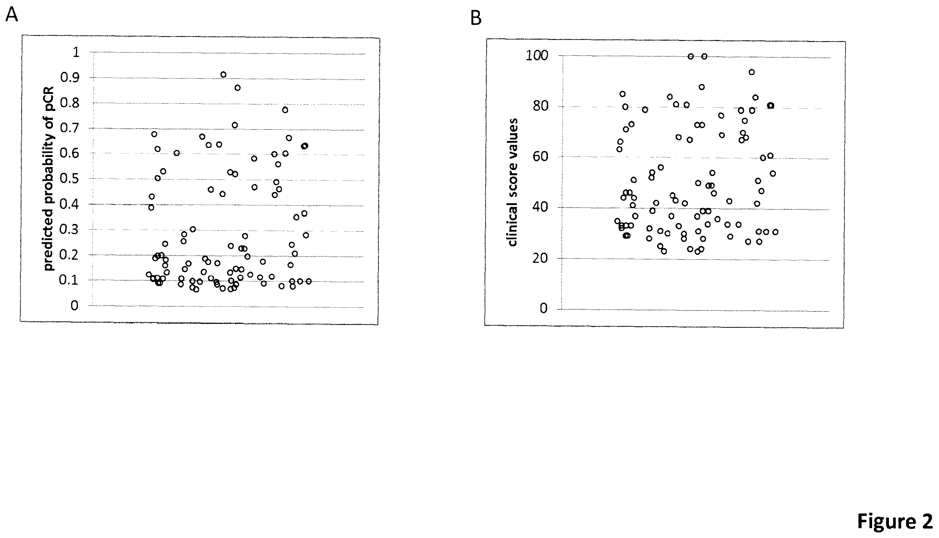

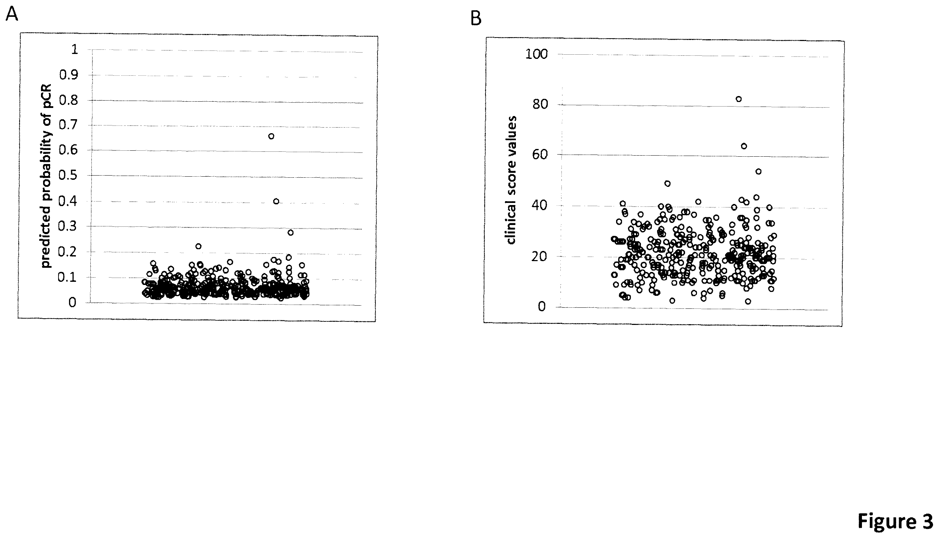

Clinical meaningfulness of the score is also reflected by the distribution of values in two other cohorts analyzed with the MammaTyper^{®} assay: the S080 study (ClinicalTrials.gov Identifier: NCT00149214) and the 1^{st} MammaTyper^{®} endocrine study - see **Figure 2** and **Figure 3****,** respectively. The S080 study represents a high risk cohort of patients treated by neo-adjuvant chemotherapy and the 1^{st} MammaTyper^{®} endocrine study represents a low risk cohort of patients who received endocrine therapy only. As the predicted probability to achieve a pCR is correlated with the aggressiveness of the tumor, high pCR scores represent aggressive tumors and low pCR scores represent tumors with lower risk of recurrence.

### Establishment of thresholds

Clinical decision making using the herein described scores can be based on the predicted probabilities of pCR which can be determined for each sample and also based on a binary output (high/low predicted probability of pCR) using a decision threshold. Several decision thresholds were established according to different rationales. These thresholds were validated in the Techno/Prepare cohorts (see Example 7 below).

**Table 2. Samples are classified into "low" and "high" predicted probability of pCR based on the indicated cut-off values for the score. The 25% quantile was used for descriptive analysis and marks a group of tumors with especially low probability of pCR.**

| **Thresholds for separation of high and low responders** | **Value** |
|---|---|
| 25% quantile from training cohort (Q1) | <27 /≥ 27 |
| 50% quantile from training cohort (Q2) | <42 /≥ 42 |
| 75% quantile from training cohort (Q3) | <69 /≥ 69 |
| threshold corresponding to 20% predicted probability of pCR in training cohort | <47 /≥ 47 |
| threshold corresponding to 90% sensitivity in S080 | <50 /≥ 50 |
| threshold corresponding to 10% predicted probability of pCR in Techno/Prepare | <46 /≥ 46 |
| threshold corresponding to 20% predicted probability of pCR in Techno/Prepare | <64 /≥ 64 |
| 50% quantile in *HER2 positive (non-luminal)* samples in training cohort | <74/≥74 |
| 50% quantile in *luminal B-like (HER2 positive)* samples in training cohort | <43/≥43 |

Applying the quartiles from the training study as thresholds, a clinical meaningful separation of breast cancer subtypes can be seen.

**Table 3. Distribution of MammaTyper^{®} luminal B-like samples in the 3^{rd} neo-adjuvant study throughout the 4 quartiles of clinical score.**

| **MammaTyper^{®} subtype** | **Samples in 1st quartile** | **Samples in 2nd quartile** | **Samples in 3rd quartile** | **Samples in 4th quartile** |
|---|---|---|---|---|
| Luminal B-like (HER2 positive) | 20% | 28% | 52% | 0% |
| Luminal B-like (HER2 negative) | 33% | 52% | 15% | 0% |

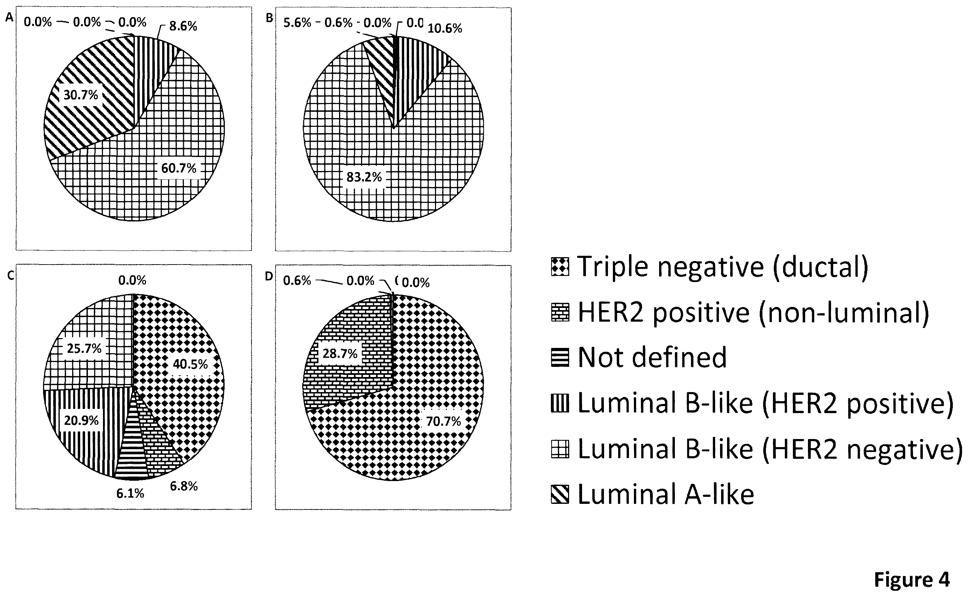

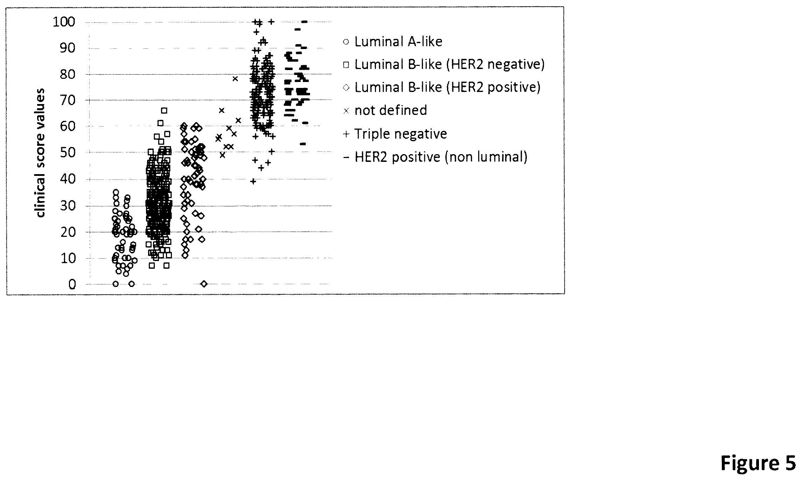

The results are shown in **Figure 4** and **Figure 5****.**

### Example 5: Validation of score 1 in an independent cohort (S080)

The first set of samples used for validation of score 1 (Examples 3 and 4) were routine FFPE biopsies taken from patients enrolled in the S080 neo-adjuvant chemotherapy trial (ClinicalTrials.gov Identifier: NCT00149214). From a 10µm curl prepared from each sample, total RNA was extracted using the RNXtract^{®} kit (see Example 1). Total RNA was then subjected to the MammaTyper^{®} RT-qPCR test (see Example 2) for relative quantification of the four breast cancer markers HER2/ERBB2, ER/ESR1, PgR/PGR and Ki67/MKI67 on the mRNA level on a LightCycler^{®} 480 II.

Valid MammaTyper^{®} results and information on achievement of pCR (yes/no) could be obtained from 91 of 105 included samples.

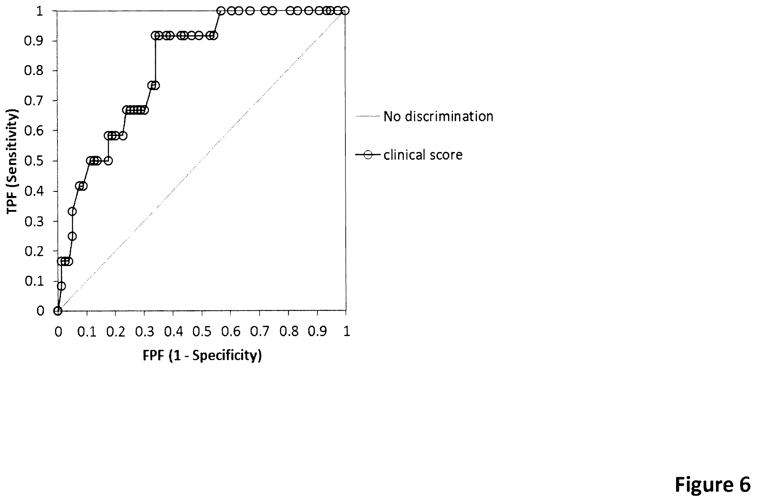

ROC analysis of the score in this independent cohort resulted in a high AUC value which reflects a high predictive power of the score **(****Figure 6****).**

**Table 4. AUC values from ROC analysis of the three scores in the samples from the S080 study.**

| **Predictor** | **AUC** | **95% CI of AUC** | |
|---|---|---|---|
| Clinical score | 0.813 | 0.701 | to 0.925 |

### Example 6: Prognostic information in an endocrine cohort

To analyze if score 1 (Examples 3 and 4) also contains prognostic information for patients treated with endocrine therapy only, a ROC analysis of the clinical score for prediction of a distant event in the 1^{st} endocrine study was performed. For comparison an optimal score for such a prediction of a distant event was generated on the full dataset (best fit).

The AUC achieved in this analysis demonstrates the ability of the score to also predict distant recurrence in adjuvant endocrine treatment setting. Of note, the clinical score which was found by an independent approach (logistic fit against pCR (yes/no)) performs almost as good (similar AUC) when applied to the prognosis as a score which represents the best fit on the 1^{st} endocrine study data.

**Table 5. AUC values from ROC analysis of the clinical score and a reference score (best fit on the full dataset) for detection of a distant event (metastasis) in the samples from the 1^{st} endocrine study.**

| **Predictor** | **AUC** | **95% CI of AUC** | | **p-value** |
|---|---|---|---|---|
| Clinical score | 0.653 | 0.549 | to 0.756 | 0.0019 |
| Best logistic fit | 0.675 | 0.573 | to 0.777 | 0.0004 |

### Example 7: Second validation of score 1 in an independent cohort (Techno/Prepare)

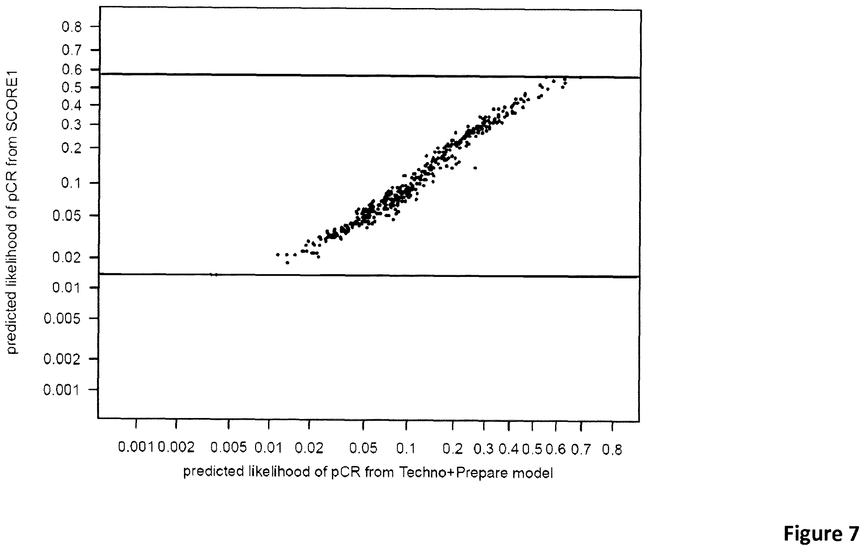

The pCR prediction score was validated in a retrospective analysis of FFPE biopsy samples from patients treated by neo-adjuvant chemotherapy (+/- anti-ERBB2/HER2 therapy) during the Techno/Prepare trials (ClinicalTrials.gov Identifiers NCT00795899 and NCT00544232, respectively). To validate the universality of the actual score formula, a comparison of the predicted probabilities of pCR for each sample determined once using the pre-defined score and once using a score generated independently in the Techno/Prepare cohort was performed. By plotting these two predicted probabilities of pCR in an x/y-plot, it can be nicely seen that the optimal prediction based on the Techno/Prepare cohorts matches well with the predicted probabilities of pCR by the pre-defined score (Figure 7).

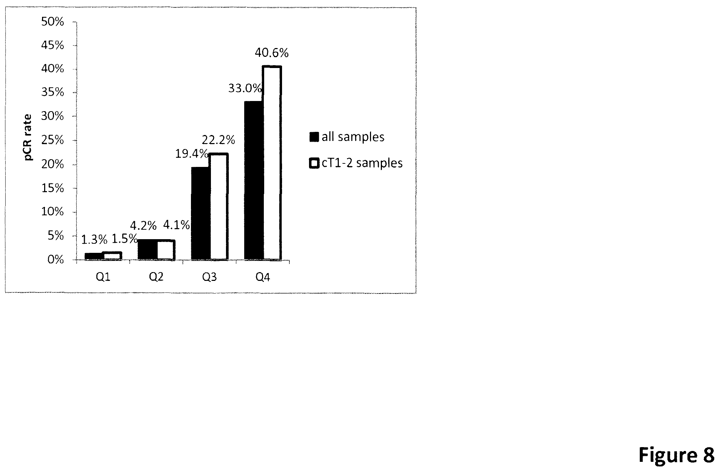

The comparison of pCR rates in groups of samples divided according to pre-defined quartile thresholds from the training cohort shows a clear association of the score and the pCR rate, wherein the pCR rate in the two lower quartiles can be seen as low and the pCR rate in two upper quartiles can be seen as high **(****Figure 8****).**

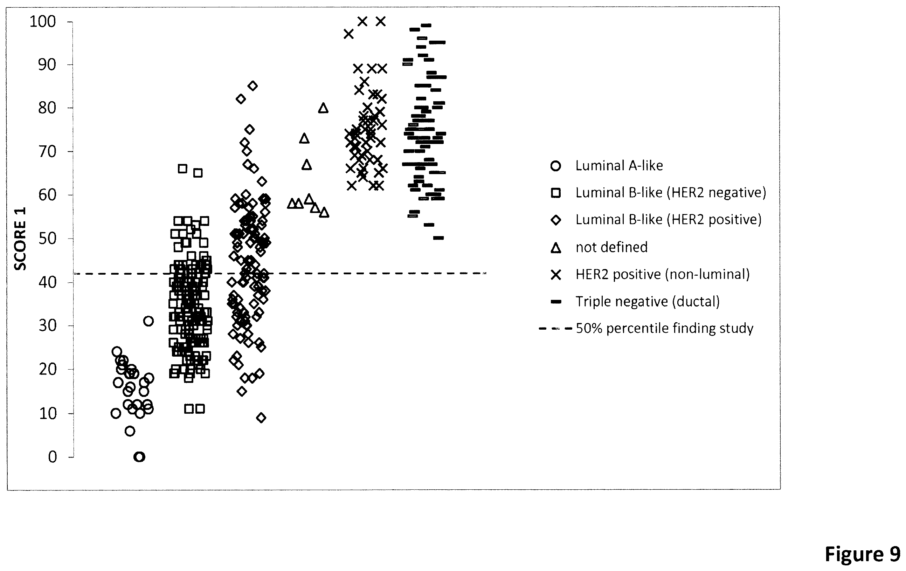

**Table 6. Distribution of samples according to MammaTyper^{®} subtypes (St Gallen 2013 guidelines) over the low and high pCR rate zone (see also Figure 9).**

| | ***Luminal A-like*** | ***Luminal B-like (HER2 negative)*** | ***Luminal B-like (HER2 positive)*** | ***not defined*** | ***HER2 positive (non-luminal)*** | ***Triple negative (ductal)*** |
|---|---|---|---|---|---|---|
| **Zone 1 (3% pCR)** | 14% | 63% | 24% | 0% | 0% | 0% |
| **Zone 2 (25% pCR)** | 0% | 17% | 25% | 4% | 22% | 33% |

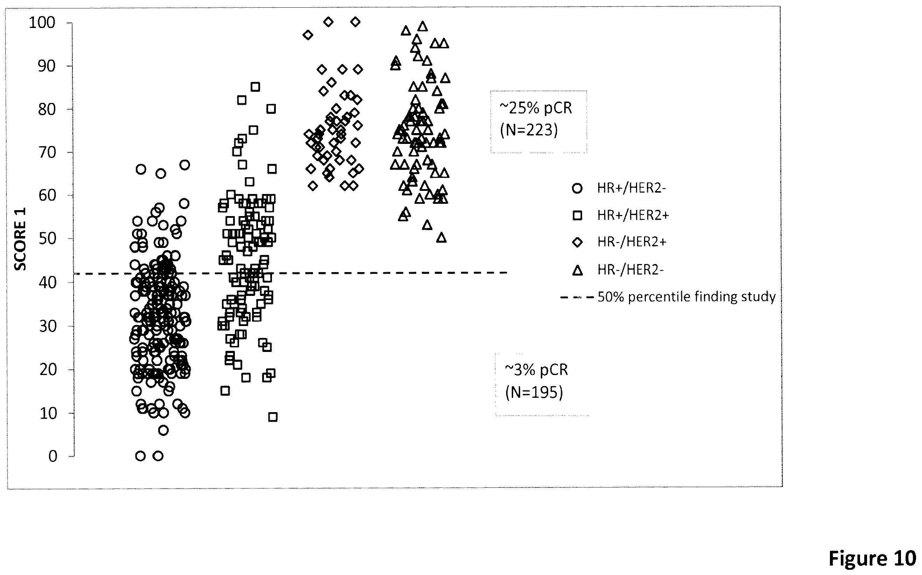

**Table 7. Distribution of samples according to sample groups as defined in Figure 10 over the low and high pCR rate zone.**

| | **HR+/HER2-** | **HR+/HER2+** | **HR-/HER2+** | **HR-/HER2-** |
|---|---|---|---|---|
| **Zone 1 (3% pCR)** | 76% | 24% | 0% | 0% |
| **Zone 2 (25% pCR)** | 18% | 26% | 22% | 33% |

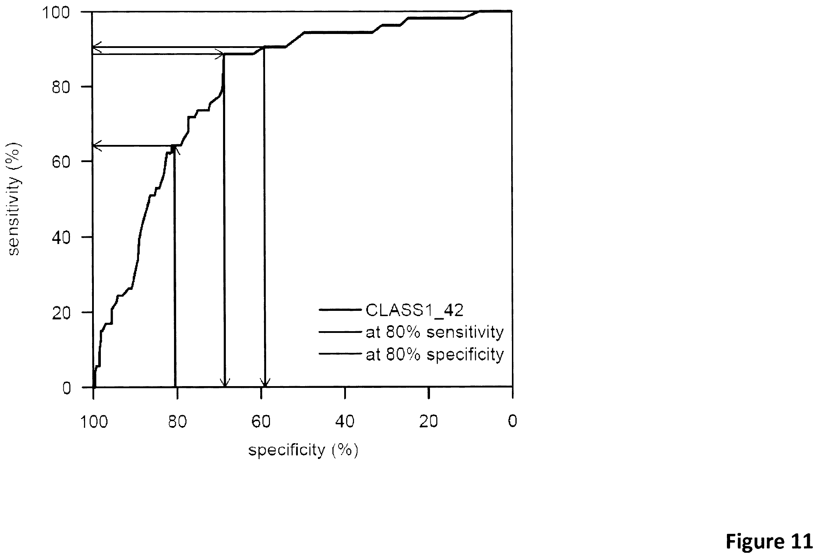

When analyzing the score over all samples of the Techno/Prepare cohorts using the continuous score, a high AUC could be demonstrated illustrating the high predictive power of the score **(****Figure 11****).**

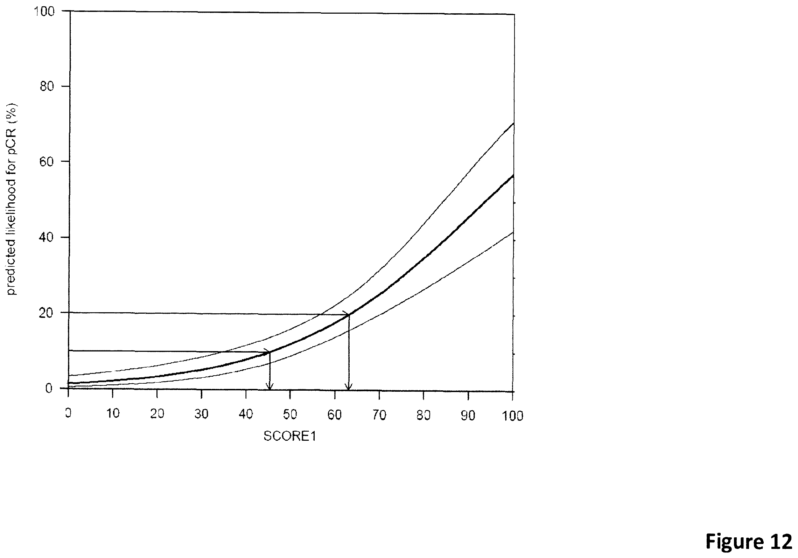

The clinical score was also applied in a regression model to patients of the Techno/Prepare cohorts in order to obtain a function of the continuous score 1 estimating the likelihood of a pCR and the corresponding 95%-confidence interval **(****Figure 12****).**

**Table 8. Statistical analysis of predictive power of the continuous clinical score in the samples from Techno/Prepare.**

| | | |
|---|---|---|
| samples | n | 324 |
| logistic model | convergence | yes |
| | (unit) odds ratio | 1.055 (1.038 .. 1.072) |
| | (10-units) odds ratio | 1.711 (1.457 .. 2.009) |
| | (100-units) odds ratio | 214.975 (43.049 .. 1073.523) |
| | p(Wald) | <.0001 |
| at 80% sensitivity | threshold | <49 / >=49 |
| | sensitivity | 88.7% |
| | specificity | 68.6% |
| at 80% specificity | threshold | <63 / >=63 |
| | sensitivity | 64.2% |
| | specificity | 80.4% |
| ROC analysis | number of distinct levels | 87 |
| | estimated AUC | 0.805 (0.747 .. 0.864) |
| | std. dev. of AUC | 0.030 |
| | p(AUC < 0.5) | <.0001 |

Also the binary use of the score (high/low) yields highly significant predictive power in this cohort which is even maintained when taking additional known predictors of pCR into account.

**Table 9. Univariate statistical analysis of predictive power of the clinical score applied in a binary manner (high/low) in the samples from Techno/Prepare.**

| | |
|---|---|
| n | 324 |
| logistic model | calculated |
| odds ratio | 13.838 (5.339 .. 35.864) |
| p(Wald) | <.0001 |
| sensitivity | 90.6% (79.3% .. 96.9%) |
| specificity | 59.0% (52.9% .. 65.0%) |
| PPV | 30.2% (23.2% .. 38.0%) |
| NPV | 97.0% (93.1% .. 99.0%) |

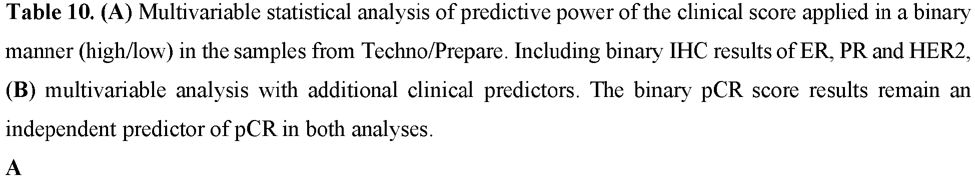

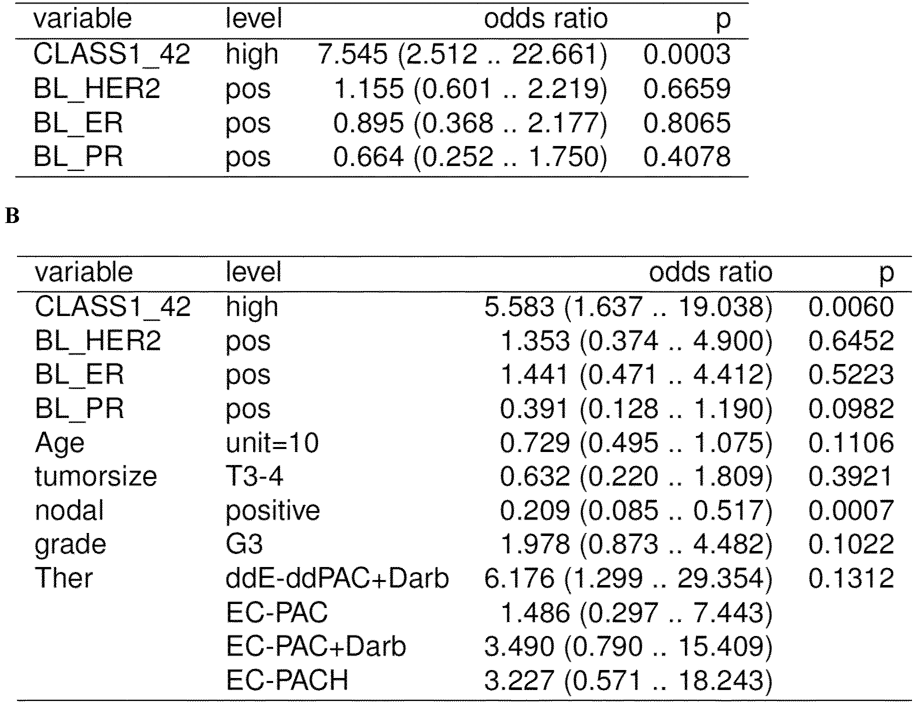

The clinical usefulness of the additional decision thresholds is illustrated by the significant separation of responders from non-responders as shown in **Table 11.**

**Table 11. Validation of additional thresholds as described above in the subset of cT1-T2 tumors. TP= true positive, FP= false positive, FN= false negative, TN= true negative, PPV= positive predictive value, NPV= negative predictive value. PPV corresponds to pCR rate in test positive group, 1-NPV corresponds to pCR rate in test negative group.**

| **T1-2** | **TP** | **FP** | **FN** | **TN** | **PPV** | **1-NPV** | **NPV** | **p Wald** | **odds ratio** | **comment** |
|---|---|---|---|---|---|---|---|---|---|---|
| **CLASS1_42** | 48 | 111 | 5 | 160 | 30.2% | 3.0% | 97.0% | <.0001 | 13.8 | primary objective |
| **CLASS1_69** | 28 | 41 | 25 | 230 | 40.6% | 9.8% | 90.2% | <.0001 | 6.3 | |
| **CLASS1_47** | 47 | 88 | 6 | 183 | 34.8% | 3.2% | 96.8% | <.0001 | 16.3 | |
| **CLASS1_50** | 42 | 84 | 11 | 187 | 33.3% | 5.6% | 94.4% | <.0001 | 8.5 | |
| **CLASS1_10P** | 47 | 91 | 6 | 180 | 34.1% | 3.2% | 96.8% | <.0001 | 15.5 | |
| **CLASS1_20P** | 34 | 51 | 19 | 220 | 40.0% | 7.9% | 92.1% | <.0001 | 7.7 | |
| **CLASS1_74** | 7 | 13 | 11 | 8 | 35.0% | 33.3% | 66.7% | 0.9234 | 1.1 | **HER2 pos (non luminal) only** |
| **CLASS1_43** | 11 | 26 | 2 | 36 | 29.7% | 4.8% | 94.7% | 0.0123 | 7.6 | **Luminal B-like (HER2-positive) only** |

The predictive power of the score together with the threshold 42 is especially high in the group of ESR1-or PGR-positive patients but also in the group of luminal B ERBB2/HER2-positive patients.

**Table 12. Validation of threshold 42 in different groups of samples. Subtypes defined according to MammaTyper^{®} (St Gallen 2013 guidelines).**

| **subset for CLASS_42** | **TP** | **FP** | **FN** | **TN** | **PPV** | **1-NPV** | **NPV** | **p Wald** | **odds ratio** |
|---|---|---|---|---|---|---|---|---|---|
| **all** | 56 | 167 | 6 | 189 | 25.1% | 3.1% | 96.9% | <.0001 | 10.6 |
| **T1-2, HER2 pos non luminal** | 11 | 21 | 0 | 0 | 34.4% | NA | NA | NA | NA |
| **T1-2, Luminal A-like** | 0 | 0 | 0 | 22 | NA | NA | 100% | NA | NA |
| **T1-2, Triple negative** | 20 | 35 | 0 | 0 | 36.4% | NA | NA | NA | NA |
| **T1-2, ESR1 and PGR negative** | 31 | 56 | 0 | 0 | 35.6% | NA | NA | NA | NA |
| **T1-2, Luminal B-like (HER2-negative)** | 3 | 24 | 4 | 104 | 11.1% | 3.7% | 96.3% | 0.139 | 3.3 |
| **T1-2, Luminal B-like (HER2-positive)** | 12 | 28 | 1 | 34 | 30.0% | 2.9% | 97.1% | 0.0124 | 14.6 |
| **T1-2, ESR1 or PGR positve** | 17 | 55 | 5 | 160 | 23.6% | 3.0% | 97.0% | <.0001 | 9.9 |

The score also carries prognostic information for non-responders which can be useful for the further management after neo-adjuvant chemotherapy is completed.

### Example 8: Estimation of suitable threshold ranges for score 1

In the original study, the main threshold 42 was validated for score 1. Other thresholds were systematically evaluated for their suitability for prediction of pCR. The following criteria define the clinical utility of a threshold, however as for all diagnostic tests, a tradeoff between the optimum of different criteria must be made to have a meaningful test:
- PPV (positive predictive value): Rate of true positive result among all positive results. Value should be high;
- NPV (negative predictive value): Rate of true negative results among negative results. Value should be high;
- Sensitivity: Rate of true positive results among all positive samples (pCR yes). Value should be high;
- Specificity: Rate of true negative results among all negative samples (pCR no). Value should be high;
- Youden index: Sensitivity + Specificity -100. Value should be high.

Based on the aforementioned criteria, a threshold for score 1 between 38 and 49 would lead to clinically meaningful results in which non-responders can be ruled out by the test while the responders are enriched in the group with high scores. The same range can be specifically applied for luminal ERBB2/HER2-positive tumors.

**Table 13. Estimation of suitable threshold ranges for score 1 (predicting probability of pCR).**

| **analysis configuration** | | | | **cross table (ypT0_ypN0)** | | | | | **basic predictive performance (ypT0_ypN0)** | | | | |
|---|---|---|---|---|---|---|---|---|---|---|---|---|---|
| **analysis set** | **subgroup** | **score** | **cutoff** | **n** | **TP** | **FP** | **FN** | **TN** | **PPV** | **NPV** | **sensiti vity** | **specifi city** | **Youden index (Sens + Spec -100)** |
| main | all | SCORE1 | 0 | 418 | 62 | 356 | 0 | 0 | 14.8 | n.a. | 100 | 0 | 0 |
| main | all | SCORE1 | 1 | 418 | 62 | 354 | 0 | 2 | 14.9 | 100 | 100 | 0.6 | 0.6 |
| main | all | SCORE1 | 2 | 418 | 62 | 354 | 0 | 2 | 14.9 | 100 | 100 | 0.6 | 0.6 |
| main | all | SCORE1 | 3 | 418 | 62 | 354 | 0 | 2 | 14.9 | 100 | 100 | 0.6 | 0.6 |
| main | all | SCORE1 | 4 | 418 | 62 | 354 | 0 | 2 | 14.9 | 100 | 100 | 0.6 | 0.6 |
| main | all | SCORE1 | 5 | 418 | 62 | 354 | 0 | 2 | 14.9 | 100 | 100 | 0.6 | 0.6 |
| main | all | SCORE1 | 6 | 418 | 62 | 354 | 0 | 2 | 14.9 | 100 | 100 | 0.6 | 0.6 |
| main | all | SCORE1 | 7 | 418 | 62 | 353 | 0 | 3 | 14.9 | 100 | 100 | 0.8 | 0.8 |
| main | all | SCORE1 | 8 | 418 | 62 | 353 | 0 | 3 | 14.9 | 100 | 100 | 0.8 | 0.8 |
| main | all | SCORE1 | 9 | 418 | 62 | 353 | 0 | 3 | 14.9 | 100 | 100 | 0.8 | 0.8 |
| main | all | SCORE1 | 10 | 418 | 62 | 352 | 0 | 4 | 15 | 100 | 100 | 1.1 | 1.1 |
| main | all | SCORE1 | 11 | 418 | 62 | 349 | 0 | 7 | 15.1 | 100 | 100 | 2 | 2 |
| main | all | SCORE1 | 12 | 418 | 62 | 345 | 0 | 11 | 15.2 | 100 | 100 | 3.1 | 3.1 |
| main | all | SCORE1 | 13 | 418 | 62 | 342 | 0 | 14 | 15.3 | 100 | 100 | 3.9 | 3.9 |
| main | all | SCORE1 | 14 | 418 | 62 | 342 | 0 | 14 | 15.3 | 100 | 100 | 3.9 | 3.9 |
| main | all | SCORE1 | 15 | 418 | 62 | 342 | 0 | 14 | 15.3 | 100 | 100 | 3.9 | 3.9 |
| main | all | SCORE1 | 16 | 418 | 62 | 339 | 0 | 17 | 15.5 | 100 | 100 | 4.8 | 4.8 |
| main | all | SCORE1 | 17 | 418 | 62 | 338 | 0 | 18 | 15.5 | 100 | 100 | 5.1 | 5.1 |
| main | all | SCORE1 | 18 | 418 | 62 | 336 | 0 | 20 | 15.6 | 100 | 100 | 5.6 | 5.6 |
| main | all | SCORE1 | 19 | 418 | 62 | 332 | 0 | 24 | 15.7 | 100 | 100 | 6.7 | 6.7 |
| main | all | SCORE1 | 20 | 418 | 61 | 322 | 1 | 34 | 15.9 | 97.1 | 98.4 | 9.6 | 8 |
| main | all | SCORE1 | 21 | 418 | 61 | 316 | 1 | 40 | 16.2 | 97.6 | 98.4 | 11.2 | 9.6 |
| main | all | SCORE1 | 22 | 418 | 61 | 313 | 1 | 43 | 16.3 | 97.7 | 98.4 | 12.1 | 10.5 |
| main | all | SCORE1 | 23 | 418 | 61 | 305 | 1 | 51 | 16.7 | 98.1 | 98.4 | 14.3 | 12.7 |
| main | all | SCORE1 | 24 | 418 | 61 | 301 | 1 | 55 | 16.9 | 98.2 | 98.4 | 15.4 | 13.8 |
| main | all | SCORE1 | 25 | 418 | 61 | 296 | 1 | 60 | 17.1 | 98.4 | 98.4 | 16.9 | 15.3 |
| main | all | SCORE1 | 26 | 418 | 61 | 291 | 1 | 65 | 17.3 | 98.5 | 98.4 | 18.3 | 16.7 |
| main | all | SCORE1 | 27 | 418 | 61 | 281 | 1 | 75 | 17.8 | 98.7 | 98.4 | 21.1 | 19.5 |
| main | all | SCORE1 | 28 | 418 | 60 | 274 | 2 | 82 | 18 | 97.6 | 96.8 | 23 | 19.8 |
| main | all | SCORE1 | 29 | 418 | 60 | 270 | 2 | 86 | 18.2 | 97.7 | 96.8 | 24.2 | 21 |
| main | all | SCORE1 | 30 | 418 | 60 | 264 | 2 | 92 | 18.5 | 97.9 | 96.8 | 25.8 | 22.6 |
| main | all | SCORE1 | 31 | 418 | 60 | 258 | 2 | 98 | 18.9 | 98 | 96.8 | 27.5 | 24.3 |
| main | all | SCORE1 | 32 | 418 | 59 | 249 | 3 | 107 | 19.2 | 97.3 | 95.2 | 30.1 | 25.3 |
| main | all | SCORE1 | 33 | 418 | 59 | 236 | 3 | 120 | 20 | 97.6 | 95.2 | 33.7 | 28.9 |
| main | all | SCORE1 | 34 | 418 | 59 | 226 | 3 | 130 | 20.7 | 97.7 | 95.2 | 36.5 | 31.7 |
| main | all | SCORE1 | 35 | 418 | 59 | 222 | 3 | 134 | 21 | 97.8 | 95.2 | 37.6 | 32.8 |
| main | all | SCORE1 | 36 | 418 | 59 | 214 | 3 | 142 | 21.6 | 97.9 | 95.2 | 39.9 | 35.1 |
| main | all | SCORE1 | 37 | 418 | 59 | 209 | 3 | 147 | 22 | 98 | 95.2 | 41.3 | 36.5 |
| main | all | SCORE1 | 38 | 418 | 59 | 199 | 3 | 157 | 22.9 | 98.1 | 95.2 | 44.1 | 39.3 |
| main | all | SCORE1 | 39 | 418 | 58 | 191 | 4 | 165 | 23.3 | 97.6 | 93.5 | 46.3 | 39.8 |
| main | all | SCORE1 | 40 | 418 | 57 | 183 | 5 | 173 | 23.8 | 97.2 | 91.9 | 48.6 | 40.5 |
| main | all | SCORE1 | 41 | 418 | 57 | 173 | 5 | 183 | 24.8 | 97.3 | 91.9 | 51.4 | 43.3 |
| main | all | SCORE1 | 42 | 418 | 56 | 167 | 6 | 189 | 25.1 | 96.9 | 90.3 | 53.1 | 43.4 |
| main | all | SCORE1 | 43 | 418 | 54 | 160 | 8 | 196 | 25.2 | 96.1 | 87.1 | 55.1 | 42.2 |
| main | all | SCORE1 | 44 | 418 | 54 | 153 | 8 | 203 | 26.1 | 96.2 | 87.1 | 57 | 44.1 |
| main | all | SCORE1 | 45 | 418 | 53 | 146 | 9 | 210 | 26.6 | 95.9 | 85.5 | 59 | 44.5 |
| main | all | SCORE1 | 46 | 418 | 53 | 141 | 9 | 215 | 27.3 | 96 | 85.5 | 60.4 | 45.9 |
| main | all | SCORE1 | 47 | 418 | 53 | 138 | 9 | 218 | 27.7 | 96 | 85.5 | 61.2 | 46.7 |
| main | all | SCORE1 | 48 | 418 | 53 | 137 | 9 | 219 | 27.9 | 96.1 | 85.5 | 61.5 | 47 |
| main | all | SCORE1 | 49 | 418 | 53 | 133 | 9 | 223 | 28.5 | 96.1 | 85.5 | 62.6 | 48.1 |
| main | all | SCORE1 | 50 | 418 | 47 | 131 | 15 | 225 | 26.4 | 93.8 | 75.8 | 63.2 | 39 |
| main | all | SCORE1 | 51 | 418 | 46 | 128 | 16 | 228 | 26.4 | 93.4 | 74.2 | 64 | 38.2 |
| main | all | SCORE1 | 52 | 418 | 45 | 121 | 17 | 235 | 27.1 | 93.3 | 72.6 | 66 | 38.6 |
| main | all | SCORE1 | 53 | 418 | 44 | 119 | 18 | 237 | 27 | 92.9 | 71 | 66.6 | 37.6 |
| main | all | SCORE1 | 54 | 418 | 44 | 115 | 18 | 241 | 27.7 | 93.1 | 71 | 67.7 | 38.7 |
| main | all | SCORE1 | 55 | 418 | 43 | 109 | 19 | 247 | 28.3 | 92.9 | 69.4 | 69.4 | 38.8 |
| main | all | SCORE1 | 56 | 418 | 42 | 107 | 20 | 249 | 28.2 | 92.6 | 67.7 | 69.9 | 37.6 |
| main | all | SCORE1 | 57 | 418 | 42 | 104 | 20 | 252 | 28.8 | 92.6 | 67.7 | 70.8 | 38.5 |
| main | all | SCORE1 | 58 | 418 | 42 | 101 | 20 | 255 | 29.4 | 92.7 | 67.7 | 71.6 | 39.3 |
| main | all | SCORE1 | 59 | 418 | 39 | 98 | 23 | 258 | 28.5 | 91.8 | 62.9 | 72.5 | 35.4 |
| main | all | SCORE1 | 60 | 418 | 38 | 92 | 24 | 264 | 29.2 | 91.7 | 61.3 | 74.2 | 35.5 |
| main | all | SCORE1 | 61 | 418 | 37 | 90 | 25 | 266 | 29.1 | 91.4 | 59.7 | 74.7 | 34.4 |
| main | all | SCORE1 | 62 | 418 | 37 | 88 | 25 | 268 | 29.6 | 91.5 | 59.7 | 75.3 | 35 |
| main | all | SCORE1 | 63 | 418 | 37 | 83 | 25 | 273 | 30.8 | 91.6 | 59.7 | 76.7 | 36.4 |
| main | all | SCORE1 | 64 | 418 | 37 | 81 | 25 | 275 | 31.4 | 91.7 | 59.7 | 77.2 | 36.9 |
| main | all | SCORE1 | 65 | 418 | 36 | 79 | 26 | 277 | 31.3 | 91.4 | 58.1 | 77.8 | 35.9 |
| main | all | SCORE1 | 66 | 418 | 36 | 74 | 26 | 282 | 32.7 | 91.6 | 58.1 | 79.2 | 37.3 |
| main | all | SCORE1 | 67 | 418 | 33 | 71 | 29 | 285 | 31.7 | 90.8 | 53.2 | 80.1 | 33.3 |
| main | all | SCORE1 | 68 | 418 | 31 | 67 | 31 | 289 | 31.6 | 90.3 | 50 | 81.2 | 31.2 |
| main | all | SCORE1 | 69 | 418 | 31 | 63 | 31 | 293 | 33 | 90.4 | 50 | 82.3 | 32.3 |
| main | all | SCORE1 | 70 | 418 | 30 | 62 | 32 | 294 | 32.6 | 90.2 | 48.4 | 82.6 | 31 |
| main | all | SCORE1 | 71 | 418 | 30 | 58 | 32 | 298 | 34.1 | 90.3 | 48.4 | 83.7 | 32.1 |
| main | all | SCORE1 | 72 | 418 | 29 | 56 | 33 | 300 | 34.1 | 90.1 | 46.8 | 84.3 | 31.1 |
| main | all | SCORE1 | 73 | 418 | 26 | 48 | 36 | 308 | 35.1 | 89.5 | 41.9 | 86.5 | 28.4 |
| main | all | SCORE1 | 74 | 418 | 24 | 43 | 38 | 313 | 35.8 | 89.2 | 38.7 | 87.9 | 26.6 |
| main | all | SCORE1 | 75 | 418 | 21 | 41 | 41 | 315 | 33.9 | 88.5 | 33.9 | 88.5 | 22.4 |
| main | all | SCORE1 | 76 | 418 | 17 | 36 | 45 | 320 | 32.1 | 87.7 | 27.4 | 89.9 | 17.3 |
| main | all | SCORE1 | 77 | 418 | 17 | 34 | 45 | 322 | 33.3 | 87.7 | 27.4 | 90.4 | 17.8 |
| main | all | SCORE1 | 78 | 418 | 16 | 28 | 46 | 328 | 36.4 | 87.7 | 25.8 | 92.1 | 17.9 |
| main | all | SCORE1 | 79 | 418 | 15 | 24 | 47 | 332 | 38.5 | 87.6 | 24.2 | 93.3 | 17.5 |
| main | all | SCORE1 | 80 | 418 | 14 | 23 | 48 | 333 | 37.8 | 87.4 | 22.6 | 93.5 | 16.1 |
| main | all | SCORE1 | 81 | 418 | 13 | 19 | 49 | 337 | 40.6 | 87.3 | 21 | 94.7 | 15.7 |
| main | all | SCORE1 | 82 | 418 | 11 | 19 | 51 | 337 | 36.7 | 86.9 | 17.7 | 94.7 | 12.4 |
| main | all | SCORE1 | 83 | 418 | 11 | 16 | 51 | 340 | 40.7 | 87 | 17.7 | 95.5 | 13.2 |
| main | all | SCORE1 | 84 | 418 | 11 | 14 | 51 | 342 | 44 | 87 | 17.7 | 96.1 | 13.8 |
| main | all | SCORE1 | 85 | 418 | 11 | 12 | 51 | 344 | 47.8 | 87.1 | 17.7 | 96.6 | 14.3 |
| main | all | SCORE1 | 86 | 418 | 10 | 10 | 52 | 346 | 50 | 86.9 | 16.1 | 97.2 | 13.3 |
| main | all | SCORE1 | 87 | 418 | 10 | 9 | 52 | 347 | 52.6 | 87 | 16.1 | 97.5 | 13.6 |
| main | all | SCORE1 | 88 | 418 | 9 | 8 | 53 | 348 | 52.9 | 86.8 | 14.5 | 97.8 | 12.3 |
| main | all | SCORE1 | 89 | 418 | 8 | 8 | 54 | 348 | 50 | 86.6 | 12.9 | 97.8 | 10.7 |
| main | all | SCORE1 | 90 | 418 | 6 | 7 | 56 | 349 | 46.2 | 86.2 | 9.7 | 98 | 7.7 |
| main | all | SCORE1 | 91 | 418 | 5 | 7 | 57 | 349 | 41.7 | 86 | 8.1 | 98 | 6.1 |
| main | all | SCORE1 | 92 | 418 | 3 | 7 | 59 | 349 | 30 | 85.5 | 4.8 | 98 | 2.8 |
| main | all | SCORE1 | 93 | 418 | 3 | 6 | 59 | 350 | 33.3 | 85.6 | 4.8 | 98.3 | 3.1 |
| main | all | SCORE1 | 94 | 418 | 3 | 6 | 59 | 350 | 33.3 | 85.6 | 4.8 | 98.3 | 3.1 |
| main | all | SCORE1 | 95 | 418 | 3 | 5 | 59 | 351 | 37.5 | 85.6 | 4.8 | 98.6 | 3.4 |
| main | all | SCORE1 | 96 | 418 | 2 | 4 | 60 | 352 | 33.3 | 85.4 | 3.2 | 98.9 | 2.1 |
| main | all | SCORE1 | 97 | 418 | 1 | 4 | 61 | 352 | 20 | 85.2 | 1.6 | 98.9 | 0.5 |
| main | all | SCORE1 | 98 | 418 | 1 | 3 | 61 | 353 | 25 | 85.3 | 1.6 | 99.2 | 0.8 |
| main | all | SCORE1 | 99 | 418 | 1 | 2 | 61 | 354 | 33.3 | 85.3 | 1.6 | 99.4 | 1 |
| main | Luminal-B-like (HER2 positive) | SCORE1 | 0 | 101 | 17 | 84 | 0 | 0 | 16.8 | n.a. | 100 | 0 | 0 |
| main | Luminal-B-like (HER2 positive) | SCORE1 | 1 | 101 | 17 | 84 | 0 | 0 | 16.8 | n.a. | 100 | 0 | 0 |
| main | Luminal-B-like (HER2 positive) | SCORE1 | 2 | 101 | 17 | 84 | 0 | 0 | 16.8 | n.a. | 100 | 0 | 0 |
| main | Luminal-B-like (HER2 positive) | SCORE1 | 3 | 101 | 17 | 84 | 0 | 0 | 16.8 | n.a. | 100 | 0 | 0 |
| main | Luminal-B-like (HER2 positive) | SCORE1 | 4 | 101 | 17 | 84 | 0 | 0 | 16.8 | n.a. | 100 | 0 | 0 |
| main | Luminal-B-like (HER2 positive) | SCORE1 | 5 | 101 | 17 | 84 | 0 | 0 | 16.8 | n.a. | 100 | 0 | 0 |
| main | Luminal-B-like (HER2 positive) | SCORE1 | 6 | 101 | 17 | 84 | 0 | 0 | 16.8 | n.a. | 100 | 0 | 0 |
| main | Luminal-B-like (HER2 positive) | SCORE1 | 7 | 101 | 17 | 84 | 0 | 0 | 16.8 | n.a. | 100 | 0 | 0 |
| main | Luminal-B-like (HER2 positive) | SCORE1 | 8 | 101 | 17 | 84 | 0 | 0 | 16.8 | n.a. | 100 | 0 | 0 |
| main | Luminal-B-like (HER2 positive) | SCORE1 | 9 | 101 | 17 | 84 | 0 | 0 | 16.8 | n.a. | 100 | 0 | 0 |
| main | Luminal-B-like (HER2 positive) | SCORE1 | 10 | 101 | 17 | 83 | 0 | 1 | 17 | 100 | 100 | 1.2 | 1.2 |
| main | Luminal-B-like (HER2 positive) | SCORE1 | 11 | 101 | 17 | 83 | 0 | 1 | 17 | 100 | 100 | 1.2 | 1.2 |
| main | Luminal-B-like (HER2 positive) | SCORE1 | 12 | 101 | 17 | 83 | 0 | 1 | 17 | 100 | 100 | 1.2 | 1.2 |
| main | Luminal-B-like (HER2 positive) | SCORE1 | 13 | 101 | 17 | 83 | 0 | 1 | 17 | 100 | 100 | 1.2 | 1.2 |
| main | Luminal-B-like (HER2 positive) | SCORE1 | 14 | 101 | 17 | 83 | 0 | 1 | 17 | 100 | 100 | 1.2 | 1.2 |
| main | Luminal-B-like (HER2 positive) | SCORE1 | 15 | 101 | 17 | 83 | 0 | 1 | 17 | 100 | 100 | 1.2 | 1.2 |
| main | Luminal-B-like (HER2 positive) | SCORE1 | 16 | 101 | 17 | 82 | 0 | 2 | 17.2 | 100 | 100 | 2.4 | 2.4 |
| main | Luminal-B-like (HER2 positive) | SCORE1 | 17 | 101 | 17 | 82 | 0 | 2 | 17.2 | 100 | 100 | 2.4 | 2.4 |
| main | Luminal-B-like (HER2 positive) | SCORE1 | 18 | 101 | 17 | 82 | 0 | 2 | 17.2 | 100 | 100 | 2.4 | 2.4 |
| main | Luminal-B-like (HER2 positive) | SCORE1 | 19 | 101 | 17 | 80 | 0 | 4 | 17.5 | 100 | 100 | 4.8 | 4.8 |
| main | Luminal-B-like (HER2 positive) | SCORE1 | 20 | 101 | 16 | 80 | 1 | 4 | 16.7 | 80 | 94.1 | 4.8 | -1.1 |
| main | Luminal-B-like (HER2 positive) | SCORE1 | 21 | 101 | 16 | 80 | 1 | 4 | 16.7 | 80 | 94.1 | 4.8 | -1.1 |
| main | Luminal-B-like (HER2 positive) | SCORE1 | 22 | 101 | 16 | 79 | 1 | 5 | 16.8 | 83.3 | 94.1 | 6 | 0.1 |
| main | Luminal-B-like (HER2 positive) | SCORE1 | 23 | 101 | 16 | 78 | 1 | 6 | 17 | 85.7 | 94.1 | 7.1 | 1.2 |
| main | Luminal-B-like (HER2 positive) | SCORE1 | 24 | 101 | 16 | 77 | 1 | 7 | 17.2 | 87.5 | 94.1 | 8.3 | 2.4 |
| main | Luminal-B-like (HER2 positive) | SCORE1 | 25 | 101 | 16 | 77 | 1 | 7 | 17.2 | 87.5 | 94.1 | 8.3 | 2.4 |
| main | Luminal-B-like (HER2 positive) | SCORE1 | 26 | 101 | 16 | 76 | 1 | 8 | 17.4 | 88.9 | 94.1 | 9.5 | 3.6 |
| main | Luminal-B-like (HER2 positive) | SCORE1 | 27 | 101 | 16 | 74 | 1 | 10 | 17.8 | 909 | 94.1 | 11.9 | 6 |
| main | Luminal-B-like (HER2 positive) | SCORE1 | 28 | 101 | 16 | 73 | 1 | 11 | 18 | 91.7 | 94.1 | 13.1 | 7.2 |
| main | Luminal-B-like (HER2 positive) | SCORE1 | 29 | 101 | 16 | 71 | 1 | 13 | 18.4 | 92.9 | 94.1 | 15.5 | 9.6 |
| main | Luminal-B-like (HER2 positive) | SCORE1 | 30 | 101 | 16 | 71 | 1 | 13 | 18.4 | 92.9 | 94.1 | 15.5 | 9.6 |
| main | Luminal-B-like (HER2 positive) | SCORE1 | 31 | 101 | 16 | 68 | 1 | 16 | 19 | 94.1 | 94.1 | 19 | 13.1 |
| main | Luminal-B-like (HER2 positive) | SCORE1 | 32 | 101 | 16 | 66 | 1 | 18 | 19.5 | 94.7 | 94.1 | 21.4 | 15.5 |
| main | Luminal-B-like (HER2 positive) | SCORE1 | 33 | 101 | 16 | 63 | 1 | 21 | 20.3 | 95.5 | 94.1 | 25 | 19.1 |
| main | Luminal-B-like (HER2 positive) | SCORE1 | 34 | 101 | 16 | 60 | 1 | 24 | 21.1 | 96 | 94.1 | 28.6 | 22.7 |
| main | Luminal-B-like (HER2 positive) | SCORE1 | 35 | 101 | 16 | 59 | 1 | 25 | 21.3 | 96.2 | 94.1 | 29.8 | 23.9 |
| main | Luminal-B-like (HER2 positive) | SCORE1 | 36 | 101 | 16 | 56 | 1 | 28 | 22.2 | 96.6 | 94.1 | 33.3 | 27.4 |
| main | Luminal-B-like (HER2 positive) | SCORE1 | 37 | 101 | 16 | 53 | 1 | 31 | 23.2 | 96.9 | 94.1 | 36.9 | 31 |
| main | Luminal-B-like (HER2 positive) | SCORE1 | 38 | 101 | 16 | 51 | 1 | 33 | 23.9 | 97.1 | 94.1 | 39.3 | 33.4 |
| main | Luminal-B-like (HER2 positive) | SCORE1 | 39 | 101 | 16 | 49 | 1 | 35 | 24.6 | 97.2 | 94.1 | 41.7 | 35.8 |
| main | Luminal-B-like (HER2 positive) | SCORE1 | 40 | 101 | 16 | 47 | 1 | 37 | 25.4 | 97.4 | 94.1 | 44 | 38.1 |
| main | Luminal-B-like (HER2 positive) | SCORE1 | 41 | 101 | 16 | 44 | 1 | 40 | 26.7 | 97.6 | 94.1 | 47.6 | 41.7 |
| main | Luminal-B-like (HER2 positive) | SCORE1 | 42 | 101 | 15 | 40 | 2 | 44 | 27.3 | 95.7 | 88.2 | 52.4 | 40.6 |
| main | Luminal-B-like (HER2 positive) | SCORE1 | 43 | 101 | 14 | 38 | 3 | 46 | 26.9 | 93.9 | 82.4 | 54.8 | 37.2 |
| main | Luminal-B-like (HER2 positive) | SCORE1 | 44 | 101 | 14 | 36 | 3 | 48 | 28 | 94.1 | 82.4 | 57.1 | 39.5 |
| main | Luminal-B-like (HER2 positive) | SCORE1 | 45 | 101 | 14 | 35 | 3 | 49 | 28.6 | 94.2 | 82.4 | 58.3 | 40.7 |
| main | Luminal-B-like (HER2 positive) | SCORE1 | 46 | 101 | 14 | 32 | 3 | 52 | 30.4 | 94.5 | 82.4 | 61.9 | 44.3 |
| main | Luminal-B-like (HER2 positive) | SCORE1 | 47 | 101 | 14 | 31 | 3 | 53 | 31.1 | 94.6 | 82.4 | 63.1 | 45.5 |
| main | Luminal-B-like (HER2 positive) | SCORE1 | 48 | 101 | 14 | 30 | 3 | 54 | 31.8 | 94.7 | 82.4 | 64.3 | 46.7 |
| main | Luminal-B-like (HER2 positive) | SCORE1 | 49 | 101 | 14 | 28 | 3 | 56 | 33.3 | 94.9 | 82.4 | 66.7 | 49.1 |
| main | Luminal-B-like (HER2 positive) | SCORE1 | 50 | 101 | 10 | 27 | 7 | 57 | 27 | 89.1 | 58.8 | 67.9 | 26.7 |
| main | Luminal-B-like (HER2 positive) | SCORE1 | 51 | 101 | 9 | 25 | 8 | 59 | 26.5 | 88.1 | 52.9 | 70.2 | 23.1 |
| main | Luminal-B-like (HER2 positive) | SCORE1 | 52 | 101 | 9 | 20 | 8 | 64 | 31 | 88.9 | 52.9 | 76.2 | 29.1 |
| main | Luminal-B-like (HER2 positive) | SCORE1 | 53 | 101 | 8 | 19 | 9 | 65 | 29.6 | 87.8 | 47.1 | 77.4 | 24.5 |
| main | Luminal-B-like (HER2 positive) | SCORE1 | 54 | 101 | 8 | 17 | 9 | 67 | 32 | 88.2 | 47.1 | 79.8 | 26.9 |
| main | Luminal-B-like (HER2 positive) | SCORE1 | 55 | 101 | 7 | 14 | 10 | 70 | 33.3 | 87.5 | 41.2 | 83.3 | 24.5 |
| main | Luminal-B-like (HER2 positive) | SCORE1 | 56 | 101 | 7 | 12 | 10 | 72 | 36.8 | 87.8 | 41.2 | 85.7 | 26.9 |
| main | Luminal-B-like (HER2 positive) | SCORE1 | 57 | 101 | 7 | 11 | 10 | 73 | 38.9 | 88 | 41.2 | 86.9 | 28.1 |
| main | Luminal-B-like (HER2 positive) | SCORE1 | 58 | 101 | 7 | 9 | 10 | 75 | 43.8 | 88.2 | 41.2 | 89.3 | 30.5 |
| main | Luminal-B-like (HER2 positive) | SCORE1 | 59 | 101 | 5 | 7 | 12 | 77 | 41.7 | 86.5 | 29.4 | 91.7 | 21.1 |
| main | Luminal-B-like (HER2 positive) | SCORE1 | 60 | 101 | 4 | 5 | 13 | 79 | 44.4 | 85.9 | 23.5 | 94 | 17.5 |
| main | Luminal-B-like (HER2 positive) | SCORE1 | 61 | 101 | 3 | 5 | 14 | 79 | 37.5 | 84.9 | 17.6 | 94 | 11.6 |
| main | Luminal-B-like (HER2 positive) | SCORE1 | 62 | 101 | 3 | 5 | 14 | 79 | 37.5 | 84.9 | 17.6 | 94 | 11.6 |
| main | Luminal-B-like (HER2 positive) | SCORE1 | 63 | 101 | 3 | 5 | 14 | 79 | 37.5 | 84.9 | 17.6 | 94 | 11.6 |
| main | Luminal-B-like (HER2 positive) | SCORE1 | 64 | 101 | 3 | 4 | 14 | 80 | 42.9 | 85.1 | 17.6 | 95.2 | 12.8 |
| main | Luminal-B-like (HER2 positive) | SCORE1 | 65 | 101 | 3 | 4 | 14 | 80 | 42.9 | 85.1 | 17.6 | 95.2 | 12.8 |
| main | Luminal-B-like (HER2 positive) | SCORE1 | 66 | 101 | 3 | 4 | 14 | 80 | 42.9 | 85.1 | 17.6 | 95.2 | 12.8 |
| main | Luminal-B-like (HER2 positive) | SCORE1 | 67 | 101 | 2 | 4 | 15 | 80 | 33.3 | 84.2 | 11.8 | 95.2 | 7 |
| main | Luminal-B-like (HER2 positive) | SCORE1 | 68 | 101 | 2 | 3 | 15 | 81 | 40 | 84.4 | 11.8 | 96.4 | 8.2 |
| main | Luminal-B-like (HER2 positive) | SCORE1 | 69 | 101 | 2 | 3 | 15 | 81 | 40 | 84.4 | 11.8 | 96.4 | 8.2 |
| main | Luminal-B-like (HER2 positive) | SCORE1 | 70 | 101 | 2 | 3 | 15 | 81 | 40 | 84.4 | 11.8 | 96.4 | 8.2 |
| main | Luminal-B-like (HER2 positive) | SCORE1 | 71 | 101 | 2 | 2 | 15 | 82 | 50 | 84.5 | 11.8 | 97.6 | 9.4 |
| main | Luminal-B-like (HER2 positive) | SCORE1 | 72 | 101 | 2 | 2 | 15 | 82 | 50 | 84.5 | 11.8 | 97.6 | 9.4 |
| main | Luminal-B-like (HER2 positive) | SCORE1 | 73 | 101 | 2 | 1 | 15 | 83 | 66.7 | 84.7 | 11.8 | 98.8 | 10.6 |
| main | Luminal-B-like (HER2 positive) | SCORE1 | 74 | 101 | 2 | 1 | 15 | 83 | 66.7 | 84.7 | 11.8 | 98.8 | 10.6 |
| main | Luminal-B-like (HER2 positive) | SCORE1 | 75 | 101 | 2 | 1 | 15 | 83 | 66.7 | 84.7 | 11.8 | 98.8 | 10.6 |
| main | Luminal-B-like (HER2 positive) | SCORE1 | 76 | 101 | 1 | 1 | 16 | 83 | 50 | 83.8 | 5.9 | 98.8 | 4.7 |
| main | Luminal-B-like (HER2 positive) | SCORE1 | 77 | 101 | 1 | 1 | 16 | 83 | 50 | 83.8 | 5.9 | 98.8 | 4.7 |
| main | Luminal-B-like (HER2 positive) | SCORE1 | 78 | 101 | 1 | 1 | 16 | 83 | 50 | 83.8 | 5.9 | 98.8 | 4.7 |
| main | Luminal-B-like (HER2 positive) | SCORE1 | 79 | 101 | 1 | 1 | 16 | 83 | 50 | 83.8 | 5.9 | 98.8 | 4.7 |
| main | Luminal-B-like (HER2 positive) | SCORE1 | 80 | 101 | 1 | 1 | 16 | 83 | 50 | 83.8 | 5.9 | 98.8 | 4.7 |
| main | Luminal-B-like (HER2 positive) | SCORE1 | 81 | 101 | 1 | 1 | 16 | 83 | 50 | 83.8 | 5.9 | 98.8 | 4.7 |
| main | Luminal-B-like (HER2 positive) | SCORE1 | 82 | 101 | 1 | 1 | 16 | 83 | 50 | 83.8 | 5.9 | 98.8 | 4.7 |
| main | Luminal-B-like (HER2 positive) | SCORE1 | 83 | 101 | 1 | 0 | 16 | 84 | 100 | 84 | 5.9 | 100 | 5.9 |
| main | Luminal-B-like (HER2 positive) | SCORE1 | 84 | 101 | 1 | 0 | 16 | 84 | 100 | 84 | 5.9 | 100 | 5.9 |
| main | Luminal-B-like (HER2 positive) | SCORE1 | 85 | 101 | 1 | 0 | 16 | 84 | 100 | 84 | 5.9 | 100 | 5.9 |
| main | Luminal-B-like (HER2 positive) | SCORE1 | 86 | 101 | 0 | 0 | 17 | 84 | n.a. | 83.2 | 0 | 100 | 0 |
| main | Luminal-B-like (HER2 positive) | SCORE1 | 87 | 101 | 0 | 0 | 17 | 84 | n.a. | 83.2 | 0 | 100 | 0 |
| main | Luminal-B-like (HER2 positive) | SCORE1 | 88 | 101 | 0 | 0 | 17 | 84 | n.a. | 83.2 | 0 | 100 | 0 |
| main | Luminal-B-like (HER2 positive) | SCORE1 | 89 | 101 | 0 | 0 | 17 | 84 | n.a. | 83.2 | 0 | 100 | 0 |
| main | Luminal-B-like (HER2 positive) | SCORE1 | 90 | 101 | 0 | 0 | 17 | 84 | n.a. | 83.2 | 0 | 100 | 0 |
| main | Luminal-B-like (HER2 positive) | SCORE1 | 91 | 101 | 0 | 0 | 17 | 84 | n.a. | 83.2 | 0 | 100 | 0 |
| main | Luminal-B-like (HER2 positive) | SCORE1 | 92 | 101 | 0 | 0 | 17 | 84 | n.a. | 83.2 | 0 | 100 | 0 |
| main | Luminal-B-like (HER2 positive) | SCORE1 | 93 | 101 | 0 | 0 | 17 | 84 | n.a. | 83.2 | 0 | 100 | 0 |
| main | Luminal-B-like (HER2 positive) | SCORE1 | 94 | 101 | 0 | 0 | 17 | 84 | n.a. | 83.2 | 0 | 100 | 0 |
| main | Luminal-B-like (HER2 positive) | SCORE1 | 95 | 101 | 0 | 0 | 17 | 84 | n.a. | 83.2 | 0 | 100 | 0 |
| main | Luminal-B-like (HER2 positive) | SCORE1 | 96 | 101 | 0 | 0 | 17 | 84 | n.a. | 83.2 | 0 | 100 | 0 |
| main | Luminal-B-like (HER2 positive) | SCORE1 | 97 | 101 | 0 | 0 | 17 | 84 | n.a. | 83.2 | 0 | 100 | 0 |
| main | Luminal-B-like (HER2 positive) | SCORE1 | 98 | 101 | 0 | 0 | 17 | 84 | n.a. | 83.2 | 0 | 100 | 0 |
| main | Luminal-B-like (HER2 positive) | SCORE1 | 99 | 101 | 0 | 0 | 17 | 84 | n.a. | 83.2 | 0 | 100 | 0 |

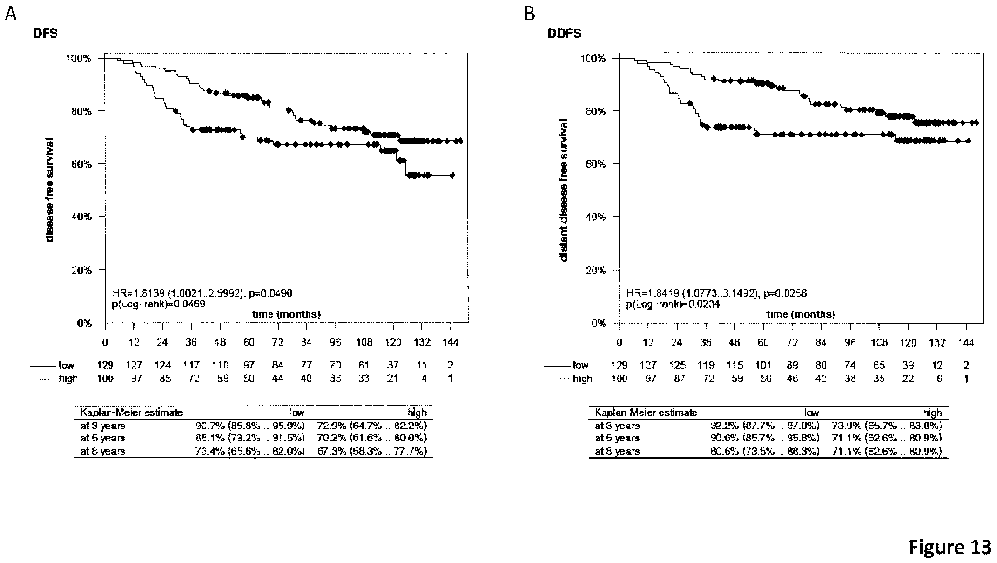

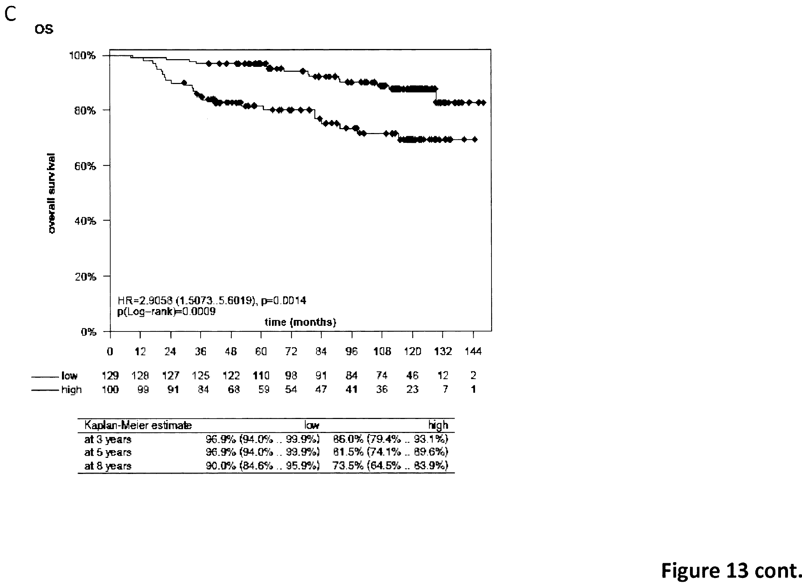

With regard to establishment of prognostic thresholds (long term outcome) other than the one validated in the Techno/Prepare cohorts (Figure 13) the following criteria may be applied:
- HR (Hazard ratio): Ratio of the hazard rates corresponding to the conditions (e.g. test results low/high). HR can be calculated for disease-free survival (DFS), distant disease-free survival (DDFS) (also referred to as distant recurrence-free survival, DRFS, herein) and overall survival (OS), wherein for primary breast cancer DDFS and OS are the most relevant clinical parameters. The values should be high or low dependent on the comparator group. In the current analysis a high HR should be achieved.
- Kaplan Meier estimates (percentage of patients with no event (DFS, DDFS or OS) at a given time point). There is no consensus about an acceptable rate of events, however a 5-10% rate of DDFS at 5 years is observed in current routine practice with stratification by IHC (Hennigs A. et. al., 2016, BMC Cancer 16(1):734).

Based on the aforementioned criteria, a threshold for score 1 between 25 and 29 would lead to clinically meaningful results in which the low group would have a significantly better survival than the high group. Even lower thresholds may also be applied and would lead to even lower recurrence risks. This cannot be seen in this sample set, as the sample size is limited.

**Table 14. Estimation of suitable threshold ranges for score 1 (prognosis).**

| | | | | **univariate Cox regression model (DFS)** | | **Kaplan-Meier estimates (DFS)** | | **univariate Cox regression model (DDFS)** | | **Kaplan-Meier estimates (DDFS)** | | **univariate Cox regression model (OS)** | | **Kaplan-Meier estimates (OS)** | |
|---|---|---|---|---|---|---|---|---|---|---|---|---|---|---|---|
| **analysis set** | **subgroup** | **score** | **cutoff** | **HR (DFS)** | **p (DFS)** | **KM (low score, DFS at 5 years)** | **KM (high score, DFS at 5 years)** | **HR (DDFS)** | **p (DDFS)** | **KM (low score, DDFS at 5 years)** | **KM (high score, DDFS at 5 years)** | **HR(OS)** | **p (OS)** | **KM (low score, OS at 5 years)** | **KM (high score, OS at 5 years)** |
| main | all | SCORE1 | 0 | n.a. | n.a. | n.a. | 0.71 | n.a. | n.a. | n.a. | 0.75 | n.a. | n.a. | n.a. | 0.84 |
| main | all | SCORE1 | 1 | 0.18 | 0.02 | n.a. | 0.72 | 0.16 | 0.01 | n.a. | 0.75 | 0.10 | 0.00 | n.a. | 0.85 |
| main | all | SCORE1 | 2 | 0.18 | 0.02 | n.a. | 0.72 | 0.16 | 0.01 | n.a. | 0.75 | 0.10 | 0.00 | n.a. | 0.85 |
| main | all | SCORE1 | 3 | 0.18 | 0.02 | n.a. | 0.72 | 0.16 | 0.01 | n.a. | 0.75 | 0.10 | 0.00 | n.a. | 0.85 |
| main | all | SCORE1 | 4 | 0.18 | 0.02 | n.a. | 0.72 | 0.16 | 0.01 | n.a. | 0.75 | 0.10 | 0.00 | n.a. | 0.85 |
| main | all | SCORE1 | 5 | 0.18 | 0.02 | n.a. | 0.72 | 0.16 | 0.01 | n.a. | 0.75 | 0.10 | 0.00 | n.a. | 0.85 |
| main | all | SCORE1 | 6 | 0.18 | 0.02 | n.a. | 0.72 | 0.16 | 0.01 | n.a. | 0.75 | 0.10 | 0.00 | n.a. | 0.85 |
| main | all | SCORE1 | 7 | 0.34 | 0.13 | n.a. | 0.72 | 0.29 | 0.09 | n.a. | 0.75 | 0.19 | 0.02 | n.a. | 0.85 |
| main | all | SCORE1 | 8 | 0.34 | 0.13 | n.a. | 0.72 | 0.29 | 0.09 | n.a. | 0.75 | 0.19 | 0.02 | n.a. | 0.85 |
| main | all | SCORE1 | 9 | 0.34 | 0.13 | n.a. | 0.72 | 0.29 | 0.09 | n.a. | 0.75 | 0.19 | 0.02 | n.a. | 0.85 |
| main | all | SCORE1 | 10 | 0.34 | 0.07 | 0.50 | 0.71 | 0.30 | 0.04 | 0.50 | 0.75 | 0.33 | 0.13 | 0.50 | 0.85 |
| main | all | SCORE1 | 11 | 0.74 | 0.60 | 0.71 | 0.71 | 0.63 | 0.43 | 0.71 | 0.75 | 0.67 | 0.58 | 0.71 | 0.84 |
| main | all | SCORE1 | 12 | 1.33 | 0.62 | 0.82 | 0.71 | 1.12 | 0.85 | 0.82 | 0.75 | 1.17 | 0.83 | 0.82 | 0.84 |
| main | all | SCORE1 | 13 | 1.37 | 0.54 | 0.86 | 0.71 | 1.14 | 0.80 | 0.86 | 0.75 | 1.62 | 0.50 | 0.86 | 0.84 |
| main | all | SCORE1 | 14 | 1.37 | 0.54 | 0.86 | 0.71 | 1.14 | 0.80 | 0.86 | 0.75 | 1.62 | 0.50 | 0.86 | 0.84 |
| main | all | SCORE1 | 15 | 1.37 | 0.54 | 0.86 | 0.71 | 1.14 | 0.80 | 0.86 | 0.75 | 1.62 | 0.50 | 0.86 | 0.84 |
| main | all | SCORE1 | 16 | 1.74 | 0.27 | 0.88 | 0.71 | 1.45 | 0.46 | 0.88 | 0.74 | 2.08 | 0.30 | 0.88 | 0.84 |
| main | all | SCORE1 | 17 | 1.87 | 0.22 | 0.89 | 0.70 | 1.56 | 0.38 | 0.89 | 0.74 | 2.24 | 0.26 | 0.89 | 0.84 |
| main | all | SCORE1 | 18 | 1.67 | 0.26 | 0.90 | 0.70 | 1.39 | 0.48 | 0.90 | 0.74 | 1.67 | 0.38 | 0.90 | 0.84 |
| main | all | SCORE1 | 19 | 1.73 | 0.19 | 0.92 | 0.70 | 1.43 | 0.39 | 0.92 | 0.74 | 2.10 | 0.21 | 0.92 | 0.84 |
| main | all | SCORE1 | 20 | 1.70 | 0.12 | 0.88 | 0.70 | 1.60 | 0.20 | 0.94 | 0.73 | 1.85 | 0.18 | 0.94 | 0.83 |
| main | all | SCORE1 | 21 | 1.30 | 0.36 | 0.83 | 0.70 | 1.48 | 0.23 | 0.93 | 0.73 | 1.86 | 0.14 | 0.95 | 0.83 |
| main | all | SCORE1 | 22 | 1.32 | 0.32 | 0.84 | 0.70 | 1.46 | 0.23 | 0.93 | 0.73 | 2.04 | 0.09 | 0.95 | 0.83 |
| main | all | SCORE1 | 23 | 1.35 | 0.26 | 0.83 | 0.70 | 1.57 | 0.14 | 0.92 | 0.72 | 2.03 | 0.07 | 0.94 | 0.83 |
| main | all | SCORE1 | 24 | 1.37 | 0.22 | 0.82 | 0.70 | 1.71 | 0.08 | 0.93 | 0.72 | 2.19 | 0.05 | 0.94 | 0.83 |
| main | all | SCORE1 | 25 | 1.44 | 0.15 | 0.82 | 0.69 | 1.74 | 0.06 | 0.92 | 0.72 | 2.45 | 0.02 | 0.95 | 0.82 |
| main | all | SCORE1 | 26 | 1.61 | 0.06 | 0.83 | 0.69 | 1.94 | 0.02 | 0.92 | 0.72 | 2.38 | 0.02 | 0.95 | 0.82 |
| main | all | SCORE1 | 27 | 1.63 | 0.04 | 0.84 | 0.68 | 1.84 | 0.02 | 0.92 | 0.71 | 2.49 | 0.01 | 0.95 | 0.82 |
| main | all | SCORE1 | 28 | 1.76 | 0.01 | 0.84 | 0.68 | 1.96 | 0.01 | 0.92 | 0.71 | 2.85 | 0.00 | 0.95 | 0.81 |
| main | all | SCORE1 | 29 | 1.56 | 0.04 | 0.82 | 0.68 | 1.67 | 0.04 | 0.89 | 0.71 | 2.69 | 0.00 | 0.94 | 0.82 |
| main | all | SCORE1 | 30 | 1.65 | 0.02 | 0.82 | 0.68 | 1.73 | 0.02 | 0.88 | 0.71 | 2.66 | 0.00 | 0.95 | 0.81 |
| main | all | SCORE1 | 31 | 1.54 | 0.04 | 0.80 | 0.68 | 1.66 | 0.03 | 0.87 | 0.71 | 2.35 | 0.00 | 0.94 | 0.81 |
| main | all | SCORE1 | 32 | 1.49 | 0.04 | 0.79 | 0.68 | 1.73 | 0.01 | 0.87 | 0.70 | 2.46 | 0.00 | 0.93 | 0.81 |
| main | all | SCORE1 | 33 | 1.33 | 0.12 | 0.78 | 0.68 | 1.45 | 0.07 | 0.85 | 0.70 | 2.31 | 0.00 | 0.93 | 0.80 |
| main | all | SCORE1 | 34 | 1.53 | 0.02 | 0.80 | 0.67 | 1.65 | 0.01 | 0.86 | 0.69 | 2.61 | 0.00 | 0.94 | 0.80 |
| main | all | SCORE1 | 35 | 1.52 | 0.02 | 0.79 | 0.67 | 1.63 | 0.02 | 0.86 | 0.69 | 2.49 | 0.00 | 0.93 | 0.80 |
| main | all | SCORE1 | 36 | 1.44 | 0.04 | 0.78 | 0.67 | 1.50 | 0.04 | 0.85 | 0.70 | 2.23 | 0.00 | 0.93 | 0.80 |
| main | all | SCORE1 | 37 | 1.49 | 0.03 | 0.79 | 0.67 | 1.60 | 0.02 | 0.85 | 0.69 | 2.37 | 0.00 | 0.93 | 0.79 |
| main | all | SCORE1 | 38 | 1.62 | 0.01 | 0.80 | 0.65 | 1.79 | 0.00 | 0.86 | 0.68 | 2.49 | 0.00 | 0.94 | 0.78 |
| main | all | SCORE1 | 39 | 1.57 | 0.01 | 0.80 | 0.65 | 1.76 | 0.00 | 0.86 | 0.67 | 2.44 | 0.00 | 0.93 | 0.78 |
| main | all | SCORE1 | 40 | 1.48 | 0.02 | 0.80 | 0.64 | 1.60 | 0.01 | 0.85 | 0.67 | 2.16 | 0.00 | 0.92 | 0.78 |
| main | all | SCORE1 | 41 | 1.49 | 0.02 | 0.80 | 0.64 | 1.65 | 0.01 | 0.85 | 0.66 | 2.14 | 0.00 | 0.92 | 0.77 |
| main | all | SCORE1 | 42 | 1.37 | 0.06 | 0.78 | 0.65 | 1.47 | 0.03 | 0.83 | 0.68 | 2.16 | 0.00 | 0.92 | 0.77 |
| main | all | SCORE1 | 43 | 1.46 | 0.02 | 0.78 | 0.64 | 1.55 | 0.02 | 0.83 | 0.67 | 2.31 | 0.00 | 0.92 | 0.77 |
| main | all | SCORE1 | 44 | 1.55 | 0.01 | 0.78 | 0.64 | 1.63 | 0.01 | 0.83 | 0.66 | 2.38 | 0.00 | 0.92 | 0.76 |
| main | all | SCORE1 | 45 | 1.49 | 0.02 | 0.78 | 0.64 | 1.53 | 0.02 | 0.82 | 0.66 | 2.04 | 0.00 | 0.91 | 0.77 |
| main | all | SCORE1 | 46 | 1.49 | 0.02 | 0.78 | 0.63 | 1.57 | 0.01 | 0.82 | 0.66 | 2.05 | 0.00 | 0.91 | 0.77 |
| main | all | SCORE1 | 47 | 1.54 | 0.01 | 0.78 | 0.63 | 1.62 | 0.01 | 0.83 | 0.65 | 2.04 | 0.00 | 0.91 | 0.76 |
| main | all | SCORE1 | 48 | 1.56 | 0.01 | 0.78 | 0.63 | 1.65 | 0.01 | 0.83 | 0.65 | 2.07 | 0.00 | 0.91 | 0.76 |
| main | all | SCORE1 | 49 | 1.64 | 0.00 | 0.79 | 0.62 | 1.72 | 0.00 | 0.83 | 0.64 | 2.16 | 0.00 | 0.91 | 0.75 |
| main | all | SCORE1 | 50 | 1.58 | 0.01 | 0.78 | 0.62 | 1.75 | 0.00 | 0.83 | 0.64 | 2.26 | 0.00 | 0.91 | 0.75 |
| main | all | SCORE1 | 51 | 1.53 | 0.01 | 0.78 | 0.62 | 1.69 | 0.00 | 0.82 | 0.64 | 2.12 | 0.00 | 0.90 | 0.76 |
| main | all | SCORE1 | 52 | 1.68 | 0.00 | 0.78 | 0.60 | 1.85 | 0.00 | 0.83 | 0.62 | 2.30 | 0.00 | 0.91 | 0.74 |
| main | all | SCORE1 | 53 | 1.58 | 0.01 | 0.78 | 0.61 | 1.71 | 0.00 | 0.82 | 0.63 | 2.28 | 0.00 | 0.91 | 0.74 |
| main | all | SCORE1 | 54 | 1.51 | 0.01 | 0.77 | 0.61 | 1.67 | 0.00 | 0.82 | 0.63 | 2.27 | 0.00 | 0.90 | 0.74 |
| main | all | SCORE1 | 55 | 1.47 | 0.02 | 0.77 | 0.62 | 1.60 | 0.01 | 0.81 | 0.64 | 2.20 | 0.00 | 0.90 | 0.74 |
| main | all | SCORE1 | 56 | 1.48 | 0.02 | 0.76 | 0.62 | 1.60 | 0.01 | 0.81 | 0.64 | 2.19 | 0.00 | 0.90 | 0.73 |
| main | all | SCORE1 | 57 | 1.43 | 0.03 | 0.76 | 0.62 | 1.54 | 0.02 | 0.80 | 0.65 | 2.06 | 0.00 | 0.90 | 0.74 |
| main | all | SCORE1 | 58 | 1.33 | 0.10 | 0.75 | 0.64 | 1.41 | 0.06 | 0.79 | 0.66 | 1.91 | 0.00 | 0.89 | 0.75 |
| main | all | SCORE1 | 59 | 1.29 | 0.15 | 0.75 | 0.64 | 1.35 | 0.11 | 0.79 | 0.66 | 1.86 | 0.00 | 0.89 | 0.74 |
| main | all | SCORE1 | 60 | 1.27 | 0.18 | 0.74 | 0.65 | 1.37 | 0.09 | 0.79 | 0.66 | 1.86 | 0.00 | 0.89 | 0.75 |
| main | all | SCORE1 | 61 | 1.34 | 0.10 | 0.74 | 0.64 | 1.45 | 0.05 | 0.79 | 0.65 | 1.96 | 0.00 | 0.89 | 0.74 |
| main | all | SCORE1 | 62 | 1.26 | 0.19 | 0.74 | 0.65 | 1.36 | 0.10 | 0.78 | 0.66 | 1.81 | 0.01 | 0.88 | 0.75 |
| main | all | SCORE1 | 63 | 1.24 | 0.23 | 0.74 | 0.64 | 1.39 | 0.09 | 0.78 | 0.66 | 1.81 | 0.01 | 0.88 | 0.75 |
| main | all | SCORE1 | 64 | 1.28 | 0.17 | 0.74 | 0.64 | 1.43 | 0.06 | 0.79 | 0.65 | 1.86 | 0.00 | 0.88 | 0.75 |
| main | all | SCORE1 | 65 | 1.28 | 0.17 | 0.74 | 0.64 | 1.42 | 0.07 | 0.79 | 0.65 | 1.83 | 0.01 | 0.88 | 0.75 |
| main | all | SCORE1 | 66 | 1.32 | 0.13 | 0.74 | 0.63 | 1.52 | 0.03 | 0.79 | 0.63 | 1.95 | 0.00 | 0.88 | 0.74 |
| main | all | SCORE1 | 67 | 1.26 | 0.21 | 0.74 | 0.64 | 1.44 | 0.07 | 0.78 | 0.64 | 1.88 | 0.00 | 0.87 | 0.74 |
| main | all | SCORE1 | 68 | 1.31 | 0.16 | 0.74 | 0.63 | 1.49 | 0.04 | 0.78 | 0.63 | 1.92 | 0.00 | 0.87 | 0.74 |
| main | all | SCORE1 | 69 | 1.27 | 0.21 | 0.74 | 0.62 | 1.51 | 0.04 | 0.78 | 0.63 | 1.92 | 0.00 | 0.87 | 0.74 |
| main | all | SCORE1 | 70 | 1.20 | 0.37 | 0.73 | 0.64 | 1.41 | 0.09 | 0.78 | 0.64 | 1.78 | 0.01 | 0.87 | 0.73 |
| main | all | SCORE1 | 71 | 1.09 | 0.66 | 0.73 | 0.66 | 1.28 | 0.24 | 0.77 | 0.66 | 1.65 | 0.03 | 0.87 | 0.74 |
| main | all | SCORE1 | 72 | 1.09 | 0.69 | 0.73 | 0.66 | 1.27 | 0.27 | 0.77 | 0.66 | 1.62 | 0.04 | 0.87 | 0.75 |
| main | all | SCORE1 | 73 | 1.07 | 0.77 | 0.73 | 0.65 | 1.23 | 0.37 | 0.77 | 0.66 | 1.73 | 0.02 | 0.87 | 0.72 |
| main | all | SCORE1 | 74 | 1.02 | 0.93 | 0.72 | 0.66 | 1.16 | 0.53 | 0.76 | 0.67 | 1.58 | 0.07 | 0.86 | 0.74 |
| main | all | SCORE1 | 75 | 1.15 | 0.54 | 0.73 | 0.63 | 1.31 | 0.26 | 0.77 | 0.64 | 1.78 | 0.02 | 0.86 | 0.72 |
| main | all | SCORE1 | 76 | 1.22 | 0.40 | 0.73 | 0.62 | 1.37 | 0.21 | 0.77 | 0.63 | 1.82 | 0.03 | 0.86 | 0.70 |
| main | all | SCORE1 | 77 | 1.10 | 0.70 | 0.72 | 0.65 | 1.23 | 0.43 | 0.76 | 0.66 | 1.60 | 0.10 | 0.86 | 0.73 |
| main | all | SCORE1 | 78 | 1.24 | 0.40 | 0.72 | 0.61 | 1.39 | 0.22 | 0.76 | 0.62 | 1.76 | 0.05 | 0.86 | 0.71 |
| main | all | SCORE1 | 79 | 1.24 | 0.43 | 0.72 | 0.61 | 1.37 | 0.27 | 0.76 | 0.62 | 1.69 | 0.09 | 0.85 | 0.72 |
| main | all | SCORE1 | 80 | 1.25 | 0.43 | 0.72 | 0.59 | 1.37 | 0.29 | 0.76 | 0.60 | 1.66 | 0.11 | 0.86 | 0.71 |
| main | all | SCORE1 | 81 | 1.12 | 0.72 | 0.72 | 0.62 | 1.33 | 0.36 | 0.76 | 0.60 | 1.53 | 0.22 | 0.85 | 0.73 |
| main | all | SCORE1 | 82 | 0.96 | 0.90 | 0.72 | 0.67 | 1.15 | 0.68 | 0.76 | 0.67 | 1.49 | 0.28 | 0.85 | 0.71 |
| main | all | SCORE1 | 83 | 0.97 | 0.94 | 0.71 | 0.69 | 1.17 | 0.67 | 0.75 | 0.69 | 1.75 | 0.13 | 0.85 | 0.68 |
| main | all | SCORE1 | 84 | 0.92 | 0.82 | 0.71 | 0.70 | 1.10 | 0.81 | 0.75 | 0.70 | 1.67 | 0.19 | 0.85 | 0.70 |
| main | all | SCORE1 | 85 | 0.85 | 0.70 | 0.71 | 0.72 | 1.02 | 0.97 | 0.75 | 0.72 | 1.54 | 0.31 | 0.85 | 0.71 |
| main | all | SCORE1 | 86 | 0.81 | 0.64 | 0.71 | 0.73 | 0.96 | 0.93 | 0.75 | 0.73 | 1.46 | 0.41 | 0.85 | 0.72 |
| main | all | SCORE1 | 87 | 0.89 | 0.79 | 0.71 | 0.71 | 1.05 | 0.91 | 0.75 | 0.71 | 1.61 | 0.30 | 0.85 | 0.71 |
| main | all | SCORE1 | 88 | 0.76 | 0.59 | 0.71 | 0.74 | 0.91 | 0.85 | 0.75 | 0.74 | 1.37 | 0.54 | 0.85 | 0.73 |
| main | all | SCORE1 | 89 | 0.81 | 0.68 | 0.71 | 0.72 | 0.97 | 0.95 | 0.75 | 0.72 | 1.45 | 0.47 | 0.85 | 0.71 |
| main | all | SCORE1 | 90 | 1.05 | 0.92 | 0.71 | 0.66 | 1.25 | 0.66 | 0.75 | 0.66 | 1.84 | 0.23 | 0.85 | 0.66 |
| main | all | SCORE1 | 91 | 1.21 | 0.71 | 0.72 | 0.62 | 1.44 | 0.47 | 0.75 | 0.62 | 2.13 | 0.14 | 0.85 | 0.62 |
| main | all | SCORE1 | 92 | 1.59 | 0.36 | 0.72 | 0.53 | 1.88 | 0.21 | 0.75 | 0.53 | 2.77 | 0.05 | 0.85 | 0.53 |
| main | all | SCORE1 | 93 | 1.83 | 0.23 | 0.72 | 0.47 | 2.17 | 0.13 | 0.76 | 0.47 | 3.14 | 0.03 | 0.85 | 0.47 |
| main | all | SCORE1 | 94 | 1.83 | 0.23 | 0.72 | 0.47 | 2.17 | 0.13 | 0.76 | 0.47 | 3.14 | 0.03 | 0.85 | 0.47 |
| main | all | SCORE1 | 95 | 1.41 | 0.56 | 0.72 | 0.54 | 1.70 | 0.37 | 0.75 | 0.54 | 2.43 | 0.13 | 0.85 | 0.54 |
| main | all | SCORE1 | 96 | 1.56 | 0.53 | 0.71 | 0.60 | 1.86 | 0.38 | 0.75 | 0.60 | 2.60 | 0.18 | 0.85 | 0.60 |
| main | all | SCORE1 | 97 | 2.37 | 0.23 | 0.72 | 0.50 | 2.80 | 0.15 | 0.75 | 0.50 | 4.00 | 0.05 | 0.85 | 0.50 |
| main | all | SCORE1 | 98 | 3.32 | 0.09 | 0.72 | 0.33 | 4.03 | 0.05 | 0.75 | 0.33 | 5.17 | 0.02 | 0.85 | 0.33 |
| main | all | SCORE1 | 99 | 1.70 | 0.60 | 0.71 | 0.50 | 2.05 | 0.48 | 0.75 | 0.50 | 2.65 | 0.33 | 0.84 | 0.50 |

### Example 9: Improvement and simplification of scores

The original 4-marker score 1 was found by logistic regression against of pCR defined as (ypT0/is **_ypN0)** in a subset of 462 samples by limiting the whole set (N=598 samples) to the samples with full IHC and clinical data available. After the full data from the training cohort became available, analyses were refined with regard to:
- Usage of all 598 samples (instead of 462);
- Exclusion of MammaTyper^{®} 40-ΔΔCq values which were based on a missing measurement (Cq 40) (N=21);
- Application of shrinkage correction (based on 5000 bootstrapped samples) to unscaled scores to correct for overfitting;
- Normalization of MKI67 by CALM2 only (more precise reference gene, than B2M);
- Setup of models based on the less than 4 markers (3 and 2 markers).

This led to the identification/determination of three scores, in addition to score 1, as possible solutions for the prediction of the probability of pCR, as shown in Tables 15 to 18.

**Table 15. Summary of scores.**

| | **Number of markers** | **Formula of unscaled score** |
|---|---|---|
| **Score 1** | 4 | su = -6.394 + 0.099 * ERBB2 - 0.279 * ESR1 - 0.108 * PGR + 0.426 * MKI67 |
| **Score 2** | 4 | su = -13.413 + 0.117 * ERBB2 - 0.288 * ESR1 - 0.067 * PGR + 0.508 * MKI67 |
| **Score 3** | 3 | su = -15.209 + 0.114 * ERBB2 -0.335 * ESR1 + 0.539 * MKI67 |
| **Score 4** | 2 | su = -10.625 - 0.324 * ESR1 + 0.527 * MKI67 |

**Table 15. Weighting of individual markers in scores 1 to 4 (ESR1 = 1).**

| | **ERBB2 (+/- 15%)** | | **ESR1 (+/- 15%)** | | **PGR (+/- 15%)** | | **MKI67 (+/- 15%)** | |
|---|---|---|---|---|---|---|---|---|
| **Score 1** | -0.35 | +/- 0.05 | 1 | +/- 0.15 | 0.39 | +/- 0.06 | -1.53 | +/- 0.23 |
| **Score 2** | -0.41 | +/- 0.06 | 1 | +/- 0.15 | 0.23 | +/- 0.03 | -1.76 | +/- 0.26 |
| **Score 3** | -0.34 | +/- 0.05 | 1 | +/- 0.15 | | | -1.61 | +/- 0.24 |
| **Score 4** | | | 1 | +/- 0.15 | | | -1.63 | +/- 0.24 |

**Table 17. AUC values of scores 1-4 for prediction of pCR/is in 598 samples**

| | **AUC** | | | **SE** |
|---|---|---|---|---|
| | **Estimate** | **95% CI** | | |
| **Score 1** | 0.789 | 0.751 | 0.827 | 0.019 |
| **Score 2** | 0.801 | 0.763 | 0.838 | 0.019 |
| **Score 3** | 0.802 | 0.765 | 0.839 | 0.019 |
| **Score 4** | 0.801 | 0.763 | 0.839 | 0.019 |

**Table 18. Comparison of AUCs (prediction of pCR/is) of scores 1-4 to test for equality. p<0.05 is regarded as significant**

| **Comparators** | | | **Difference** | | | **SE** | **Z** | **p-value** |
|---|---|---|---|---|---|---|---|---|
| | | | **Estimate** | **95% CI** | | | | |
| **Score 3** | **vs** | **Score 1** | 0.013 | 0.003 | 0.023 | 0.005 | 2.487 | **0.0129** |
| **Score 4** | **vs** | **Score 1** | 0.012 | -0.001 | 0.025 | 0.007 | 1.855 | **0.0637** |
| **Score 2** | **vs** | **Score 1** | 0.012 | 0.003 | 0.021 | 0.004 | 2.675 | **0.0075** |
| **Score 3** | **vs** | **Score 2** | 0.001 | -0.003 | 0.005 | 0.002 | 0.496 | 0.6196 |
| **Score 3** | **vs** | **Score 4** | 0.001 | -0.009 | 0.011 | 0.005 | 0.157 | 0.8754 |
| **Score 4** | **vs** | **Score 2** | 0.000 | -0.010 | 0.011 | 0.005 | 0.042 | 0.9669 |

### Example 10: Validation of score 1 in an independent cohort

Score 1 was tested as a predictor of success of neo-adjuvant chemotherapy (NACT) (+/- anti HER2), measured as pCR, in a retrospective analysis of archived samples from a single center.

85 FFPE biopsy samples from the years 2012-2018 were sourced from the archive. Samples with >20% tumor cell content were subjected to RNA extraction. Relative mRNA expression levels of ERBB2, ESR1, PGR and MKI67 were determined by RT-qPCR using the CE-IVD MammaTyper^{®} kit. The association of continuous and binary score 1 results with pCR (defined as ypT0/Tis) and partial response was analyzed.

Marker positivity rates of the 75 samples included in the final analysis were 62.7% of ER, 53.3% for PR, 40.0% for HER2 and 94.7% for Ki67 (≥20% pos cells). 42.7% of patients were pre-menopausal and all samples except one were grade 3. pCR rates over all samples and in hormone receptor (HR)-positive/HER2-negative patients only were 48.0% and 20.0%, respectively. The binary score 1 result was significantly associated with pCR over all patients (Sensitivity: 88.9%, Specificity: 51.3%, PPV: 62.8%, NPV: 83.3%) and also in HR-positive/HER2-negative patients only (Sensitivity: 83.3%, Specificity: 70.8%, PPV: 41.7%, NPV: 94.4%). ROC analysis revealed a good association of the continuous score 1 with achievement of pCR over all patients (AUC=0.756) and in the subgroup of HR+/HER2-patients (AUC=0.774). pCR rates according to St. Gallen surrogate subtype definition were similar for IHC and RT-qPCR defined subtypes in triple-negative (80.0% and 78.6% respectively) and in the HER2+ non-luminal subtype (75.0%, and 70.0% respectively). In tumors with incomplete response the continuous score 1 was significantly associated with residual tumor size (Spearman rs: -0.477 p-value: 0.0021) and %-decrease of tumor size (Spearman rs: 0.388, p-value: 0.0147).

These data confirm that score 1 may serve as a standardized tool to predict response to NACT based on a pre-treatment biopsy. For patients with inoperable luminal tumors and low predicted probability of pCR, neo-adjuvant aromatase inhibitor alone or combined with the new generation of TKIs or CDK4/6 inhibitors or Pi3KCa/mTOR inhibitors may be an alternative for downstaging of tumors.

## Claims

1. Method of predicting the probability of pathological complete response (pCR) of a breast cancer patient upon neo-adjuvant chemotherapy, said method comprising:
calculating a score unscaled (su) based on the relative expression levels of mRNA of
i) ERBB2, ESR1, PGR and MKI67,
ii) ERBB2, ESR1 and MKI67 or
iii) ESR1 and MKI67
relative to the level of expression of one or more reference genes selected from the group comprising B2M, CALM2, TBP, PUM1, MRLP19, GUSB, RPL37A and CYFIP1 in a pre-treatment breast tumor sample of the breast cancer patient as determined by reverse transcription quantitative PCR (RT-qPCR), wherein
a) a higher score su indicates a higher probability of pCF, wherein a higher relative expression level of mRNA of ERBB2 is associated with a higher su, a higher relative expression level of mRNA of ESR1 is associated with a lower su, a higher relative expression level of mRNA of PGR is associated with a lower su, and a higher relative expression level of mRNA of MKI67 is associated with a higher su; or
b) a lower score su indicates a higher probability of pCR, wherein a higher relative expression level of mRNA of ERBB2 is associated with a lower su, a higher relative expression level of mRNA of ESR1 is associated with a higher su, a higher relative expression level of mRNA of PGR is associated with a higher su, and a higher relative expression level of mRNA of MKI67 is associated with a lower su,
wherein the method comprises, prior to calculating su: determining the relative expression levels of mRNA of
i) ERBB2, ESR1, PGR and MKI67,
ii) ERBB2, ESR1 and MKI67 or
iii) ESR1 and MKI67, respectively,
in the pre-treatment breast tumor sample by RT-qPCR.

2. The method according to claim 1, wherein the neo-adjuvant chemotherapy comprises administration of a taxane, optionally wherein the neo-adjuvant chemotherapy is accompanied by the administration of an anti-ERBB2 drug if the breast cancer is an ERBB2-positive breast cancer.

3. The method according to claim 1 or 2, wherein the breast cancer is i) a luminal breast cancer, and/or ii) an ESR1- and/or PGR-positive breast cancer.

4. The method according to any one of claims 1 to 3, wherein,
i) the su is calculated based on the relative expression levels of mRNA of ERBB2, ESR1, PGR and MKI67 and in the calculation of su, the relative expression levels (RELs) of mRNA of ERBB2, ESR1, PGR and MKI67 are weighted as follows: 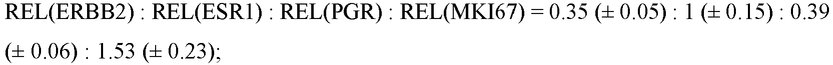 REL(ERBB2) : REL(ESR1) : REL(PGR) : REL(MKI67) = 0.35 (± 0.05) : 1 (± 0.15) : 0.39 (± 0.06) : 1.53 (± 0.23); or 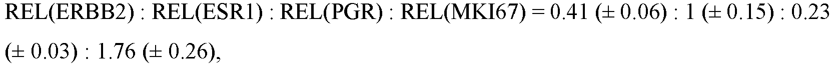 REL(ERBB2) : REL(ESR1) : REL(PGR) : REL(MKI67) = 0.41 (± 0.06) : 1 (± 0.15) : 0.23 (± 0.03) : 1.76 (± 0.26),
ii) the su is calculated based on the relative expression levels of mRNA of ERBB2, ESR1 and MKI67 and in the calculation of su, the relative expression levels (RELs) of mRNA of ERBB2, ESR1 and MKI67 are weighted as follows: $REL \left(ERBB2\right) : REL \left(ESR1\right) : REL \left(MKI67\right) = 0.34 \left(\pm 0.05\right) : 1 \left(\pm 0.15\right) : 1.61 \left(\pm 0.24\right) ,$ or
iii) the su is calculated based on the relative expression levels of mRNA of ESR1 and MKI67 and in the calculation of su, the relative expression levels (RELs) of mRNA of ESR1 and MKI67 are weighted as follows: $REL \left(ESR1\right) : REL \left(MKI67\right) = 1 \left(\pm 0.15\right) : 1.63 \left(\pm 0.24\right) .$

5. The method according to claim 4, wherein
i) the su is calculated based on the relative expression levels of mRNA of ERBB2, ESR1, PGR and MKI67, and
a) a higher score su indicates a higher probability of pCR, and wherein su is calculated by using the formula: 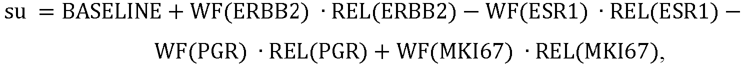 su = BASELINE + WF(ERBB2) · REL(ERBB2) - WF(ESR1) · REL(ESR1) - WF(PGR) · REL(PGR) + WF(MKI67) · REL(MKI67), or
b) a lower score su indicates a higher probability of pCF, and wherein su is calculated by using the formula: 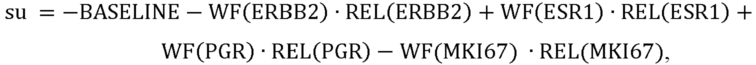 su = -BASELINE - WF(ERBB2) · REL(ERBB2) + WF(ESR1) · REL(ESR1) + WF(PGR) · REL(PGR) - WF(MKI67) · REL(MKI67),
ii) the su is calculated based on the relative expression levels of mRNA of ERBB2, ESR1 and MKI67, and
a) wherein a higher score su indicates a higher probability of pCR, and wherein su is calculated by using the formula: 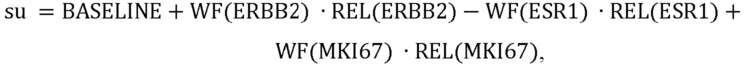 su = BASELINE + WF(ERBB2) · REL(ERBB2) - WF(ESR1) · REL(ESR1) + WF(MKI67) · REL(MKI67), or
b) wherein a lower score su indicates a higher probability of pCF, and wherein su is calculated by using the formula: 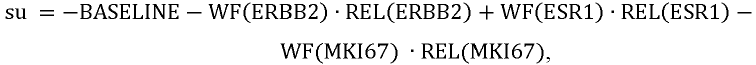 su = -BASELINE - WF(ERBB2) · REL(ERBB2) + WF(ESR1) · REL(ESR1) - WF(MKI67) · REL(MKI67), or
iii) the su is calculated based on the relative expression levels of mRNA of ESR1 and MKI67, wherein, in the calculation of su, the relative expression levels (RELs) of mRNA of ESR1 and MKI67 are weighted as follows: $REL \left(ESR1\right) : REL \left(MKI67\right) = 1 \left(\pm 0.15\right) : 1.63 \left(\pm 0.24\right) ,$
a) wherein a higher score su indicates a higher probability of pCR, and wherein su is calculated by using the formula: $su = BASELINE - WF \left(ESR1\right) ⋅ REL \left(ESR1\right) + WF \left(MKI67\right) ⋅ REL \left(MKI67\right)$ or
b) wherein a lower score su indicates a higher probability of pCF, and wherein su is calculated by using the formula: $su = - BASELINE + WF \left(ESR1\right) ⋅ REL \left(ESR1\right) - WF \left(MKI67\right) ⋅ REL \left(MKI67\right) ,$
wherein, respectively, WF(ERBB2) is a weighting factor for REL(ERBB2), WF(ESR1) is a weighting factor for REL(ESR1), WF(PGR) is a weighting factor for REL(PGR2), and WF(MKI67) is a weighting factor for REL(MKI67).

6. The method according to any one of claims 1 to 5, wherein
i) the su is calculated based on the relative expression levels of mRNA of ERBB2, ESR1, PGR and MKI67, and
a) a higher score su indicates a higher probability of pCR, and wherein su is calculated by using the formula: 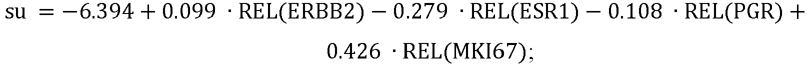 su = -6.394 + 0.099 · REL(ERBB2) - 0.279 · REL(ESR1) - 0.108 · REL(PGR) + 0.426 · REL(MKI67); or 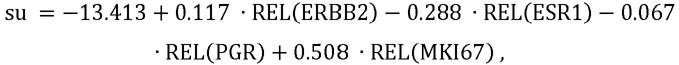 su = -13.413 + 0.117 · REL(ERBB2) - 0.288 · REL(ESR1) - 0.067 · REL(PGR) + 0.508 · REL(MKI67), or
b) wherein a lower score su indicates a higher probability of pCR, and wherein su is calculated by using the formula: 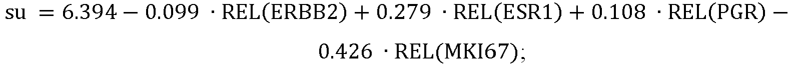 su = 6.394 - 0.099 · REL(ERBB2) + 0.279 · REL(ESR1) + 0.108 · REL(PGR) - 0.426 · REL(MKI67); or 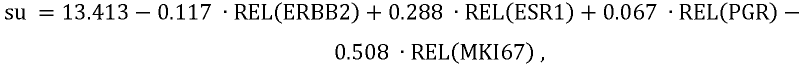 su = 13.413 - 0.117 · REL(ERBB2) + 0.288 · REL(ESR1) + 0.067 · REL(PGR) - 0.508 · REL(MKI67),
ii) the su is calculated based on the relative expression levels of mRNA of ERBB2, ESR1 and MKI67, and
a) wherein a higher score su indicates a higher probability of pCR, and wherein su is calculated by using the formula: $su = - 15.209 + 0.114 ⋅ REL \left(ERBB2\right) - 0.335 ⋅ REL \left(ESR1\right) + 0.539 ⋅ REL \left(MKI67\right) ,$ or
b) wherein a lower score su indicates a higher probability of pCR, and wherein su is calculated by using the formula: 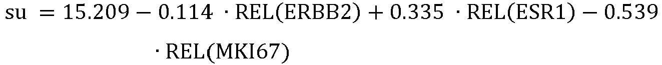 su = 15.209 - 0.114 · REL(ERBB2) + 0.335 · REL(ESR1) - 0.539 · REL(MKI67) or
iii) the su is calculated based on the relative expression levels of mRNA of ESR1 and MKI67, and
a) wherein a higher score su indicates a higher probability of pCR, and wherein su is calculated by using the formula: $su = - 10.625 - 0.324 ⋅ REL \left(ESR1\right) + 0.527 ⋅ REL \left(MKI67\right)$ or
b) wherein a lower score su indicates a higher probability of pCF, and wherein su is calculated by using the formula: $su = 10.625 + 0.324 ⋅ REL \left(ESR1\right) - 0.527 ⋅ REL \left(MKI67\right) .$

7. The method according to any one of claims 1 to 6, further comprising:
calculating a predicted probability of pCR q, wherein
a) if a higher score su indicates a higher probability of pCR, q is calculated by using the formula $q = \frac{exp \left(su\right)}{\left(1+exp\left(su\right)\right)} ;$ and
b) if a lower score su indicates a higher probability of pCR, q is calculated by using the formula $q = 1 - \frac{exp \left(su\right)}{\left(1+exp\left(su\right)\right)} ,$
wherein, preferably, a q which is equal to or greater than a pre-defined threshold indicates a high probability of pCR, and a q which is lower than a pre-defined threshold indicates a low probability of pCR.

8. The method according to any one of claims 1 to 7, further comprising:
calculating a clinical score s based on su, wherein s has a scale from 0 to 100.

9. The method according to claim 6, wherein the su is calculated based on the relative expression levels of mRNA of ERBB2, ESR1, PGR and MKI67 using the formula 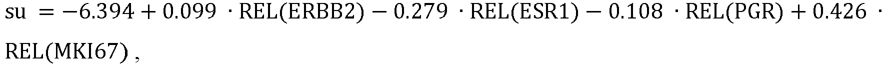 su = -6.394 + 0.099 · REL(ERBB2) - 0.279 · REL(ESR1) - 0.108 · REL(PGR) + 0.426 · REL(MKI67) , and wherein the method further comprises:
calculating a clinical score s based on su, wherein s is calculated by using the formula $s = \left(su+3.960\right) ⋅ 18.191 \left(round to 0 decimal places\right) ,$ wherein
| | | |
|---|---|---|
| if (su + 3.960) · 18.191 < 0 | s = 0, | and |
| if (su + 3.960) · 18.191 > 100 | s = 100. | |

10. The method according to any one of claims 1 to 6, 8 and 9, wherein
a) if a higher score su indicates a higher probability of pCR, a score s or a score su which is equal to or greater than a pre-defined threshold indicates a high probability of pCR, and a score s or a score su which is lower than the pre-defined threshold indicates a low probability of pCR; and
b) if a lower score su indicates a higher probability of pCR, a score s or a score su which is lower than a pre-defined threshold indicates a high probability of pCR, and a score s or a score su which is equal to or greater than the pre-defined threshold indicates a low probability of pCR.

11. Method for selecting a breast cancer treatment for a breast cancer patient, said method comprising:
calculating a score unscaled (su) based on the relative expression levels of mRNA of ERBB2, ESR1, PGR and/or MKI67 in a pre-treatment breast tumor sample of the breast cancer patient as defined in any one of claims 1, 4 to 6 and 10, and, optionally, a predicted probability of pCR q as defined in claim 7, or a clinical score s as defined in any one of claims 8 to 10; and
selecting a breast cancer treatment for the breast cancer patient based on su and, optionally, q or s, wherein
a) if a higher score su indicates a higher probability of pCR,
- neo-adjuvant chemotherapy is selected if su and, optionally, q or s are equal to or greater than a pre-defined threshold; and/or
- a breast cancer treatment selected from the group consisting of adjuvant chemotherapy, a non-chemotherapeutic treatment and endocrine therapy is selected if su and, optionally, q or s are lower than the pre-defined threshold; and
b) if a lower score su indicates a higher probability of pCR,
- neo-adjuvant chemotherapy is selected if su and, optionally, s are lower than a pre-defined threshold;
- neo-adjuvant chemotherapy is selected if q is equal to or greater than a pre-defined threshold;
- a breast cancer treatment selected from the group consisting of adjuvant chemotherapy, a non-chemotherapeutic treatment and endocrine therapy is selected if su and, optionally, s are equal to or greater than the pre-defined threshold; and/or
- a breast cancer treatment selected from the group consisting of adjuvant chemotherapy, a non-chemotherapeutic treatment and endocrine therapy is selected if q is lower than the pre-defined threshold,
wherein the method comprises, prior to calculating su and, optionally, q or s: determining the relative expression levels of mRNA of ERBB2, ESR1, PGR and/or MKI67 in the pre-treatment breast tumor sample by RT-qPCR.

12. The method according to claim 11, wherein the neo-adjuvant or adjuvant chemotherapy comprises administration of a taxane, wherein the endocrine therapy is administered in an adjuvant or a neo-adjuvant setting and/or wherein the neo-adjuvant chemotherapy or the endocrine therapy is accompanied by the administration of an anti-ERBB2 drug if the breast cancer is an ERBB2-positive breast cancer, wherein optionally the breast cancer is i) a luminal breast cancer, and/or ii) an ESR1- and/or PGR-positive breast cancer.

13. Method of prognosis of breast cancer in a breast cancer patient upon breast cancer treatment, said method comprising:
calculating a score unscaled (su) based on the relative expression levels of mRNA of ERBB2, ESR1, PGR and/or MKI67 in a pre-treatment breast tumor sample of the breast cancer patient as defined in any one of claims 1, 4 to 6 and 10, and, optionally, a predicted probability of pCR q as defined in claim 7, or a clinical score s as defined in any one of claims 8 to 10, wherein
a) if a higher score su indicates a higher probability of pCR, an su and, optionally, q or s which are equal to or greater than a pre-defined threshold indicate a negative prognosis, and/or an su and, optionally, q or s which are lower than a pre-defined threshold indicate a positive prognosis; and
b) if a lower score su indicates a higher probability of pCR, i) an su and, optionally, s which are equal to or greater than a pre-defined threshold indicate a positive prognosis, and/or an su and, optionally, s which are lower than a pre-defined threshold indicate a negative prognosis, and ii) a q which is equal to or greater than a pre-defined threshold indicates a negative prognosis, and/or a q which is lower than a pre-defined threshold indicates a positive prognosis,
wherein the method comprises, prior to calculating su and, optionally, q or s: determining the relative expression levels of mRNA of ERBB2, ESR1, PGR and/or MKI67 in the pre-treatment breast tumor sample by RT-qPCR.

14. The method according to claim 13, wherein the positive prognosis comprises an increased/high probability of distant recurrence-free survival (DRFS), disease-free survival (DFS) and/or overall survival (OS) and/or wherein the negative prognosis comprises a reduced/low probability of distant recurrence-free survival (DRFS), disease-free survival (DFS) and/or overall survival (OS).

15. The method according to claim 13 or 14, wherein the breast cancer treatment comprises neo-adjuvant or adjuvant chemotherapy, wherein optionally the breast cancer treatment comprises adjuvant endocrine therapy.

16. Use of a kit in a method according to any one of claims 1, 11 and 13 wherein the kit comprises:
at least one pair of ERBB2-specific primers;
at least one pair of ESR1-specific primers;
at least one pair of PGR-specific primers; and/or
at least one pair of MKI67-specific primers.

17. The use according to claim 16, wherein the kit further comprises at least one ERBB2-specific probe, at least one ESR1-specific probe, at least one PGR-specific probe and/or at least one MKI67-specific probe and/or at least one pair of reference gene-specific primers and, optionally, at least one reference gene-specific probe, wherein further optionally the reference gene is selected from the group consisting of B2M, CALM2, TBP, PUM1, MRLP19, GUSB, RPL37A and CYFIP1.

## Patentansprüche

1. Verfahren zur Vorhersage der Wahrscheinlichkeit eines pathologischen kompletten Ansprechens (pCR) eines Brustkrebspatienten nach einer neoadjuvanten Chemotherapie, wobei das Verfahren umfasst:
Berechnen eines unskalierten Scores (su) basierend auf den relativen Expressionsleveln von mRNA von
i) ERBB2, ESR1, PGR und MKI67,
ii) ERBB2, ESR1 und MKI67 oder
iii) ESR1 und MKI67
relativ zum Expressionslevel eines oder mehrerer Referenzgene, ausgewählt aus der Gruppe umfassend B2M, CALM2, TBP, PUM1, MRLP19, GUSB, RPL37A und CYFIP1, in einer vor der Behandlung entnommenen Brusttumorprobe des Brustkrebspatienten, wie durch quantitative Reverse-Transkriptions-PCR (RT-qPCR) bestimmt, wobei
a) ein höherer su-Score eine höhere Wahrscheinlichkeit für pCR anzeigt, wobei ein höherer relativer Expressionslevel von mRNA von ERBB2 mit einem höheren su assoziiert ist, ein höherer relativer Expressionslevel von mRNA von ESR1 mit einem niedrigeren su assoziiert ist, ein höherer relativer Expressionslevel von mRNA von PGR mit einem niedrigeren su assoziiert ist, und ein höherer relativer Expressionslevel von mRNA von MKI67 mit einem höheren su assoziiert ist; oder
b) ein niedrigerer su-Score eine höhere Wahrscheinlichkeit für pCR anzeigt, wobei ein höherer relativer Expressionslevel von mRNA von ERBB2 mit einem niedrigeren su assoziiert ist, ein höherer relativer Expressionslevel von mRNA von ESR1 mit einem höheren su assoziiert ist, ein höherer relativer Expressionslevel von mRNA von PGR mit einem höheren su assoziiert ist, und ein höherer relativer Expressionslevel von mRNA von MKI67 mit einem niedrigeren su assoziiert ist,
wobei das Verfahren vor dem Berechnen von su umfasst: Bestimmen der relativen Expressionslevel von mRNA von
i) ERBB2, ESR1, PGR und MKI67,
ii) ERBB2, ESR1 und MKI67 oder
iii) ESR1 und MKI67, jeweils,
in der vor der Behandlung entnommenen Brusttumorprobe durch RT-qPCR.

2. Verfahren nach Anspruch 1, wobei die neoadjuvante Chemotherapie die Verabreichung eines Taxans umfasst, wobei fakultativ die neoadjuvante Chemotherapie von der Verabreichung eines Anti-ERBB2-Wirkstoffs begleitet wird, wenn es sich bei dem Brustkrebs um einen ERBB2-positiven Brustkrebs handelt.

3. Verfahren nach Anspruch 1 oder 2, wobei der Brustkrebs i) ein luminaler Brustkrebs und/oder ii) ein ESR1- und/oder PGR-positiver Brustkrebs ist.

4. Verfahren nach einem der Ansprüche 1 bis 3, wobei,
i) der su basierend auf den relativen Expressionsleveln von mRNA von ERBB2, ESR1, PGR und MKI67 berechnet wird und bei der Berechnung von su die relativen Expressionslevel (REL) von mRNA von ERBB2, ESR1, PGR und MKI67 wie folgt gewichtet werden: 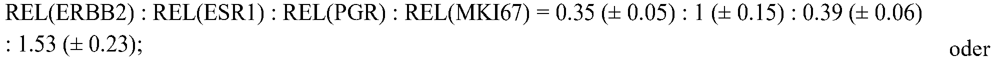 REL(ERBB2) : REL(ESR1) : REL(PGR) : REL(MKI67) = 0.35 (± 0.05) : 1 (± 0.15) : 0.39 (± 0.06) : 1.53 (± 0.23); oder 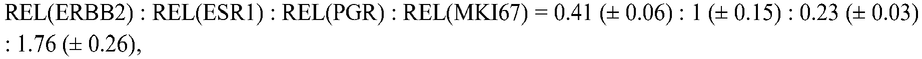 REL(ERBB2) : REL(ESR1) : REL(PGR) : REL(MKI67) = 0.41 (± 0.06) : 1 (± 0.15) : 0.23 (± 0.03) : 1.76 (± 0.26),
ii) der su basierend auf den relativen Expressionsleveln von mRNA von ERBB2, ESR1 und MKI67 berechnet wird und bei der Berechnung von su die relativen Expressionslevel (REL) von mRNA von ERBB2, ESR1 und MKI67 wie folgt gewichtet werden: $REL \left(ERBB2\right) : REL \left(ESR1\right) : REL \left(MKI67\right) = 0.34 \left(\pm 0.05\right) : 1 \left(\pm 0.15\right) : 1.61 \left(\pm 0.24\right) , oder$
iii) der su basierend auf den relativen Expressionsleveln von mRNA von ESR1 und MKI67 berechnet wird und bei der Berechnung von su die relativen Expressionslevel (REL) von mRNA von ESR1 und MKI67 wie folgt gewichtet werden: $REL \left(ESR1\right) : REL \left(MKI67\right) = 1 \left(\pm 0.15\right) : 1.63 \left(\pm 0.24\right) .$

5. Verfahren nach Anspruch 4, wobei
i) der su basierend auf den relativen Expressionsleveln von mRNA von ERBB2, ESR1, PGR und MKI67 berechnet wird, und
a) ein höherer su-Score eine höhere Wahrscheinlichkeit für pCR anzeigt, und wobei su unter Verwendung der Formel berechnet wird: 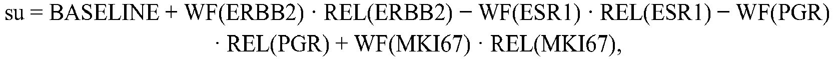 su = BASELINE + WF(ERBB2) · REL(ERBB2) - WF(ESR1) · REL(ESR1) - WF(PGR) · REL(PGR) + WF(MKI67) · REL(MKI67), oder
b) ein niedrigerer su-Score eine höhere Wahrscheinlichkeit für pCR anzeigt, und wobei su unter Verwendung der Formel berechnet wird: 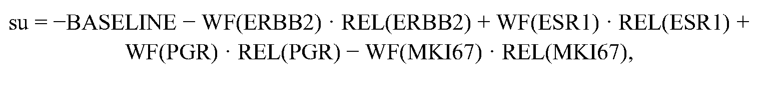 su = -BASELINE - WF(ERBB2) · REL(ERBB2) + WF(ESR1) · REL(ESR1) + WF(PGR) · REL(PGR) - WF(MKI67) · REL(MKI67),
ii) der su basierend auf den relativen Expressionsleveln von mRNA von ERBB2, ESR1 und MKI67 berechnet wird, und
a) wobei ein höherer su-Score eine höhere Wahrscheinlichkeit für pCR anzeigt, und wobei su unter Verwendung der Formel berechnet wird: 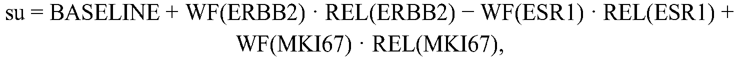 su = BASELINE + WF(ERBB2) · REL(ERBB2) - WF(ESR1) · REL(ESR1) + WF(MKI67) · REL(MKI67), oder
b) wobei ein niedrigerer su-Score eine höhere Wahrscheinlichkeit für pCR anzeigt, und wobei su unter Verwendung der Formel berechnet wird: 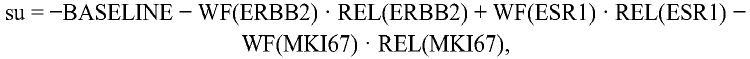 su = -BASELINE - WF(ERBB2) · REL(ERBB2) + WF(ESR1) · REL(ESR1) - WF(MKI67) · REL(MKI67), oder
iii) der su basierend auf den relativen Expressionsleveln von mRNA von ESR1 und MKI67 berechnet wird, wobei bei der Berechnung von su die relativen Expressionslevel (REL) von mRNA von ESR1 und MKI67 wie folgt gewichtet werden: $REL \left(ESR1\right) : REL \left(MKI67\right) = 1 \left(\pm 0.15\right) : 1.63 \left(\pm 0.24\right) ,$
a) wobei ein höherer su-Score eine höhere Wahrscheinlichkeit für pCR anzeigt, und wobei su unter Verwendung der Formel berechnet wird: $su = BASELINE - WF \left(ESR1\right) ⋅ REL \left(ESR1\right) + WF \left(MKI67\right) ⋅ REL \left(MKI67\right)$ oder
b) wobei ein niedrigerer su-Score eine höhere Wahrscheinlichkeit für pCR anzeigt, und wobei su unter Verwendung der Formel berechnet wird: $su = - BASELINE + WF \left(ESR1\right) ⋅ REL \left(ESR1\right) - WF \left(MKI67\right) ⋅ REL \left(MKI67\right) ,$
wobei jeweils WF(ERBB2) ein Gewichtungsfaktor für REL(ERBB2) ist, WF(ESR1) ein Gewichtungsfaktor für REL(ESR1) ist, WF(PGR) ein Gewichtungsfaktor für REL(PGR2) ist, und WF(MKI67) ein Gewichtungsfaktor für REL(MKI67) ist.

6. Verfahren nach einem der Ansprüche 1 bis 5, wobei
i) der su basierend auf den relativen Expressionsleveln von mRNA von ERBB2, ESR1, PGR und MKI67 berechnet wird, und
a) ein höherer su-Score eine höhere Wahrscheinlichkeit für pCR anzeigt, und wobei su unter Verwendung der Formel berechnet wird: 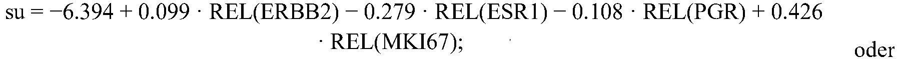 su = -6.394 + 0.099 · REL(ERBB2) - 0.279 · REL(ESR1) - 0.108 · REL(PGR) + 0.426 · REL(MKI67); oder 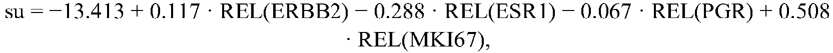 su = -13.413 + 0.117 · REL(ERBB2) - 0.288 · REL(ESR1) - 0.067 · REL(PGR) + 0.508 · REL(MKI67), oder
b) wobei ein niedrigerer su-Score eine höhere Wahrscheinlichkeit für pCR anzeigt, und wobei su unter Verwendung der Formel berechnet wird: 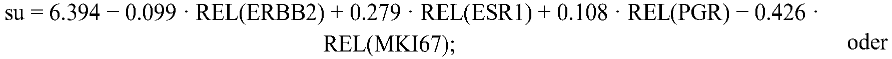 su = 6.394 - 0.099 · REL(ERBB2) + 0.279 · REL(ESR1) + 0.108 · REL(PGR) - 0.426 · REL(MKI67); oder 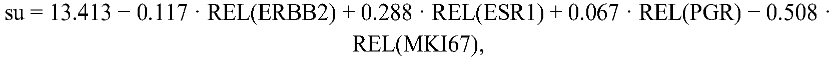 su = 13.413 - 0.117 · REL(ERBB2) + 0.288 · REL(ESR1) + 0.067 · REL(PGR) - 0.508 · REL(MKI67),
ii) der su basierend auf den relativen Expressionsleveln von mRNA von ERBB2, ESR1 und MKI67 berechnet wird, und
a) wobei ein höherer su-Score eine höhere Wahrscheinlichkeit für pCR anzeigt, und wobei su unter Verwendung der Formel berechnet wird: $su = - 15.209 + 0.114 ⋅ REL \left(ERBB2\right) - 0.335 ⋅ REL \left(ESR1\right) + 0.539 ⋅ REL \left(MKI67\right) ,$ oder
b) wobei ein niedrigerer su-Score eine höhere Wahrscheinlichkeit für pCR anzeigt, und wobei su unter Verwendung der Formel berechnet wird: $su = 15.209 - 0.114 ⋅ REL \left(ERBB2\right) + 0.335 ⋅ REL \left(ESR1\right) - 0.539 ⋅ REL \left(MKI67\right)$ oder
iii) der su basierend auf den relativen Expressionsleveln von mRNA von ESR1 und MKI67 berechnet wird, und
a) wobei ein höherer su-Score eine höhere Wahrscheinlichkeit für pCR anzeigt, und wobei su unter Verwendung der Formel berechnet wird: $su = - 10.625 - 0.324 ⋅ REL \left(ESR1\right) + 0.527 ⋅ REL \left(MKI67\right)$ oder
b) wobei ein niedrigerer su-Score eine höhere Wahrscheinlichkeit für pCR anzeigt, und wobei su unter Verwendung der Formel berechnet wird: $su = 10.625 + 0.324 ⋅ REL \left(ESR1\right) - 0.527 ⋅ REL \left(MKI67\right) .$

7. Verfahren nach einem der Ansprüche 1 bis 6, ferner umfassend:
Berechnen einer vorhergesagten Wahrscheinlichkeit für pCR, q, wobei
a) wenn ein höherer su-Score eine höhere Wahrscheinlichkeit für pCR anzeigt, q unter Verwendung der Formel berechnet wird $q = \frac{exp \left(su\right)}{\left(1+exp\left(su\right)\right)} ;$ und
b) wenn ein niedrigerer su-Score eine höhere Wahrscheinlichkeit für pCR anzeigt, q unter Verwendung der Formel berechnet wird $q = 1 - \frac{exp \left(su\right)}{\left(1+exp\left(su\right)\right)} ,$
wobei vorzugsweise ein q, das gleich oder größer als ein vordefinierter Schwellenwert ist, eine hohe Wahrscheinlichkeit für pCR anzeigt, und ein q, das niedriger als ein vordefinierter Schwellenwert ist, eine niedrige Wahrscheinlichkeit für pCR anzeigt.

8. Verfahren nach einem der Ansprüche 1 bis 7, ferner umfassend:
Berechnen eines klinischen Scores s basierend auf su, wobei s eine Skala von 0 bis 100 aufweist.

9. Verfahren nach Anspruch 6, wobei der su basierend auf den relativen Expressionsleveln von mRNA von ERBB2, ESR1, PGR und MKI67 unter Verwendung der Formel berechnet wird $su = - 6.394 + 0.099 ⋅ REL \left(ERBB2\right) - 0.279 ⋅ REL \left(ESR1\right) - 0.108 ⋅ REL \left(PGR\right) + 0.426 ⋅ REL \left(MKI67\right) ,$ und
wobei das Verfahren ferner umfasst:
Berechnen eines klinischen Scores s basierend auf su, wobei s unter Verwendung der Formel berechnet wird $s = \left(su+3.960\right) ⋅ 18.191 \left(gerundet auf 0 Dezimalstellen\right) ,$ wobei
| | |
|---|---|
| wenn (su + 3.960) · 18.191 < 0 | s = 0, und |
| wenn (su + 3.960) · 18.191 > 100 | s = 100. |

10. Verfahren nach einem der Ansprüche 1 bis 6, 8 und 9, wobei
a) wenn ein höherer su-Score eine höhere Wahrscheinlichkeit für pCR anzeigt, ein Score s oder ein Score su, der gleich oder größer als ein vordefinierter Schwellenwert ist, eine hohe Wahrscheinlichkeit für pCR anzeigt, und ein Score s oder ein Score su, der niedriger als der vordefinierte Schwellenwert ist, eine niedrige Wahrscheinlichkeit für pCR anzeigt; und
b) wenn ein niedrigerer su-Score eine höhere Wahrscheinlichkeit für pCR anzeigt, ein Score s oder ein Score su, der niedriger als ein vordefinierter Schwellenwert ist, eine hohe Wahrscheinlichkeit für pCR anzeigt, und ein Score s oder ein Score su, der gleich oder größer als der vordefinierte Schwellenwert ist, eine niedrige Wahrscheinlichkeit für pCR anzeigt.

11. Verfahren zur Auswahl einer Brustkrebsbehandlung für einen Brustkrebspatienten, wobei das Verfahren umfasst:
Berechnen eines unskalierten Scores (su) basierend auf den relativen Expressionsleveln von mRNA von ERBB2, ESR1, PGR und/oder MKI67 in einer vor der Behandlung entnommenen Brusttumorprobe des Brustkrebspatienten, wie in einem der Ansprüche 1, 4 bis 6 und 10 definiert, und, fakultativ, einer vorhergesagten Wahrscheinlichkeit für pCR, q, wie in Anspruch 7 definiert, oder eines klinischen Scores s, wie in einem der Ansprüche 8 bis 10 definiert; und
Auswählen einer Brustkrebsbehandlung für den Brustkrebspatienten basierend auf su und, fakultativ, q oder s, wobei
a) wenn ein höherer su-Score eine höhere Wahrscheinlichkeit für pCR anzeigt,
- eine neoadjuvante Chemotherapie ausgewählt wird, wenn su und, fakultativ, q oder s gleich oder größer als ein vordefinierter Schwellenwert sind; und/oder
- eine Brustkrebsbehandlung, ausgewählt aus der Gruppe bestehend aus adjuvanter Chemotherapie, einer nicht-chemotherapeutischen Behandlung und endokriner Therapie, ausgewählt wird, wenn su und, fakultativ, q oder s niedriger als der vordefinierte Schwellenwert sind; und
b) wenn ein niedrigerer su-Score eine höhere Wahrscheinlichkeit für pCR anzeigt,
- eine neoadjuvante Chemotherapie ausgewählt wird, wenn su und, fakultativ, s niedriger als ein vordefinierter Schwellenwert sind;
- eine neoadjuvante Chemotherapie ausgewählt wird, wenn q gleich oder größer als ein vordefinierter Schwellenwert ist;
- eine Brustkrebsbehandlung, ausgewählt aus der Gruppe bestehend aus adjuvanter Chemotherapie, einer nicht-chemotherapeutischen Behandlung und endokriner Therapie, ausgewählt wird, wenn su und, fakultativ, s gleich oder größer als der vordefinierte Schwellenwert sind; und/oder
- eine Brustkrebsbehandlung, ausgewählt aus der Gruppe bestehend aus adjuvanter Chemotherapie, einer nicht-chemotherapeutischen Behandlung und endokriner Therapie, ausgewählt wird, wenn q niedriger als der vordefinierte Schwellenwert ist,
wobei das Verfahren vor dem Berechnen von su und, fakultativ, q oder s umfasst: Bestimmen der relativen Expressionslevel von mRNA von ERBB2, ESR1, PGR und/oder MKI67 in der vor der Behandlung entnommenen Brusttumorprobe durch RT-qPCR.

12. Verfahren nach Anspruch 11, wobei die neoadjuvante oder adjuvante Chemotherapie die Verabreichung eines Taxans umfasst, wobei die endokrine Therapie in einem adjuvanten oder neoadjuvanten Setting verabreicht wird und/oder wobei die neoadjuvante Chemotherapie oder die endokrine Therapie von der Verabreichung eines Anti-ERBB2-Wirkstoffs begleitet wird, wenn es sich bei dem Brustkrebs um einen ERBB2-positiven Brustkrebs handelt, wobei fakultativ der Brustkrebs i) ein luminaler Brustkrebs und/oder ii) ein ESR1- und/oder PGR-positiver Brustkrebs ist.

13. Verfahren zur Prognose von Brustkrebs bei einem Brustkrebspatienten nach einer Brustkrebsbehandlung, wobei das Verfahren umfasst:
Berechnen eines unskalierten Scores (su) basierend auf den relativen Expressionsleveln von mRNA von ERBB2, ESR1, PGR und/oder MKI67 in einer vor der Behandlung entnommenen Brusttumorprobe des Brustkrebspatienten, wie in einem der Ansprüche 1, 4 bis 6 und 10 definiert, und, fakultativ, einer vorhergesagten Wahrscheinlichkeit für pCR, q, wie in Anspruch 7 definiert, oder eines klinischen Scores s, wie in einem der Ansprüche 8 bis 10 definiert, wobei
a) wenn ein höherer su-Score eine höhere Wahrscheinlichkeit für pCR anzeigt, ein su und, fakultativ, q oder s, die gleich oder größer als ein vordefinierter Schwellenwert sind, eine negative Prognose anzeigen, und/oder ein su und, fakultativ, q oder s, die niedriger als ein vordefinierter Schwellenwert sind, eine positive Prognose anzeigen; und
b) wenn ein niedrigerer su-Score eine höhere Wahrscheinlichkeit für pCR anzeigt, i) ein su und, fakultativ, s, die gleich oder größer als ein vordefinierter Schwellenwert sind, eine positive Prognose anzeigen, und/oder ein su und, fakultativ, s, die niedriger als ein vordefinierter Schwellenwert sind, eine negative Prognose anzeigen, und ii) ein q, das gleich oder größer als ein vordefinierter Schwellenwert ist, eine negative Prognose anzeigt, und/oder ein q, das niedriger als ein vordefinierter Schwellenwert ist, eine positive Prognose anzeigt,
wobei das Verfahren vor dem Berechnen von su und, fakultativ, q oder s umfasst: Bestimmen der relativen Expressionslevel von mRNA von ERBB2, ESR1, PGR und/oder MKI67 in der vor der Behandlung entnommenen Brusttumorprobe durch RT-qPCR.

14. Verfahren nach Anspruch 13, wobei die positive Prognose eine erhöhte/hohe Wahrscheinlichkeit für fernrezidivfreies Überleben (DRFS), krankheitsfreies Überleben (DFS) und/oder Gesamtüberleben (OS) umfasst und/oder wobei die negative Prognose eine reduzierte/niedrige Wahrscheinlichkeit für fernrezidivfreies Überleben (DRFS), krankheitsfreies Überleben (DFS) und/oder Gesamtüberleben (OS) umfasst.

15. Verfahren nach Anspruch 13 oder 14, wobei die Brustkrebsbehandlung eine neoadjuvante oder adjuvante Chemotherapie umfasst, wobei fakultativ die Brustkrebsbehandlung eine adjuvante endokrine Therapie umfasst.

16. Verwendung eines Kits in einem Verfahren nach einem der Ansprüche 1, 11 und 13, wobei das Kit umfasst:
mindestens ein Paar ERBB2-spezifischer Primer;
mindestens ein Paar ESR1-spezifischer Primer;
mindestens ein Paar PGR-spezifischer Primer; und/oder
mindestens ein Paar MKI67-spezifischer Primer.

17. Verwendung nach Anspruch 16, wobei das Kit ferner mindestens eine ERBB2-spezifische Sonde, mindestens eine ESR1-spezifische Sonde, mindestens eine PGR-spezifische Sonde und/oder mindestens eine MKI67-spezifische Sonde und/oder mindestens ein Paar referenzgenspezifischer Primer und, fakultativ, mindestens eine referenzgenspezifische Sonde umfasst, wobei weiter fakultativ das Referenzgen ausgewählt ist aus der Gruppe bestehend aus B2M, CALM2, TBP, PUM1, MRLP19, GUSB, RPL37A und CYFIP1.

## Revendications

1. Procédé de prédiction de la probabilité d'une réponse complète pathologique (pCR) d'un patient atteint d'un cancer du sein après une chimiothérapie néoadjuvante, ledit procédé comprenant :
le calcul d'un score non étalonné (su) sur la base des niveaux d'expression relatifs de l'ARNm de
i) ERBB2, ESR1, PGR et MKI67,
ii) ERBB2, ESR1 et MKI67 ou
iii) ESR1 et MKI67
par rapport au niveau d'expression d'un ou plusieurs gènes de référence choisis dans le groupe comprenant B2M, CALM2, TBP, PUM1, MRLP19, GUSB, RPL37A et CYFIP1, dans un échantillon de tumeur mammaire prélevé avant traitement du patient atteint d'un cancer du sein, tel que déterminé par PCR quantitative par transcription inverse (RT-qPCR), dans lequel
a) un score su plus élevé indique une probabilité plus élevée de pCR, dans lequel un niveau d'expression relatif plus élevé de l'ARNm de ERBB2 est associé à un su plus élevé, un niveau d'expression relatif plus élevé de l'ARNm de ESR1 est associé à un su plus faible, un niveau d'expression relatif plus élevé de l'ARNm de PGR est associé à un su plus faible, et un niveau d'expression relatif plus élevé de l'ARNm de MKI67 est associé à un su plus élevé ; ou
b) un score su plus faible indique une probabilité plus élevée de pCR, dans lequel un niveau d'expression relatif plus élevé de l'ARNm de ERBB2 est associé à un su plus faible, un niveau d'expression relatif plus élevé de l'ARNm de ESR1 est associé à un su plus élevé, un niveau d'expression relatif plus élevé de l'ARNm de PGR est associé à un su plus élevé, et un niveau d'expression relatif plus élevé de l'ARNm de MKI67 est associé à un su plus faible,
dans lequel le procédé comprend, préalablement au calcul de su : la détermination des niveaux d'expression relatifs de l'ARNm de
i) ERBB2, ESR1, PGR et MKI67,
ii) ERBB2, ESR1 et MKI67 ou
iii) ESR1 et MKI67, respectivement,
dans l'échantillon de tumeur mammaire prélevé avant traitement par RT-qPCR.

2. Le procédé selon la revendication 1, dans lequel la chimiothérapie néoadjuvante comprend l'administration d'un taxane, éventuellement dans lequel la chimiothérapie néoadjuvante est accompagnée de l'administration d'un médicament anti-ERBB2 si le cancer du sein est un cancer du sein ERBB2-positif.

3. Le procédé selon la revendication 1 ou 2, dans lequel le cancer du sein est i) un cancer du sein de type luminal, et/ou ii) un cancer du sein ESR1- et/ou PGR-positif.

4. Le procédé selon l'une quelconque des revendications 1 à 3, dans lequel,
i) le su est calculé sur la base des niveaux d'expression relatifs de l'ARNm de ERBB2, ESR1, PGR et MKI67 et, dans le calcul de su, les niveaux d'expression relatifs (REL) de l'ARNm de ERBB2, ESR1, PGR et MKI67 sont pondérés comme suit : 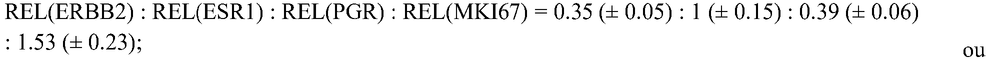 REL(ERBB2) : REL(ESR1) : REL(PGR) : REL(MKI67) = 0.35 (± 0.05) : 1 (± 0.15) : 0.39 (± 0.06) : 1.53 (± 0.23); ou 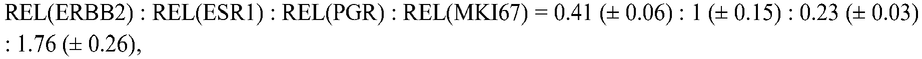 REL(ERBB2) : REL(ESR1) : REL(PGR) : REL(MKI67) = 0.41 (± 0.06) : 1 (± 0.15) : 0.23 (± 0.03) : 1.76 (± 0.26),
ii) le su est calculé sur la base des niveaux d'expression relatifs de l'ARNm de ERBB2, ESR1 et MKI67 et, dans le calcul de su, les niveaux d'expression relatifs (REL) de l'ARNm de ERBB2, ESR1 et MKI67 sont pondérés comme suit : $REL \left(ERBB2\right) : REL \left(ESR1\right) : REL \left(MKI67\right) = 0.34 \left(\pm 0.05\right) : 1 \left(\pm 0.15\right) : 1.61 \left(\pm 0.24\right) , ou$
iii) le su est calculé sur la base des niveaux d'expression relatifs de l'ARNm de ESR1 et MKI67 et, dans le calcul de su, les niveaux d'expression relatifs (REL) de l'ARNm de ESR1 et MKI67 sont pondérés comme suit : $REL \left(ESR1\right) : REL \left(MKI67\right) = 1 \left(\pm 0.15\right) : 1.63 \left(\pm 0.24\right) .$

5. Le procédé selon la revendication 4, dans lequel
i) le su est calculé sur la base des niveaux d'expression relatifs de l'ARNm de ERBB2, ESR1, PGR et MKI67, et
a) un score su plus élevé indique une probabilité plus élevée de pCR, et dans lequel su est calculé en utilisant la formule : 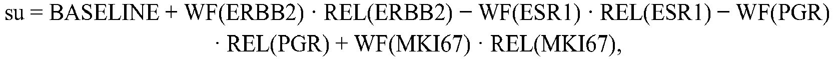 su = BASELINE + WF(ERBB2) · REL(ERBB2) - WF(ESR1) · REL(ESR1) - WF(PGR) · REL(PGR) + WF(MKI67) · REL(MKI67), ou
b) un score su plus faible indique une probabilité plus élevée de pCR, et dans lequel su est calculé en utilisant la formule : 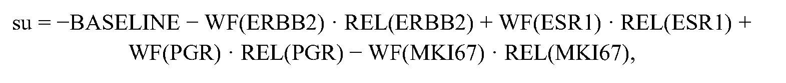 su = -BASELINE - WF(ERBB2) · REL(ERBB2) + WF(ESR1) · REL(ESR1) + WF(PGR) · REL(PGR) - WF(MKI67) · REL(MKI67),
ii) le su est calculé sur la base des niveaux d'expression relatifs de l'ARNm de ERBB2, ESR1 et MKI67, et
a) dans lequel un score su plus élevé indique une probabilité plus élevée de pCR, et dans lequel su est calculé en utilisant la formule : 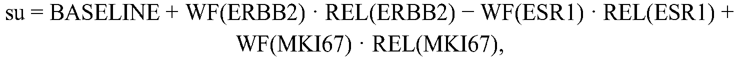 su = BASELINE + WF(ERBB2) · REL(ERBB2) - WF(ESR1) · REL(ESR1) + WF(MKI67) · REL(MKI67), ou
b) dans lequel un score su plus faible indique une probabilité plus élevée de pCR, et dans lequel su est calculé en utilisant la formule : 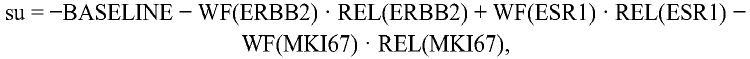 su = -BASELINE - WF(ERBB2) · REL(ERBB2) + WF(ESR1) · REL(ESR1) - WF(MKI67) · REL(MKI67), ou
iii) le su est calculé sur la base des niveaux d'expression relatifs de l'ARNm de ESR1 et MKI67, dans lequel, dans le calcul de su, les niveaux d'expression relatifs (REL) de l'ARNm de ESR1 et MKI67 sont pondérés comme suit : $REL \left(ESR1\right) : REL \left(MKI67\right) = 1 \left(\pm 0.15\right) : 1.63 \left(\pm 0.24\right) ,$
a) dans lequel un score su plus élevé indique une probabilité plus élevée de pCR, et dans lequel su est calculé en utilisant la formule : $su = BASELINE - WF \left(ESR1\right) ⋅ REL \left(ESR1\right) + WF \left(MKI67\right) ⋅ REL \left(MKI67\right)$ ou
b) dans lequel un score su plus faible indique une probabilité plus élevée de pCR, et dans lequel su est calculé en utilisant la formule : $su = - BASELINE + WF \left(ESR1\right) ⋅ REL \left(ESR1\right) - WF \left(MKI67\right) ⋅ REL \left(MKI67\right) ,$
dans lequel, respectivement, WF(ERBB2) est un facteur de pondération pour REL(ERBB2), WF(ESR1) est un facteur de pondération pour REL(ESR1), WF(PGR) est un facteur de pondération pour REL(PGR2), et WF(MKI67) est un facteur de pondération pour REL(MKI67).

6. Le procédé selon l'une quelconque des revendications 1 à 5, dans lequel
i) le su est calculé sur la base des niveaux d'expression relatifs de l'ARNm de ERBB2, ESR1, PGR et MKI67, et
a) un score su plus élevé indique une probabilité plus élevée de pCR, et dans lequel su est calculé en utilisant la formule : 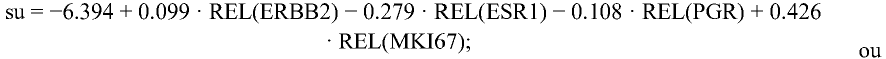 su = -6.394 + 0.099 · REL(ERBB2) - 0.279 · REL(ESR1) - 0.108 · REL(PGR) + 0.426 · REL(MKI67); ou 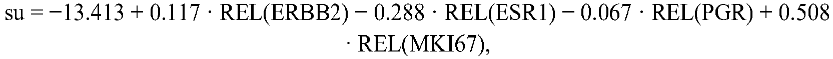 su = -13.413 + 0.117 · REL(ERBB2) - 0.288 · REL(ESR1) - 0.067 · REL(PGR) + 0.508 · REL(MKI67), ou
b) dans lequel un score su plus faible indique une probabilité plus élevée de pCR, et dans lequel su est calculé en utilisant la formule : 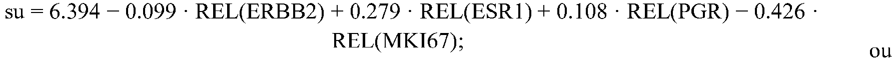 su = 6.394 - 0.099 · REL(ERBB2) + 0.279 · REL(ESR1) + 0.108 · REL(PGR) - 0.426 · REL(MKI67); ou 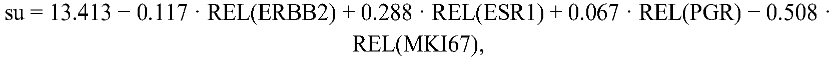 su = 13.413 - 0.117 · REL(ERBB2) + 0.288 · REL(ESR1) + 0.067 · REL(PGR) - 0.508 · REL(MKI67),
ii) le su est calculé sur la base des niveaux d'expression relatifs de l'ARNm de ERBB2, ESR1 et MKI67, et
a) dans lequel un score su plus élevé indique une probabilité plus élevée de pCR, et dans lequel su est calculé en utilisant la formule : $su = - 15.209 + 0.114 ⋅ REL \left(ERBB2\right) - 0.335 ⋅ REL \left(ESR1\right) + 0.539 ⋅ REL \left(MKI67\right) ,$ ou
b) dans lequel un score su plus faible indique une probabilité plus élevée de pCR, et dans lequel su est calculé en utilisant la formule : $su = 15.209 - 0.114 ⋅ REL \left(ERBB2\right) + 0.335 ⋅ REL \left(ESR1\right) - 0.539 ⋅ REL \left(MKI67\right)$ ou
iii) le su est calculé sur la base des niveaux d'expression relatifs de l'ARNm de ESR1 et MKI67, et
a) dans lequel un score su plus élevé indique une probabilité plus élevée de pCR, et dans lequel su est calculé en utilisant la formule : $su = - 10.625 - 0.324 ⋅ REL \left(ESR1\right) + 0.527 ⋅ REL \left(MKI67\right)$ ou
b) dans lequel un score su plus faible indique une probabilité plus élevée de pCR, et dans lequel su est calculé en utilisant la formule : $su = 10.625 + 0.324 ⋅ REL \left(ESR1\right) - 0.527 ⋅ REL \left(MKI67\right) .$

7. Le procédé selon l'une quelconque des revendications 1 à 6, comprenant en outre :
le calcul d'une probabilité prédite de pCR, q, dans lequel
a) si un score su plus élevé indique une probabilité plus élevée de pCR, q est calculé en utilisant la formule $q = \frac{exp \left(su\right)}{\left(1+exp\left(su\right)\right)} ;$ et
b) si un score su plus faible indique une probabilité plus élevée de pCR, q est calculé en utilisant la formule $q = 1 - \frac{exp \left(su\right)}{\left(1+exp\left(su\right)\right)} ,$
dans lequel, de préférence, un q qui est supérieur ou égal à un seuil prédéfini indique une probabilité élevée de pCR, et un q qui est inférieur à un seuil prédéfini indique une faible probabilité de pCR.

8. Le procédé selon l'une quelconque des revendications 1 à 7, comprenant en outre :
le calcul d'un score clinique s sur la base de su, dans lequel s présente une échelle de 0 à 100.

9. Le procédé selon la revendication 6, dans lequel le su est calculé sur la base des niveaux d'expression relatifs de l'ARNm de ERBB2, ESR1, PGR et MKI67 en utilisant la formule $su = - 6.394 + 0.099 ⋅ REL \left(ERBB2\right) - 0.279 ⋅ REL \left(ESR1\right) - 0.108 ⋅ REL \left(PGR\right) + 0.426 ⋅ REL \left(MKI67\right) ,$ et
dans lequel le procédé comprend en outre :
le calcul d'un score clinique s sur la base de su, dans lequel s est calculé en utilisant la formule $s = \left(su+3.960\right) ⋅ 18.191 \left(arrondi à 0 chiffre après la virgule\right) ,$ dans lequel
| | |
|---|---|
| si (su + 3.960) · 18.191 < 0 | s = 0, et |
| si (su + 3.960) · 18.191 > 100 | s = 100. |

10. Le procédé selon l'une quelconque des revendications 1 à 6, 8 et 9, dans lequel
a) si un score su plus élevé indique une probabilité plus élevée de pCR, un score s ou un score su qui est supérieur ou égal à un seuil prédéfini indique une probabilité élevée de pCR, et un score s ou un score su qui est inférieur au seuil prédéfini indique une faible probabilité de pCR ; et
b) si un score su plus faible indique une probabilité plus élevée de pCR, un score s ou un score su qui est inférieur à un seuil prédéfini indique une probabilité élevée de pCR, et un score s ou un score su qui est supérieur ou égal au seuil prédéfini indique une faible probabilité de pCR.

11. Procédé de sélection d'un traitement du cancer du sein pour un patient atteint d'un cancer du sein, ledit procédé comprenant :
le calcul d'un score non étalonné (su) sur la base des niveaux d'expression relatifs de l'ARNm de ERBB2, ESR1, PGR et/ou MKI67 dans un échantillon de tumeur mammaire prélevé avant traitement du patient atteint d'un cancer du sein tel que défini selon l'une quelconque des revendications 1, 4 à 6 et 10, et, éventuellement, d'une probabilité prédite de pCR, q, telle que définie selon la revendication 7, ou d'un score clinique s, tel que défini selon l'une quelconque des revendications 8 à 10 ; et
la sélection d'un traitement du cancer du sein pour le patient atteint d'un cancer du sein sur la base de su et, éventuellement, de q ou de s, dans lequel
a) si un score su plus élevé indique une probabilité plus élevée de pCR,
- la chimiothérapie néoadjuvante est sélectionnée si su et, éventuellement, q ou s sont supérieurs ou égaux à un seuil prédéfini ; et/ou
- un traitement du cancer du sein choisi dans le groupe constitué de la chimiothérapie adjuvante, un traitement non chimiothérapeutique et une thérapie endocrinienne est sélectionné si su et, éventuellement, q ou s sont inférieurs au seuil prédéfini ; et
b) si un score su plus faible indique une probabilité plus élevée de pCR,
- la chimiothérapie néoadjuvante est sélectionnée si su et, éventuellement, s sont inférieurs à un seuil prédéfini ;
- la chimiothérapie néoadjuvante est sélectionnée si q est supérieur ou égal à un seuil prédéfini ;
- un traitement du cancer du sein choisi dans le groupe constitué de la chimiothérapie adjuvante, un traitement non chimiothérapeutique et une thérapie endocrinienne est sélectionné si su et, éventuellement, s sont supérieurs ou égaux au seuil prédéfini ; et/ou
- un traitement du cancer du sein choisi dans le groupe constitué de la chimiothérapie adjuvante, un traitement non chimiothérapeutique et une thérapie endocrinienne est sélectionné si q est inférieur au seuil prédéfini,
dans lequel le procédé comprend, préalablement au calcul de su et, éventuellement, de q ou s : la détermination des niveaux d'expression relatifs de l'ARNm de ERBB2, ESR1, PGR et/ou MKI67 dans l'échantillon de tumeur mammaire prélevé avant traitement par RT-qPCR.

12. Le procédé selon la revendication 11, dans lequel la chimiothérapie néoadjuvante ou adjuvante comprend l'administration d'un taxane, dans lequel la thérapie endocrinienne est administrée dans un cadre adjuvant ou néoadjuvant et/ou dans lequel la chimiothérapie néoadjuvante ou la thérapie endocrinienne est accompagnée de l'administration d'un médicament anti-ERBB2 si le cancer du sein est un cancer du sein ERBB2-positif, dans lequel éventuellement le cancer du sein est i) un cancer du sein de type luminal, et/ou ii) un cancer du sein ESR1- et/ou PGR-positif.

13. Procédé de pronostic du cancer du sein chez un patient atteint d'un cancer du sein lors d'un traitement du cancer du sein, ledit procédé comprenant :
le calcul d'un score non étalonné (su) sur la base des niveaux d'expression relatifs de l'ARNm de ERBB2, ESR1, PGR et/ou MKI67 dans un échantillon de tumeur mammaire prélevé avant traitement du patient atteint d'un cancer du sein tel que défini selon l'une quelconque des revendications 1, 4 à 6 et 10, et, éventuellement, d'une probabilité prédite de pCR, q, telle que définie selon la revendication 7, ou d'un score clinique s, tel que défini selon l'une quelconque des revendications 8 à 10, dans lequel
a) si un score su plus élevé indique une probabilité plus élevée de pCR, un su et, éventuellement, q ou s qui sont supérieurs ou égaux à un seuil prédéfini indiquent un pronostic défavorable, et/ou un su et, éventuellement, q ou s qui sont inférieurs à un seuil prédéfini indiquent un pronostic favorable ; et
b) si un score su plus faible indique une probabilité plus élevée de pCR, i) un su et, éventuellement, s qui sont supérieurs ou égaux à un seuil prédéfini indiquent un pronostic favorable, et/ou un su et, éventuellement, s qui sont inférieurs à un seuil prédéfini indiquent un pronostic défavorable, et ii) un q qui est supérieur ou égal à un seuil prédéfini indique un pronostic défavorable, et/ou un q qui est inférieur à un seuil prédéfini indique un pronostic favorable,
dans lequel le procédé comprend, préalablement au calcul de su et, éventuellement, de q ou s : la détermination des niveaux d'expression relatifs de l'ARNm de ERBB2, ESR1, PGR et/ou MKI67 dans l'échantillon de tumeur mammaire prélevé avant traitement par RT-qPCR.

14. Le procédé selon la revendication 13, dans lequel le pronostic favorable comprend une probabilité accrue/élevée de survie sans récidive à distance (DRFS), de survie sans maladie (DFS) et/ou de survie globale (OS) et/ou dans lequel le pronostic défavorable comprend une probabilité réduite/faible de survie sans récidive à distance (DRFS), de survie sans maladie (DFS) et/ou de survie globale (OS).

15. Le procédé selon la revendication 13 ou 14, dans lequel le traitement du cancer du sein comprend une chimiothérapie néoadjuvante ou adjuvante, dans lequel éventuellement le traitement du cancer du sein comprend une thérapie endocrinienne adjuvante.

16. Utilisation d'un kit dans un procédé selon l'une quelconque des revendications 1, 11 et 13, le kit comprenant :
au moins une paire d'amorces spécifiques de ERBB2 ;
au moins une paire d'amorces spécifiques de ESR1 ;
au moins une paire d'amorces spécifiques de PGR ; et/ou
au moins une paire d'amorces spécifiques de MKI67.

17. L'utilisation selon la revendication 16, dans laquelle le kit comprend en outre au moins une sonde spécifique de ERBB2, au moins une sonde spécifique de ESR1, au moins une sonde spécifique de PGR et/ou au moins une sonde spécifique de MKI67 et/ou au moins une paire d'amorces spécifiques de gènes de référence et, éventuellement, au moins une sonde spécifique de gènes de référence, dans lequel en outre éventuellement le gène de référence est choisi dans le groupe constitué de B2M, CALM2, TBP, PUM1, MRLP19, GUSB, RPL37A et CYFIP1.
